(19) Europäisches Patentamt
European Patent Office
Office européen des brevets

(11) EP 1 656 373 B1

(12) FASCICULE DE BREVET EUROPEEN

(45) Date de publication et mention de la délivrance du brevet:
**18.01.2012 Bulletin 2012/03**

(21) Numéro de dépôt: **04742558.2**

(22) Date de dépôt: **22.04.2004**

(51) Int Cl.:
***C07D 417/12** (2006.01)*

(86) Numéro de dépôt international:
**PCT/FR2004/000981**

(87) Numéro de publication internationale:
**WO 2004/096798 (11.11.2004 Gazette 2004/46)**

(54) **DERIVES DE 2-ACYLAMINO-4-PHENYLTHIAZOLE, LEUR PREPARATION ET LEUR UTILISATION EN TANT QU'ANTAGONISTES DES CHIMIOKINES**

2-ACYLAMINO-4-PHENYLTHIAZOLDERIVATE, IHRE HERSTELLUNG UND ANWENDUNG ALS CHEMOKIN ANTAGONISTEN

DERIVATIVES OF 2-ACYLAMINO-4-PHENYLTHIAZOLE, PREPARATION METHOD THEREOF AND USE OF SAME AS CHEMOKINEANTAGONISTS

(84) Etats contractants désignés:
**AT BE BG CH CY CZ DE DK EE ES FI FR GB GR HU IE IT LI LU MC NL PL PT RO SE SI SK TR**
Etats d'extension désignés:
**AL HR LT LV MK**

(30) Priorité: **25.04.2003 FR 0305213**

(43) Date de publication de la demande:
**17.05.2006 Bulletin 2006/20**

(73) Titulaire: **SANOFI**
**75013 Paris (FR)**

(72) Inventeurs:
- **CARAYON, Pierre**
**F-34090 Montpellier (FR)**
- **CASELLAS, Pierre**
**F-34070 Montpellier (FR)**
- **FLOUTARD, Daniel**
**F-34980 Combaillaux (FR)**
- **FRAISSE, Pierre**
**75013 Paris (FR)**
- **JEGHAM, Samir**
**F-34980 Montferrier-Sur-Lez (FR)**
- **LABEEUW, Bernard**
**F-34080 Montpellier (FR)**

(74) Mandataire: **Domenego, Bertrand et al**
**Cabinet Lavoix**
**2, place d'Estienne d'Orves**
**75441 Paris Cedex 09 (FR)**

(56) Documents cités:

| | |
|---|---|
| **EP-A- 0 001 727** | **EP-A1- 0 638 553** |
| **WO-A-02/18335** | **WO-A-02/051397** |
| **WO-A-02/066460** | **WO-A-02/081449** |
| **DD-A- 55 034** | **FR-A- 2 296 497** |
| **JP-A- 2004 256 429** | **US-A- 5 656 642** |
| **US-A1- 2002 119 980** | |

- **LOMBARDINO, JOSEPH G. ET AL: "Potent antiinflammatory N-heterocyclic 3-carboxamides of 4-hydroxy-2-methyl-2H-1,2-benzothiazine 1,1-dioxide" JOURNAL OF MEDICINAL CHEMISTRY 16(5), 493-6, 1973, XP000938875**
- **SAGI, KAZUYUKI ET AL: "Rational Design, Synthesis, and Structure-Activity Relationships of Novel Factor Xa Inhibitors: (2-Substituted-4-amidinophenyl)pyruvic and propionic Acids" JOURNAL OF MEDICINAL CHEMISTRY , 46(10), 1845-1857 CODEN: JMCMAR; ISSN: 0022-2623, 2003, XP002303208**
- **PEARSON A J ET AL: "PREPARATION OF FUNCTIONALIZED P-PHENYLENEDIAMINE DERIVATIVES USING ARENE-IRON CHEMISTRY" JOURNAL OF ORGANIC CHEMISTRY, AMERICAN CHEMICAL SOCIETY. EASTON, US, vol. 61, no. 4, 1996, pages 1297-1305, XP002938137 ISSN: 0022-3263**
- **DATABASE BEILSTEIN [Online] BEILSTEIN INSTITUTE FOR ORGANIC CHEMISTRY, FRANKFURT-MAIN, DE; XP002303209 Database accession no. 15473-90-2 (RN) & J. PHARM. SCI., vol. 56, 1967, pages 38-42,**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- **DATABASE BEILSTEIN [Online] BEILSTEIN INSTITUTE FOR ORGANIC CHEMISTRY, FRANKFURT-MAIN, DE; XP002303210 Database accession no. 8555931 (RN), 7129024(RN) & J.MED.CHEM., vol. 43, 2 avril 2000 (2000-04-02), pages 649-663,**
- **BLICKE F F ET AL: "Basic-alkyl Esters of p-(Aminoalkyl)-benzoic Acids" JOURNAL OF THE AMERICAN CHEMICAL SOCIETY, AMERICAN CHEMICAL SOCIETY, WASHINGTON, DC, US, vol. 65, no. 12, 22 décembre 1943 (1943-12-22), pages 2281-2284, XP002130313 ISSN: 0002-7863**

Remarques:

Le dossier contient des informations techniques présentées postérieurement au dépôt de la demande et ne figurant pas dans le présent fascicule.

## Description

La présente invention se rapporte à des dérivés de 2-acylamino-4-phénylthiazole, à leur préparation et à leur application en thérapeutique.

La demande de brevet EP0001727 décrit des dérivés d'aminothiazoles ayant des propriétés immunomodulatrices. L'article de Lombardino, Joseph G (J. Med. Chem 16(5), 493-6, 1973) décrit des composés N-hétérocyclique 3-carbowamides de 4-hydroxy-2-méthyl-2H-1,2-benzothiazine 1,1-dioxide possédant une activité anti-inflammatoire. La demande de brevet WO 02/051397 décrit des dérivés d'anilines inhibiteurs de la production de cytokines. La demande de brevet WO 02/081449 décrit des dérivés bipipéridinyl inhibiteurs des récepteurs chimiokines.

La présente invention a pour objet des composés répondant à la formule (Ia) :

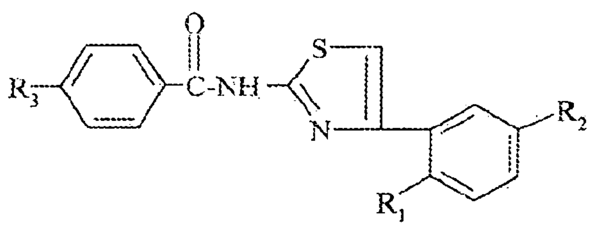

(Ia)

/

dans laquelle :

- $R_1$ représente un atome d'halogène, un groupe $(C_1\text{-}C_4)$alkyle, hydroxyle, $(C_1\text{-}C_4)$alcoxy, $(C_3\text{-}C_8)$cycloalkyloxy, allyloxy, cyclopropylméthoxy, $(C_1\text{-}C_4)$alkylthio ; et/ou
- $R_2$ représente un atome d'halogène, un groupe $(C_1\text{-}C_8)$alkyle, trifluoromethyle, $(C_3\text{-}C_{10})$cycloalkyle, phényle, $(C_1\text{-}C_8)$ alcoxy, allyloxy, $(C_3\text{-}C_8)$cycloalkylméthoxy, $(C_3\text{-}C_8)$cycloalkyloxy, $(C_3\text{-}C_8)$cycloalkylméthyle; et/ou
- $R_3$ représente un groupe choisi parmi :

  a)

    a1) -O-$(C_2\text{-}C_4)$alk-A;
    a2) -O-$(C_1\text{-}C_4)$alk-B;
    a3) -O-E;

  b) -$(C_1\text{-}C_4)$alk-A;
  c) -B ;
  d)

    d1) -$(C_1\text{-}C_4)$alk-$NR_4$-$(C_2\text{-}C_3)$alk-A ;
    d2) -$(C_1\text{-}C_4)$alk-$NR_4$-$(C_1\text{-}C_3)$alk-B;

  e)

    e1) -$CONR_4$-$(C_2\text{-}C_4)$alk-A ;
    e2) -$CONR_4$-$(C_1\text{-}C_4)$alk-B ;
    e3) -$CONR_4$-E ;

  f)

    f1) -CO-D-$(C_1\text{-}C_2)$alk-A ;
    f2) -CO-G-A ;
    f3)

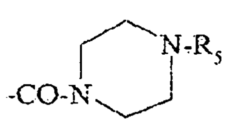

;

f4)

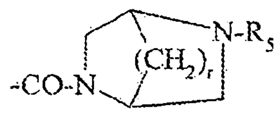 ;

f5)

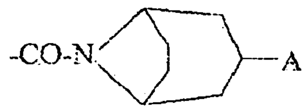 ;

f6)

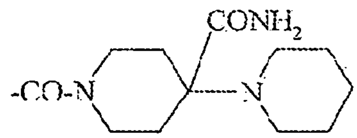 ;

f7)

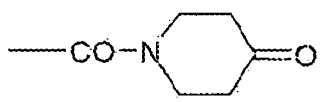 ;

- $R_4$ représente un atome d'hydrogène ou un groupe $(C_1\text{-}C_4)$alkyle ;
- A représente un groupe $NR_5R_6$ ;
- B représente un groupe

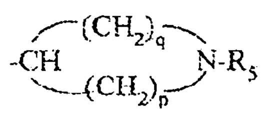 ;

- D représente un groupe

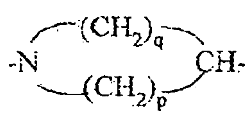 ;

- E représente un groupe

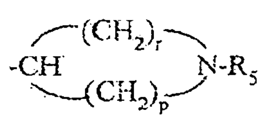 ;

- G représente un groupe

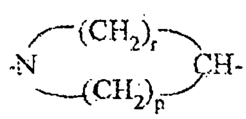 ;

- $R_5$ et $R_6$ représentent chacun indépendamment l'un de l'autre un atome d'hydrogène, un groupe $(C_1\text{-}C_6)$alkyle, allyle, $(C_2\text{-}C_4)$ alk-O-$(C_1\text{-}C_4)$alkyle, $(C_2\text{-}C_4)$alk-OH, $(C_1\text{-}C_3)$alk-CON$(R_4)_2$, $(C_2\text{-}C_3)$alk-NHCO-$(C_1\text{-}C_4)$alkyle, $(C_3\text{-}C_7)$ cycloalkyle, $(C_3\text{-}C_7)$cycloalkylméthyle, -CO-$(C_1\text{-}C_4)$alkyle, benzyle, pyrrolidinyle éventuellement substituée par un groupe -CO$(C_1\text{-}C_4)$alkyle, tétrahydropyranyle, tétrahydropyranylméthyle, diméthyltétrahydropyranyl, tétrahydrofuryle, tétrahydrofurylméthyle ;
- ou $R_5$ et $R_6$ ensemble avec l'atome d'azote auquel ils sont liés constituent un radical hétérocyclique choisi parmi : aziridinyle, azétidinyle, pyrrolidinyle, pipéridinyle, perhydroazépinyle, morpholinyle, pipérazinyle, tropanyle, quinuclidinyle, 2-azabicyclo[2,2,1]heptanyle, 2-azabicyclo[2,2,2]octanyle, lesdits radicaux hétérocycliques étant non substitués ou substitués par un groupe phényle, halogénophényle, trifluorométhylphényle, trifluorométhyle, hydroxy, methoxy, hydroxyméthyle, méthoxyméthyle, formamido, trifluoroacétylamino, un groupement -$NR_4R_7$, tetrahydropyran-4-ylamino, -CON$(R_4)_2$, -$CONR_4R'_4$, - $CH_2CON(R_4)_2$ , $(C_1\text{-}C_4)$alkyle-$CONR_4$-, $(C_3\text{-}C_8)$cycloalkyle-$CONR_4$-, $(C_1\text{-}C_4)$alkyle-$OCONR_4$-, $((C_1\text{-}C_4)$alkyle-OCO$)_2$N-, $(C_1\text{-}C_4)$alkyle-COO- ; ou substitués par un ou plusieurs groupes méthyle ;
- $R'_4$ représente un groupe $(CH_2)_s$ lié à l'atome de carbone porteur de -$CONR_4R'_4$ ;
- $R_7$ représente un atome d'hydrogène, un $(C_1\text{-}C_4)$alkyle, un groupe -$SO_2CH_3$ ou $R_4$ et $R_7$ ensemble avec l'atome d'azote auquel ils sont liés constituent un radical pyrrolidinyle ou piperidinyle ;
- p représente 1, 2, 3, 4 ou 5 ;
- q représente 0, 1 ou 2 ;
- r représente 1 ou 2 ;
- s représente 2 ou 3 ;
- p + q étant inférieure ou égal à 5 ;
- p + r étant inférieur ou égal à 5 ;
- alk représente un alkylène ;
  à l'état de base ou de sel d'addition à un acide, ainsi qu'à létat d'hydrate ou de solvat.

Les composés de formule (Ia) peuvent comporter un ou plusieurs atomes de carbone asymétriques. Ils peuvent donc exister sous forme d'énantiomères ou de diastéréoisomères. Ces énantiomeres, diastéréoisomères, ainsi que leurs mélanges, y compris les mélanges racémiques, font partie de l'invention. De même, les stéréoisomères axiaux et équatoriaux, endo et exo, ainsi que leurs mélanges font partie due l'invention.
Les composés de formule (Ia) peuvent exister à l'état de bases ou de sels d'addition à des acides. De tels sels d'addition font partie de l'invention.
Ces sels sont avantageusement préparés avec des acides pharmaceutiquement acceptables, mais les sels d'autres acides utiles, par exemple, pour la purification ou l'isolement des composés de formule (Ia) font également partie de l'invention.
Les composés de formule (la) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.
Dans le cadre de la présente invention, on entend par :

- un atome d'halogène : un fluor, un chlore, un brome ou un iode ;
- un groupe alkyle : un groupe aliphatique saturé monovalent linéaire ou ramifié comportant 1 à 4 atomes de carbone ou, le cas échéant, 1 à 8 atomes de carbone. A titre d'exemples, on peut citer les groupes méthyle, éthyle, propyle, isopropyle, butyle, isobutyle, *tert*butyle, pentyle, néopentyle, *tert*pentyle, etc ;
- un groupe cycloalkyle : un groupe alkyle cyclique comportant 3 à 8 atomes de carbone ou, le cas échéant, 3 à 10 atomes de carbone, éventuellement ponté. A titre d'exemples, on peut citer les groupes cyclopropyle, cyclobutyle, cyclopentyle, cyclohexyle, cycloheptyle, cyclooctyle, adamantyle :
- un groups alcoxy : un radical -O-allyle où le groupe alkyle est tel que précédemment défini ;

un groupe alkylène : un groupe aliphatique saturé bivalent linéaire ou ramifié comportant 1 à 3 atomes de carbone ou, le cas échéant, 2 à 3 ou 2 à 4 atomes de carbone.
Plus particulièrement, parmi ces composés on peut citer les composés dans lesquels:

- $R_1$ représente un groupe $(C_1\text{-}C_4)$alcoxy, cyclopropylméthoxy ou $(C_1\text{-}C_4)$alkylthio ; et/ou
- $R_2$ représente un atome d'halogène, un groupe $(C_1\text{-}C_8)$alkyle, trifluorométhyle, $(C_3\text{-}C_{10})$cycloalkyle ou $(C_1\text{-}C_8)$ alcoxy; et/ou
- $R_3$ représente un groupe f2 ou e2.

à l'état de base ou de sel d'addition à un acide, ainsi qu'à létat d'hydrate ou de solvat.

Parmi les composés de formule (Ia) de l'invention, on peut citer notamment les composés suivants :

- 4-((4-[3-(R)-(acétylamino)pyrrolidin-1-yl]pipéridin-1-yl)carbonyl)-N-[4-(5-butyl-2-méthoxyphényl)-1,3-thiazol-2-yl] benzamide;
- ester éthylique (1-(1(4-(4-(5-Butyl-2-methoxy-phenyl)-thiazol-2-ylcarbarmoyl)-piperidin-4-yl)-pyrrolidin-3-yl) de l'acide carbamique;
- N-(4-(5-butyl-2-methoxy-phenyl)-thiazol-2-yl)-4-(4-(3-(R)acetylamino-pyrrolidin-1-yl)-piperidine-1-carbonyl)-benzamide:
- N-(4-(5-butyl-2-methoxy-phenyl)-thiazol-2-yl)-4-(4-(tetrahydro-pyran-4-ylamino)-piperidine-1-carbonyl)-benzamide;
- N-(4-(5-Cyclohexyl-2-methoxy-phenyl)-thiazol-2-yl)-4-(2-(tetrahydro-pyran-4-ylamino)-ethoxy)-benzamide;
- N-(4-(5-Ethyl-2-methoxy-phenyl)-thiazol-2-yl)-4-(3-(tetrahydro-pyran-4-ylamino)-propyl)-benzamide;
- N-(4-(5-cyclohexyl-2-ethoxy-phenyl)-thiazol-2-yl)-4-(4-(pyrrolidin-1-yl)-piperidine-1-carbonyl)-benzamide;
- N-(4-(5-cyclohexyl-2-methoxy-phenyl)-thiazol-2-yl)-N'-pyrrolidin-2-ylmethyl-terephtalamide;
- N-(4-(5-butyl-2-methoxy-phenyl)-thiazol-2-yl)-4-(4-(3-(R)-(cyclopropanecarbonyl-amino)-pyrrolidin-1-yl)-piperidine-1-carbonyl)-benzamide;
- N-(4-(5-butyl-2-methoxy-phenyl)-thiazol-2-yl)-4-(4-(3-(R)-isobutyrylamino-pyyrolidin-1-yl)-piperidine-1-carbonyl)-benzamide;
- N-(4-(5-cyclohexyl-2-methoxy-phenyl)-thiazol-2-yl)-4-(4-(pyrrolidin-1-yl)-piperidine-1-carbonyl)-benzamide;
- N-(4-(5-butyl-2-methoxy-phenyl)-thiazol-2-yl)-4-(4-(3-(R)-hydroxy-pyrrolidin-1-yl)-piperidine-1-carbonyl)-benzamide;
- N-(4-(5-Butyl-2-methoxy-phenyl)-thiazol-2-yl)N'-((S)-(1-ethyl-pyrrolidin-2-yl)-methyl)-terephtalamide;
- N-(4-(5-butyl-2-methoxy-phenyl)-thiazol-2-yl)-4-(4-(3-hydroxy-azetidin-1-yl)-piperidine-1-carbonyl)-benzamide;
- N-(4-(5-butyl-2-methoxy-phenyl)-thiazol-2-yl)-4-(4-(3-(S)-acetylamino-pyrrolidin-1-yl)-piperidine-1-carbonyl)-benzamide;
- N-(4-(5-Ethyl-2-ethoxy-phenyl)-thiazol-2-yl)-N'-piperidin-3-yl-terephtalamide;
- N-(4-(5-Ethyl-2-ethoxy-phenyl)-thiazol-2-yl)-4-(4-(3-(R)-hydroxy-pyrrolidin-1-yl)-piperidine-1-carbonyl)-benzamide ;
- N-(4-(5-Ethyl-ethoxy-phenyl)-thiazol-2-yl)-N'-((S)-(1-ethyl-pyrrolidin-2-yl)-methyl)-terephtalamide;
- N-(4-(5-Cyclohexyl-2-ethoxy-phenyl)-thiazol-2-yl)-N'-((S)(1-ethyl-pyrrolidin-2-yl)-methyl)-terephtalamide;
- N-(4-(5-Cyclohexyl-2-methoxy-phenyl)-thiazol-2-yl)-N'-((S)-(1-ethyl-pyrrolidin-2-yl)-methyl)-terephtalamide;
- N-(4-(5-Cyclopentyl-2-methoxy-phenyl)-thiazol-2-yl)-4-(4-(tetrahydro-pyran-4-ylamino)-piperidine-1-carbonyl)-benzamide;
- N-(4-(5-Hexyl-2-methoxy-phenyl)thiazol-2-yl)-4-(4-(tetrahydro-pyran-4-ylamino)-piperidine-1-carbonyl)-benzamide;
- N-(4-(5-Cyclohexyl-2-methoxy-phenyl)-thiazol-2-yl)-4-(4-(3-(R)-acetylamino-pyrrolidin-1-yl)-piperidine-1-carbonyl)-benzamide;
- N-(4-(5-Butyl-2-methoxy-phenyl)-thiazol-2-yl)-4-(4-(3-(R)-formylamino-pyrrolidin-1-yl)-piperidine-1-carbonyl)-benzamide;
- N-(4-(5-Cyclohexyl-2-methoxy-phenyl)-thiazol-2-yl)-4-(4-(3-(S)-hydroxymethyl-pyrrolidin-1-yl)-piperidine-1-carbonyl)-benzamide;
- N-(4-(5-Propyl-2-methoxy-phenyl)-thiazol-2-yl)-4-(4-(3-(R)-acetylamino-pyrrolidin-1-yl)-piperidine-1-carbonyl)-benzamide;
- N-(4-(5-Cyclopentyl-2-methoxy-phenyl)-thiazol-2-yl)-4-(4-(3-(R)-acetylamino-pyrrolidin-1-yl)-piperidine-1-carbonyl)-benzamide;
- Ester ethylique de l'acide 1-(1-(4-(4-(5-Butyl-2-methoxy-phenyl)-thiazol-2-ylcarbamoyl)-benzoyl)-piperidin-4-yl)-pyrrolidin-3-(R)-yl-propionique;
- N-(4-(5-Cyclohexyl-2-methoxy-phenyl)-thiazol-2-yl)-4-(4-(3-(R)-propionylamino-pyrrolidin-1-yl)-piperidine-1-carbonyl)-benzamide;
- N-(4-(5-Cyclohexyl-2-methoxy-phenyl)-thiazol-2-yl)-4-(4-(3-(R)-butyrylamino-pyrrolidin-1-yl)-piperidine-1-carbonyl)-benamide;
- N-(4-(5-Cyclohexyl-2-ethoxy-phenyl)-triazol-2-yl)-4-(4-(3-(R)-acetylamino-pyrrolidin-1-yl)-piperidine-1-carbonyl)-benzamide;
- N-(4-(5-Ethyl-2-ethoxy-phenyl)-thiazol-2-yl)-4-(4-(pyrrolidin-1-yl)-piperidine-1-carbonyl)-benzamide;
- N-(4-(5-Ethyl-2-ethoxy-phenyl)-thiazol-2-yl)-N'-((R)-(1-ethyl-pyrrolidin-2-yl)-methyl)-terephtalamide;
- Ester methylique de l'acide 1-(1-(4-(4-(5-Butyl-2-methoxy-phenyl)-thiazol-2-ylcarbamoyl)-piperidin-4-yl)-pyrrolidin-3-yl)-carbamique;
- N-(4-(5-Cyclopentyl-2-methoxy-phenyl)-thiazol-2-yl)-4-(4-(3-(R)-propionylamino-pyrrolidin-1-yl)-piperidine-1-carbonyl)-benzamide;

- N-(4-(5-Butyl-2-ethoxy-phenyl)-thiazol-2-yl)-4-(4-(3-(R)-propionylamino-pyrrolidin-1-yl)-piperidine-1-carbonyl)-benzamide;
- N-(4-(5-Cyclopentyl-2-ethoxy-phenyl)-thiazol-2-yl)-4-(4-(3-(R)-propionylamino-pyrrolidin-1-yl)-piperidine-1-carbonyl)-benzamide;
- Ester ethylique de l'acide 1-(1-(4-(4-(5-Cyclopentyl-2-ethoxy-phenyl)-thiazol-2-ylcarbamoyl)-piperidin-4-yl)-pyrrolidin-3-yl)-carbamique.

Certains des composés de formule (Ia) peuvent également servir en tant qu'intermédiaires pour la préparation d'autres composés de formule (Ia), ainsi qu'il apparaîtra dans les exemples donnés ci-après.
Dans ce qui suit, on entend par groupe protecteur Pg un groupe qui permet, d'une part, de protéger une fonction réactive telle qu'un hydroxy ou une amine pendant une synthèse et, d'autre part, de régénérer la fonction réactive intacte en tin de synthèse. Des exemples de groupes protecteurs ainsi que des méthodes de protection et de déprotection sont donnés dans « Protective Groups in Organic Synthesis », Green et al., 2nd Edition (John Wiley & Sons, Inc., New York).
On entend par groupe partant, dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un mésyle, tosyle, triflate, acétyle, etc. Des exemples de groupes partants ainsi que des références pour leur préparation sont donnés dans « Advances in Organic Chemistry », J. March, 3rd Edition, Wiley Interscience, p. 310-316.
Conformément à l'invention, on peut préparer les composés de formule générale (Ia) par le procédé qui suit.
Ce procédé est caractérisé en ce que :

on traite un dérivé fonctionnel d'un acide de formule:

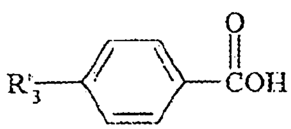

(II)

dans laquelle $R'_3$ représenté $R_3$ tel que définis ci-dessus pour (Ia) ou un groupe choisi parmi :

. OPg, Pg étant un groupe protecteur tel que le *tert*-butyle, benzoyle ou arylsulfonyle choisi parmi phénylsulfonyle, tolylsulfonyle ou naphtylsulfonyle ;
. -O-($C_1$-$C_3$)alk-Q, Q étant un groupe diméthoxyméthyle, diéthoxyméthyle ou formyle ;
. -O-($C_2$-$C_4$)alk-OX, X représentant un atome d'hydrogène, un groupe tetrahydropyranyle ou un groupe $SO_2R'$, R' étant un groupe méthyle ou tolyle ;
. -($C_1$-$C_3$)alk-Q ;
. -($C_1$-$C_4$)alk-Hal, Hal représentant un atome d'halogène ;
. -I ;
. -COOH ; -COOR avec R représentant un atome d'hydrogène, un ($C_1$-$C_4$)alkyle, ou un benzyle non substitué ou substitué sur le phényle par un groupe methoxy ;
. -CONH-($C_1$-$C_3$)alk-Q ;

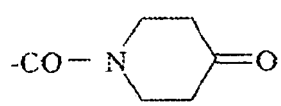 ;

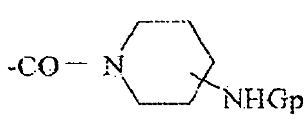

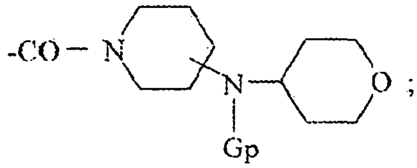

. a)

a1) -O-($C_2$-$C_4$)alk-A' ;
a2) -O-($C_1$-$C_4$)alk-B';
. a3) -O-E' ;

. b) -($C_1$-$C_4$)alk-A' ;
. c)-B' ;
. d)

d1) -($C_1$-$C_4$)alk-$NR_4$-($C_2$-$C_3$)alk-A' ;
d2) -($C_1$-$C_4$)alk-$NR_4$-($C_1$-$C_3$)alk-B' ;

. e)

e1) -$CONR_4$-($C_2$-$C_4$)alk-A' ;
e2) -$CONR_4$-($C_1$-$C_4$)alk-B' ;
e3) -$CONR_4$-E' ;

. f)

f1) -CO-D-($C_1$-$C_2$)alk-A' ;
f2) -CO-G-A' ;
f3)

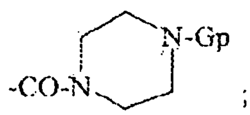

f4)

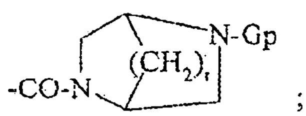

f5)

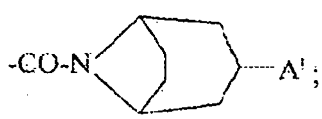

dans laquelle :

- A', B', E' représentent respectivement les groupes A, B, E tels que définis pour (Ia) dans lesquels $R_5$ est remplacé par Gp ;
- Gp représente un groupe protecteur de l'azote choisi parmi : Boc, Fmoc, ($C_1$-$C_4$)alcanoyle, benzyloxycarbonyle ou benzyle ;

par un dérivé de 2-aminothiazole de formule :

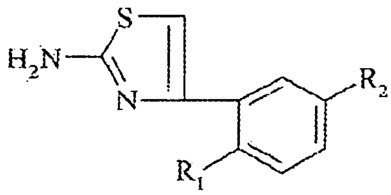 (III)

dans laquelle $R_1$ et $R_2$ sont tels que définis pour (Ia) puis, le cas échéant, on transforme le composé ainsi obtenu de formule :

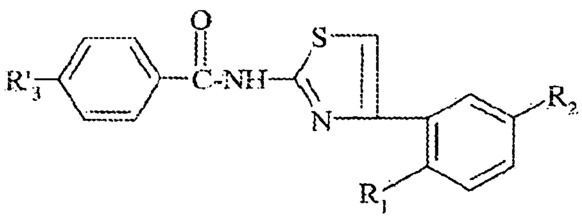 (IV)

dans laquelle:

- $R_1$ ou $R_2$ sont tels que définis pour (Ia) ;
- $R'_3$ représente un groupe choisi parmi :

  • -OPg, Pg étant un groupe protecteur tel que le *tert*-butyle, benzoyle ou arylsulfonyle choisi parmi phénylsulfonyle, tolylsulfonyle ou naphtylsulfonyle ;
  • -O-($C_1$-$C_3$)alk-Q, Q étant un groupe diméthoxyméthyle, diéthoxyméthyle ou formyle ;
  • -O-($C_1$-$C_4$)alk-OX, X représentant un atome d'hydrogène, un groupe tétrahydropyranyle ou un groupe $SO_2R$, R' étant un groupe méthyle ou tolyle ;
  • -($C_1$-$C_3$)alk-Q ;
  • -($C_1$-$C_4$)alk-Hal, Hal représentant un atome d'halogène ;
  . -1 ;
  • -COOH ; -COOR avec R représentant un atome d'hydrogène, un ($C_1$-$C_4$)alkyle, ou un benzyle non substitué ou substitué sur le phényle par un groupe méthoxy ;
  • -CONH-($C_1$-$C_3$)alk-Q ;

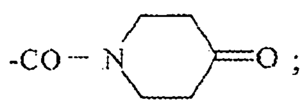

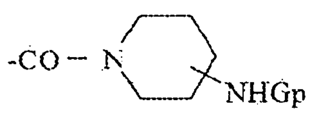

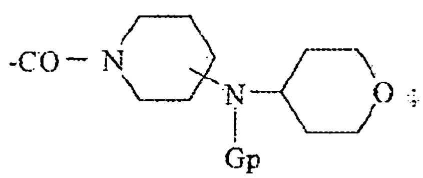

  • a)

a1) -O-($C_2$-$C_4$)alk-A' ;
a2) -O-($C_1$-$C_4$)alk-B';
a3) -O-E ;

- b) -($C_1$-$C_4$)alk-A' ;
- c) -B';
- d)

d1) -($C_1$-$C_4$)alk-$NR_4$-($C_2$-$C_3$)alk-A' ;
d2) -($C_1$-$C_4$)alk-$NR_4$-($C_1$-$C_3$)alk-B' ;

- e)

e1) -$CONR_4$-($C_2$-$C_4$)alk-A' ;
e2) -$CONR_4$-($C_1$-$C_4$)alk-B';
c3) -$CONR_4$-E' ;

- f)

f1) -CO-D-($C_1$-$C_2$)alk-A' ;

f2) -CO-G-A' ;

f3)

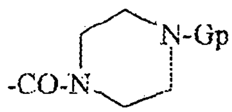 ;

f4)

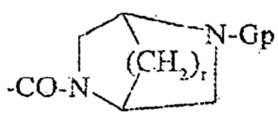 ;

f5)

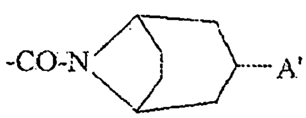 ;

dans laquelle :

- A', B', E' représentent respectivement les groupes A, B, E tels que définis pour (Ia) dans lesquels $R_5$ est remplacé par Gp ;
- Gp représente un groupe protecteur de l'azote choisi parmi : Boc, Fmoc, ($C_1$-$C_4$)alcanoyle, benzyloxycarbonyle ou benzyle.

Par dérivé fonctionnel d'un acide de formule (II), on entend un chlorure d'acide, un anhydride mixte ou symétrique, ou encore l'acide opportunément activé par exemple avec l'hexafluoro phosphate de benzotriazol-1-yloxytris (diméthylamino) phosphonium (BOP) ou le O-benzotriazol-1-yl-N,N,N',N'-tétraméthyluronium hexafluorophosphate (HBTU) ou le O-benzotriazol-1-yl-N,N,N',N'-tétraméthyluronium tétrafluoroborate (TBTU), par exemple.
L'étape est effectuée dans un solvant aprotique tel que le dichloromethane, l'acétonitrile, le THF ou le DMF, en milieu basique.
Les composés de formule (II) sont préparés par des méthodes connues qui varient selon la valeur du substituant

$R_3$ ou $R'_3$ du composé de formule (II).

Lorsque un ou plusieurs subsitutants $R_1$, $R_2$ et/ou $R'_3$ représentent un groupement contenant une fonction amine ou hydroxyle, ces fonctions peuvent être protégées intermédiairement : une fonction amine peut êre protégée par un groupement alcanoyle, benzyle, *tert*-butoxycarbonyle (Boc), benzyloxycarbonyle, ou 9-fluorénylméthoxycarbonyle (Fmoc), par exemple ; une fonction hydroxyle peut être protégée sous forme d'éther ou d'ester, par exemple.

Pour préparer un composé de formule (Ia) dans laquelle $R_3$ représente un groupe a) tel que défini pour (Ia) on peut préparer un composé de formule (II) dans laquelle $R_3$ représente un groupe a) en procédant selon le Schéma réactionnel ci-après, exemplifié pour a1), puis utiliser le procédé selon l'invention.

SCHEMA 1

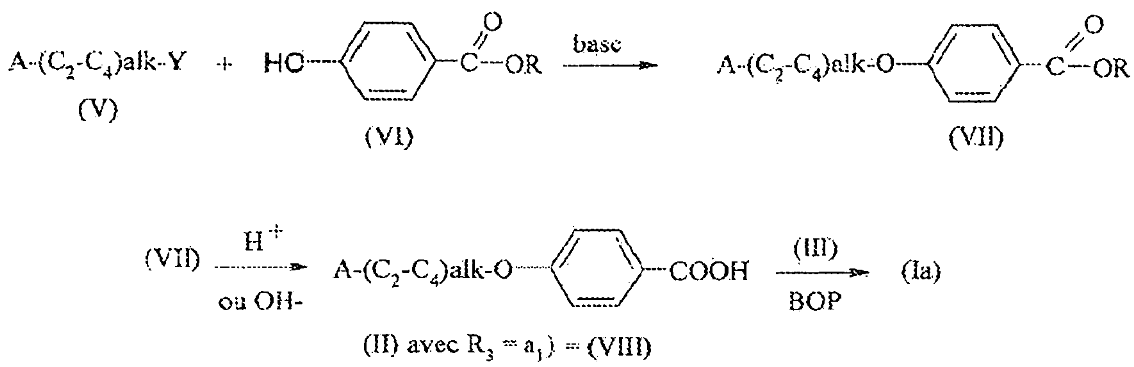


(V) (VI) (VII)

(II) avec $R_3 = a_1$) = (VIII)

Y = groupe partant

R = $(C_1\text{-}C_4)$alkyle.

On peut également procéder selon le Schéma 1, mais remplacer dans le compose de formule (V), le groupe parlant Y par un groupe hydroxyle, selon la réaction de Mitsunobu, Bull. Chem. Soc. Japan, 1967, 40, 2380.

Alternativement, pour préparer un composé de formule (1a) dans laquelle $k_3$ représente un groupe a), on peut préparer un composé de formule (IV) contenant un groupe $R'_3$, puis dans une étape ultérieure le transformer en groupe $R_3$ selon le Schéma rédactionnel suivant :

SCHEMA 2

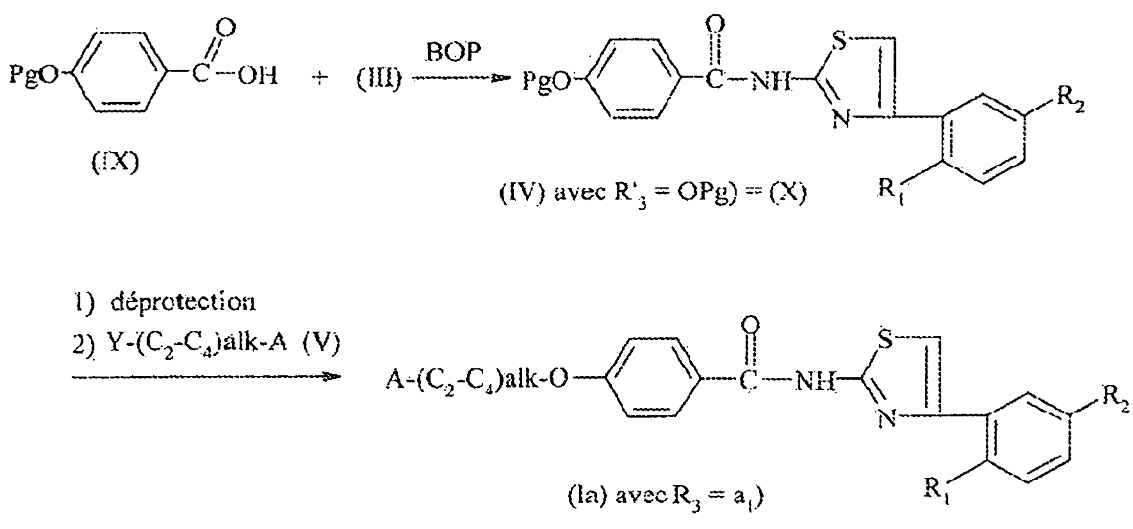


(IX)

(IV) avec $R'_3$ = OPg) = (X)

(Ia) avec $R_3 = a_1$)

Y : groupe partant.

Pg : groupement protecteur de l'oxygène, tel que *tert*-butyle, benzoyle ou arylsulfonyle, par exemple.

En remplaçant le composé de formule (V) par un composé de formule Y-E dans laquelle E est tel que décrit ci-dessus, le substituant $R_5$ étant éventuellement remplacé par un groupe protecteur de l'azote, on obtient un composé de formule (Ia) dans laquelle $R_3$ = a3).

On peut également préparer un composé de formule (Ia) dans laquelle $R_3$ représente a) à partir d'un composé comprenant un groupe $R'_3$, selon le Schéma réactionnel ci-après :

SCHEMA 3

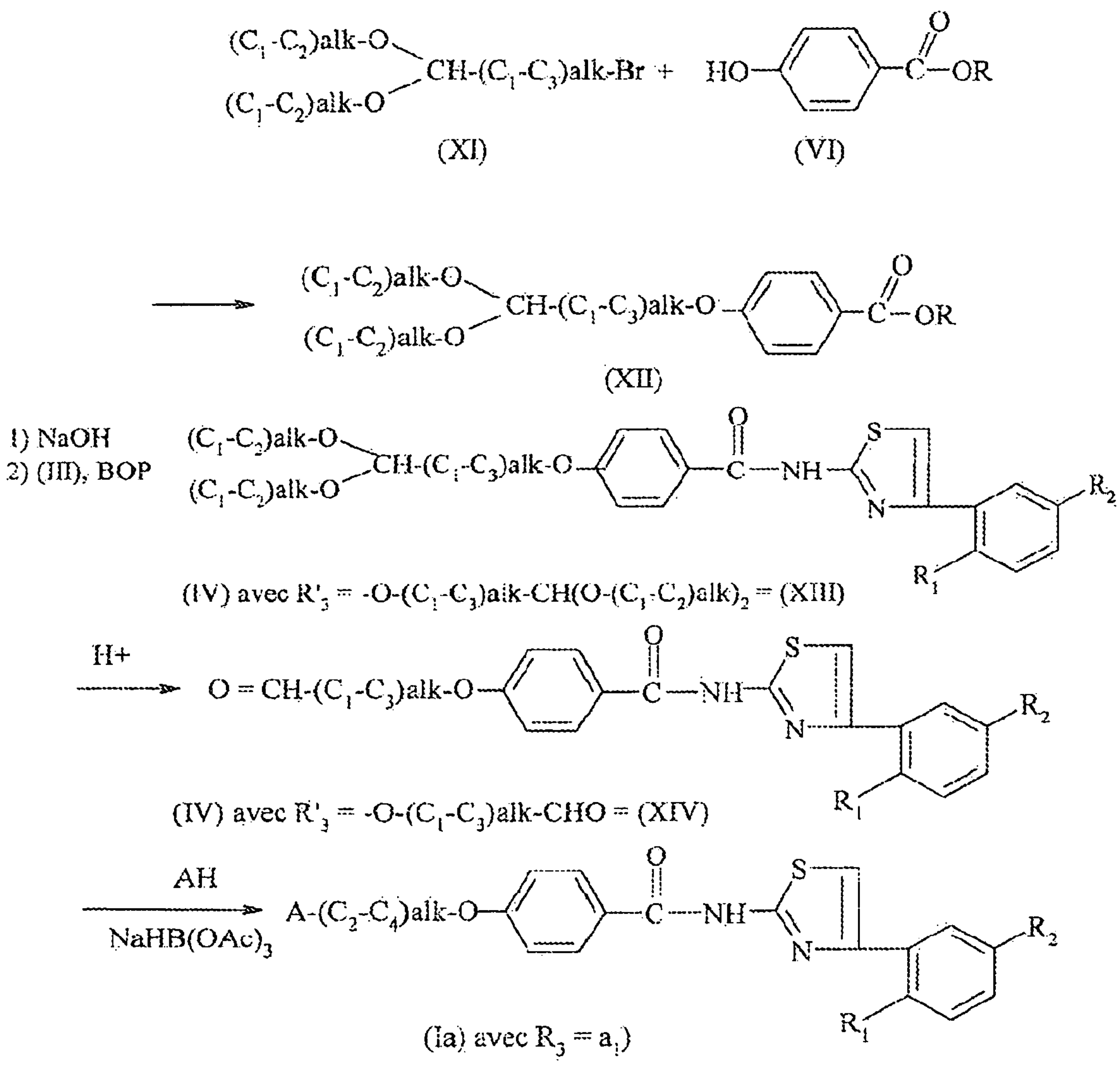


A la dernière étape, l'addition de l'amine est effectuée selon Synth. Commun., 1998, 28 (10), 1897-1905, J. Org. Chem., 1992, 57 (11), 3218-3225, J. Org. Chem, 1996, 61, 3849-3862, Tetrahedron Lett., 1990, 31, 5595-5598.

Une autre façon de préparer un composé de formule (Ia) dans laquelle $R_3$ représente un groupe a), à partir d'un composé de formule (IV) contenant $R'_3$ est représentée dans le Schéma réactionnel ci-aprés :

SCHEMA 4

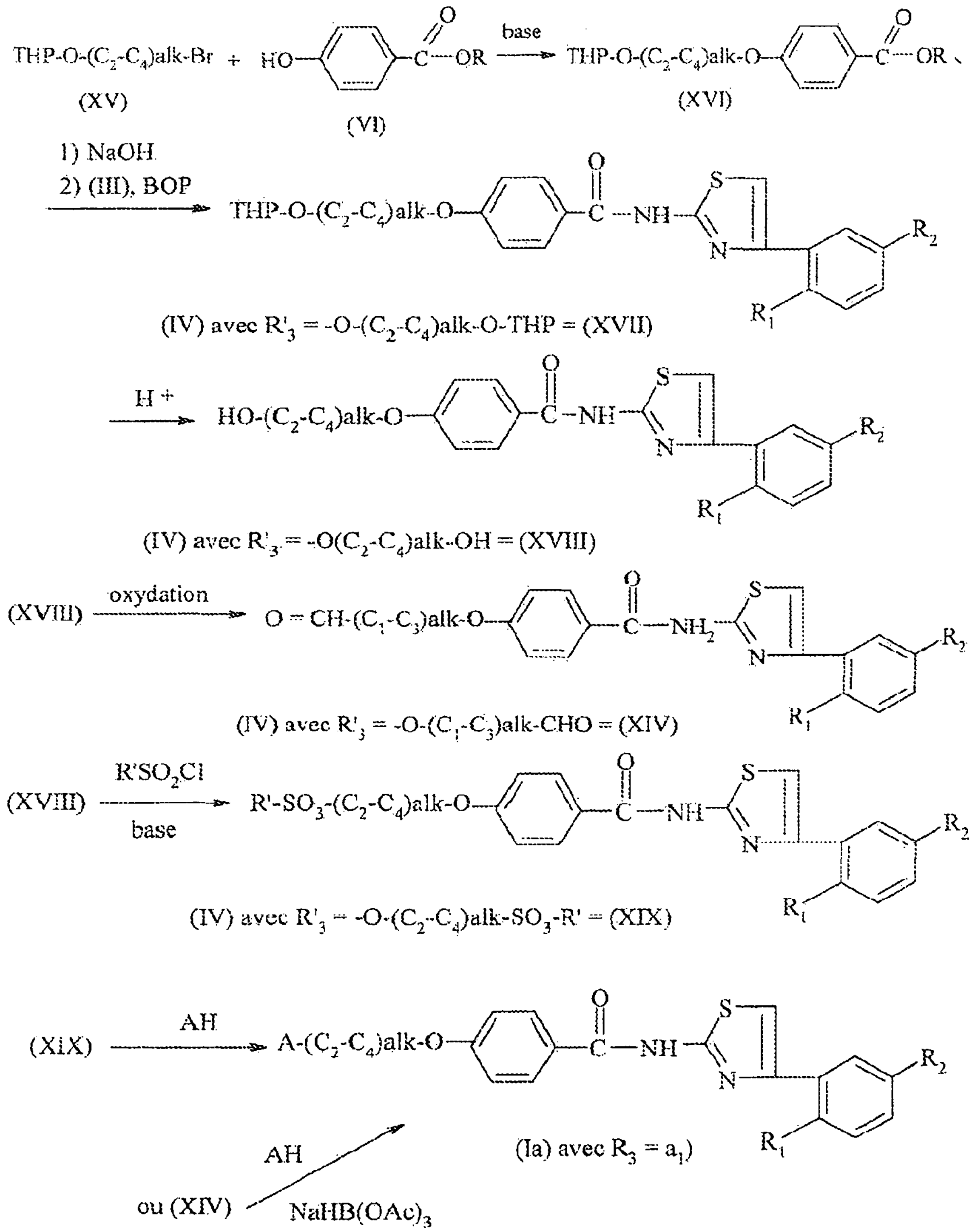


R' = méthyle ou tolyle

THP = tétrahydropyran-2-yle.

La transformation du composé de formule (XVIII) par oxydation peut être effectuée par une oxydation de Swern, par exemple.

Un exemple de préparation d'un composé de formule (II) dans laquelle $R'_3$ = al) est explicité ci-après :

SCHEMA 5

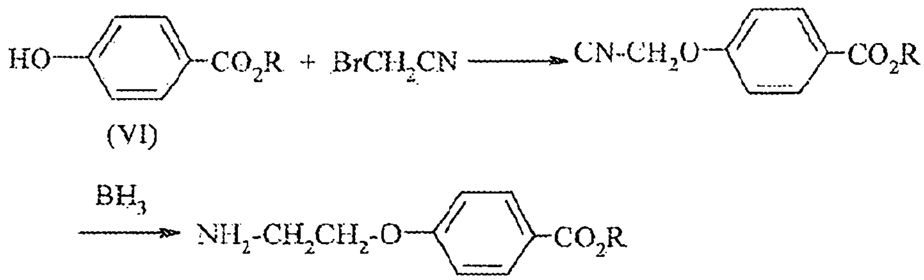


(VI)

(II) avec $R'_3$ = $-OCH_2CH_2NH_2$ = (XX)

A partir du composé (XX) ainsi obtenu, on peut ensuite préparer différents composés (II) en substituant convenablement l'amine primaire.

Pour préparer un composé de formule (Ia) dans laquelle $R_3$ est un groupe a2) ou a3), on peut également procéder par action d'un composé de formule (II) dans laquelle $R_3$ = a2) ou a3) sur un composé de formule (III) ; le composé de formule (II) étant préparé selon le Schéma réactionnel suivant exemplifié pour a2) :

SCHEMA 5 bis

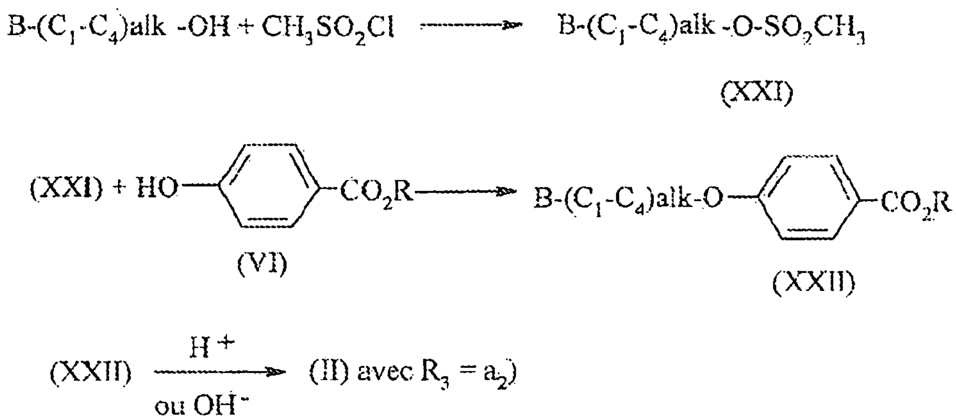


(XXI)

(VI)

(XXII)

Les acides de formule :

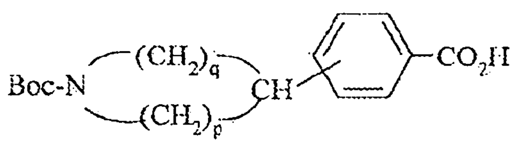

permettent de préparer des composés de formule (IV) dans laquelle $R'_3$ = c), par action sur un aminothiazole de formule (III).

Pour préparer un composé de formule (Ia) dans laquelle $R_3$ est un groupe b), on peut procéder par action d'un composé de formule (II) dans laquelle $R_3$ = b) sur un composé de formule (III) ; le composé de formule (II) ou son ester étant préparé selon le Schéma réactionnel suivant :

SCHEMA 6

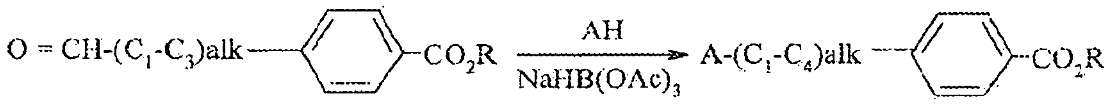


(XXIII) (XXIV)

On peut également préparer un composé de formule (Ia) dans laquelle $R_3$ est un groupe b) en procédant selon le Schéma réactionnel ci-après qui reprend, en les adaptant au cas présent, les étapes schéma 3 :

SCHEMA 7

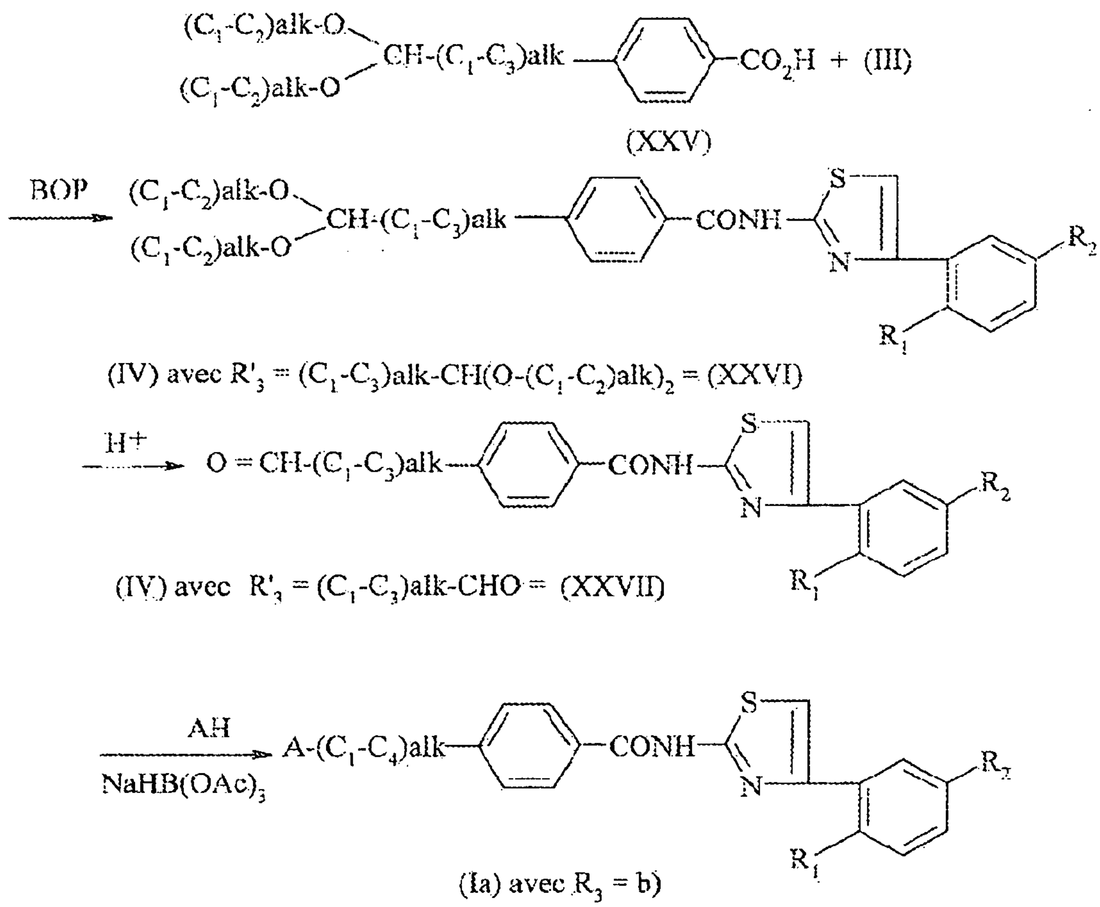


(XXV)

(IV) avec $R'_3$ = $(C_1\text{-}C_3)$alk-CH(O-$(C_1\text{-}C_2)$alk$)_2$ = (XXVI)

(IV) avec $R'_3$ = $(C_1\text{-}C_3)$alk-CHO = (XXVII)

(Ia) avec $R_3$ = b)

Dans le cas particulier où l'on prépare un composé de formule (Ia) dans laquelle $R_3$ représente un groupe b), avec $(C_1\text{-}C_4)$alk. = $(CH_2)_3$ on peut transformer un composé de formule (IV) en un composé de formule (Ia) selon le Schéma réactionnel suivant :

SCHEMA 8

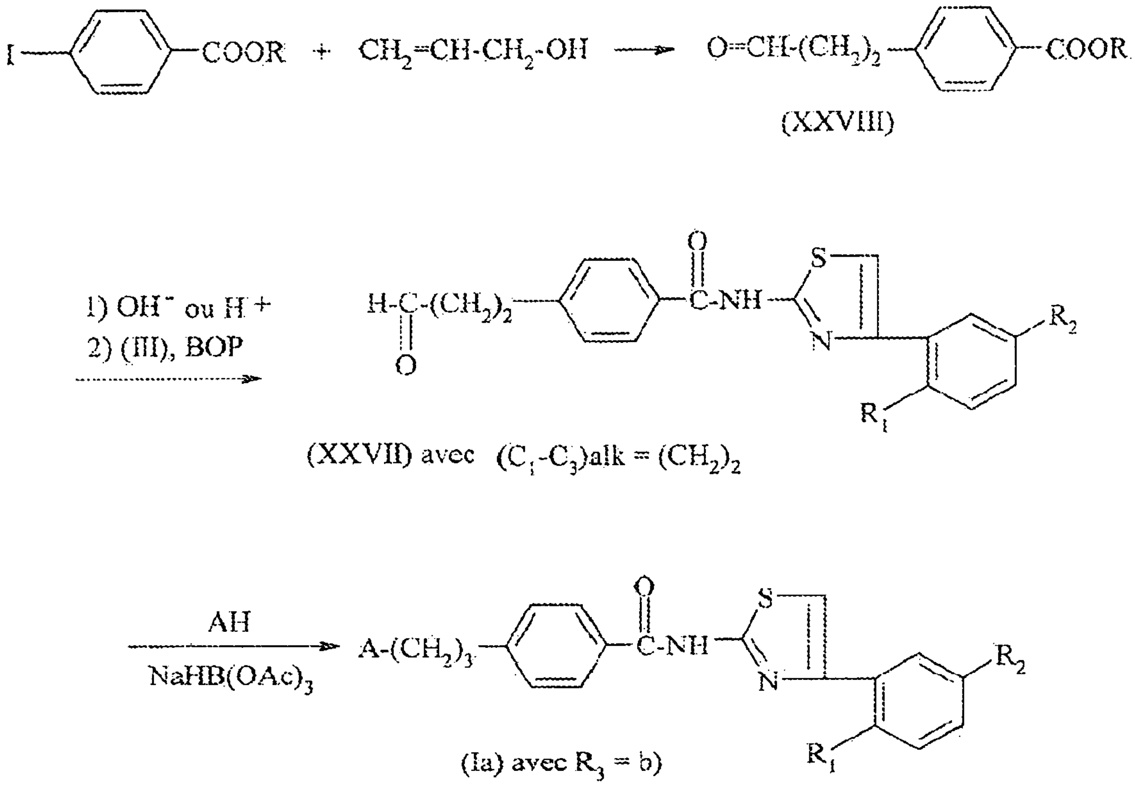


(XXVIII)

(XXVII) avec $(C_1\text{-}C_3)alk = (CH_2)_2$

(Ia) avec $R_3$ = b)

Le composé de formule (XXVII) peut également être obtenu par action de l'acide iodobenzoïque sur un composé de formule (III), puis par action du prop-2-en-1-ol sur le composé ainsi obtenu de formule (XXVII bis) :

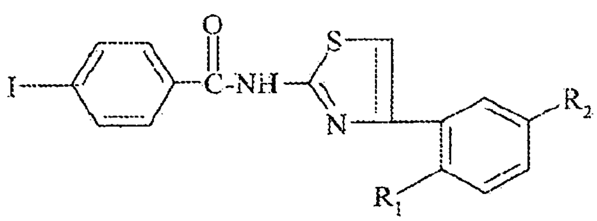

On peut également préparer un composé de formule (Ia) dans laquelle $R_3$ est b), selon le Schéma réactionnel suivant ;

SCHEMA 9

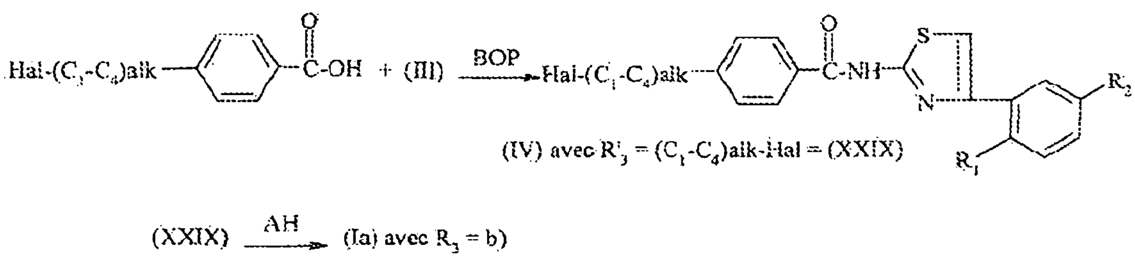


(IV) avec $R'_3 = (C_1\text{-}C_4)alk\text{-}Hal$ = (XXIX)

(XXIX) → (Ia) avec $R_3$ = b)

Hal : atome d'halogène, préférentiellement de chlore.

De maniére similaire, on peut également préparer un composé de formule (Ia) dans laquelle $R_3$ = b) à partir

d'un composé de fomule (II) préparé selon le Schéma ci-après :

SCHEMA 10

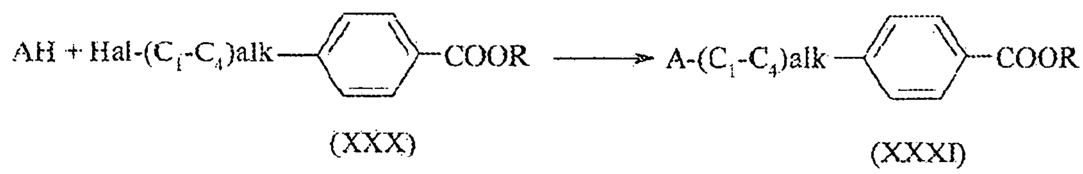

(XXX) (XXXI)

Les composés de formule (II) dans laquelle $R_3$ = c) peuvent être préparés selon SY11. Lett., 1998, 4, 379-380.

Les procédés de préparation des composés de formule (Ia) dans laquelle $R_3$ est un groupe d) sont effectués de manière semblable à ceux décrits pour la préparation des composés (Ia) dans lesquels $R_3$ = b).

Pour préparer un composé de formule (Ia) dans laquelle $R_3$ est un groupe amide substitué (groupes e) et f)) on peut procéder selon l'un des Schémas 11 ou 12 ci-après qui illustrent le cas où $R_3$ = e1) :

SCHEMA 11

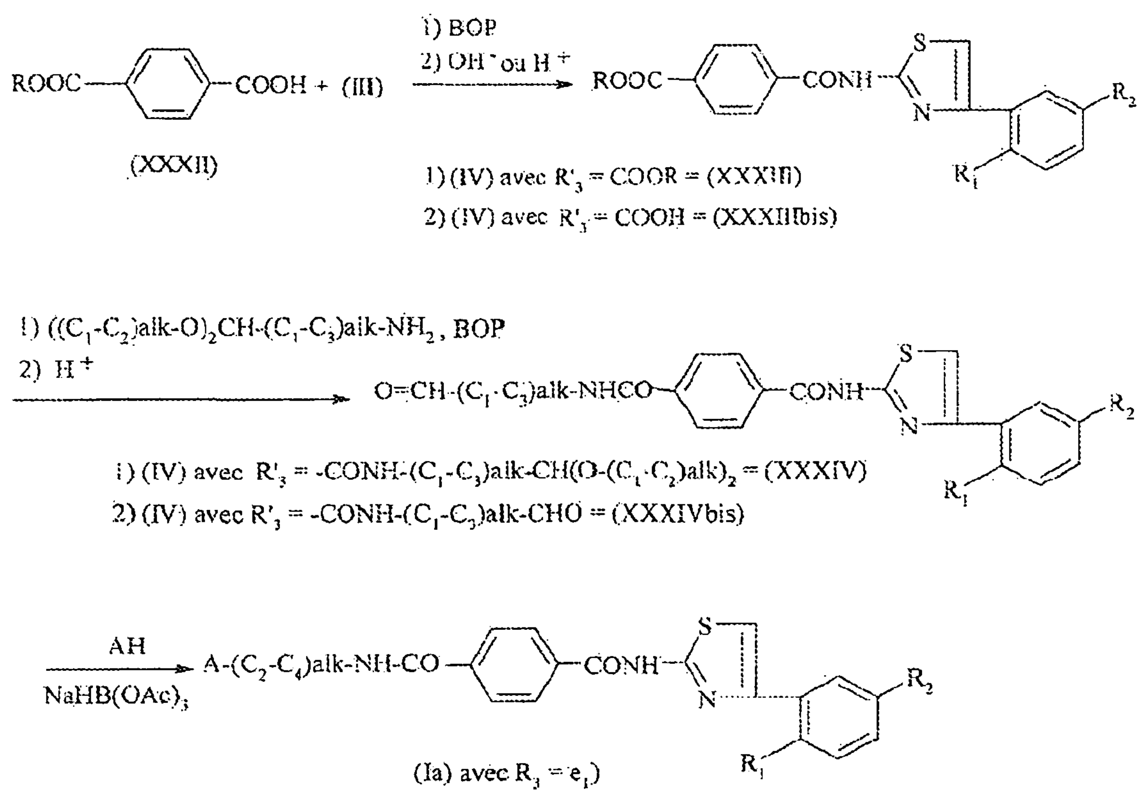


(XXXII)

(Ia) avec $R_3$ = $e_1$)

R = $(C_1-C_4)$alkyle

SCHEMA 12

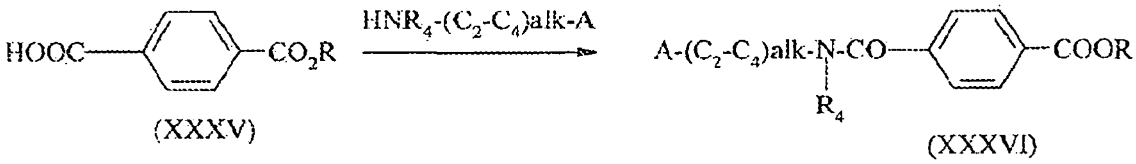


(XXXV) (XXXVI)

1) $OH^-$ ou $H^+$
2) (III), BOP
(XXXVI) (Ia) avec $R_3 = e_1$)

Selon ce mode opératoire, en utilisant comme produit de départ un composé de formule (XXXIIIbis), que l'on fait réagir sur une diamine de formule $NHR_4$-($C_2$-$C_4$)alk-A, on obtient directement un composé de formule (Ia) avec $R_3$ = e1).

Dans le cas particulier où $R_3$ est un groupe 12), et G représente un groupe pipéridine on peut procéder selon le Schéma, ci-après :

SCHEMA 13

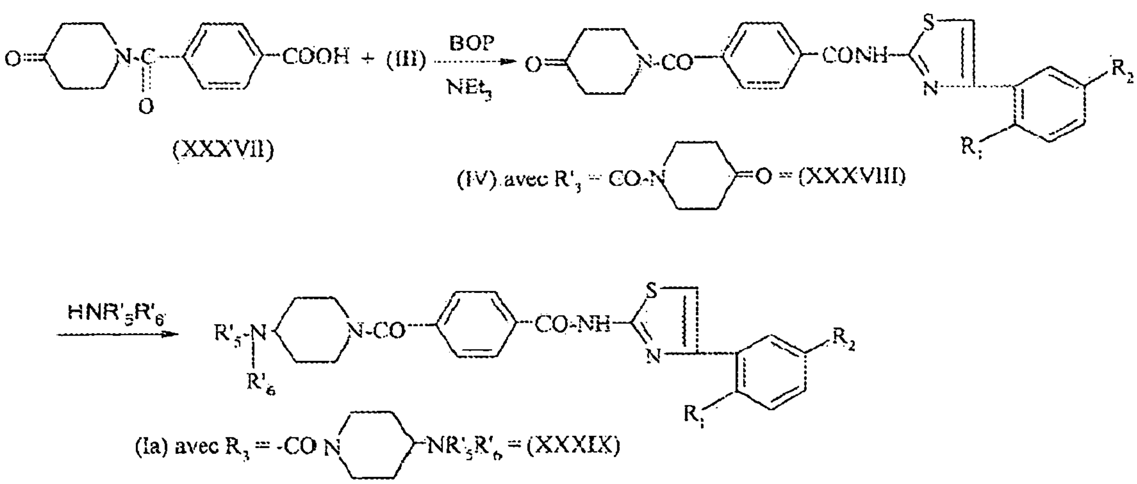


(XXXVII)

(IV) avec $R'_3$ = CO-N ... =O = (XXXVIII)

(Ia) avec $R_3$ = -CO N ... -$NR'_5R'_6$ = (XXXIX)

Les composés ainsi obtenus peuvent être ensuite transformées en composés dans lesquels $NR_5R_6$ est tel que défini dans la formule générale (Ia) par déprotection et fonctionnalisation de l'amine $NR'_5R'_6$, selon les méthodes connues de l'homme du métier.

Les composés de formule (XXXVIII) peuvent également être préparés à partir des composés de formule (XXXIIIbis) selon le schéma ci-après :

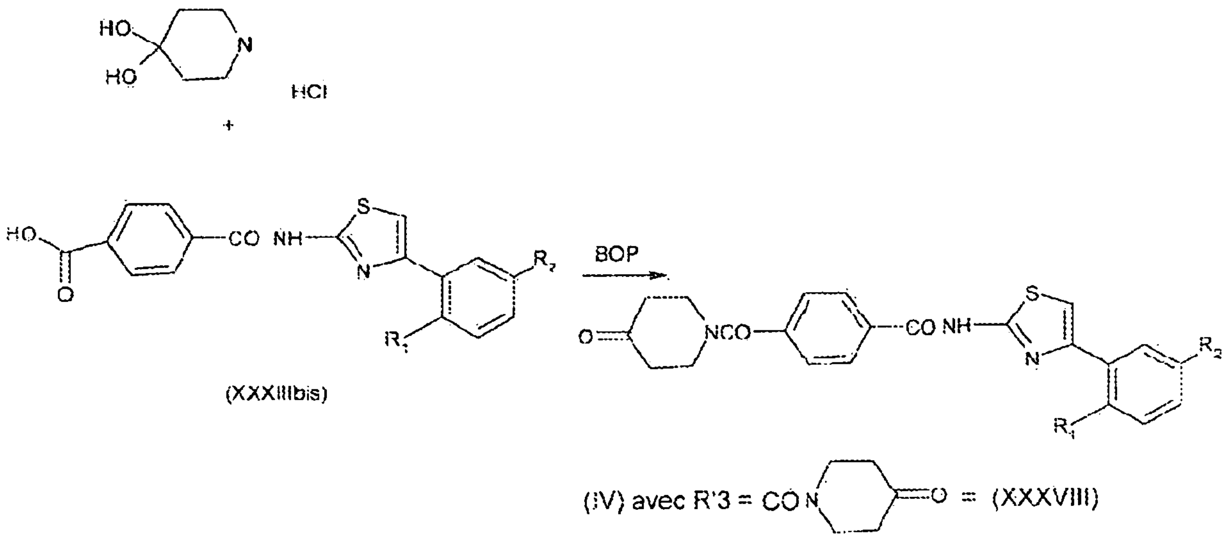


(XXXIIIbis)

(IV) avec R'3 = CO … = (XXXVIII)

Pour préparer un composé de formule (Ia) dans laquelle $R_3$ est un groupe f2) et G représente un radical pipéridinyle, on peut également procéder selon le Schéma ci-après :

SCHEMA 14

(XXXIIIbis) +

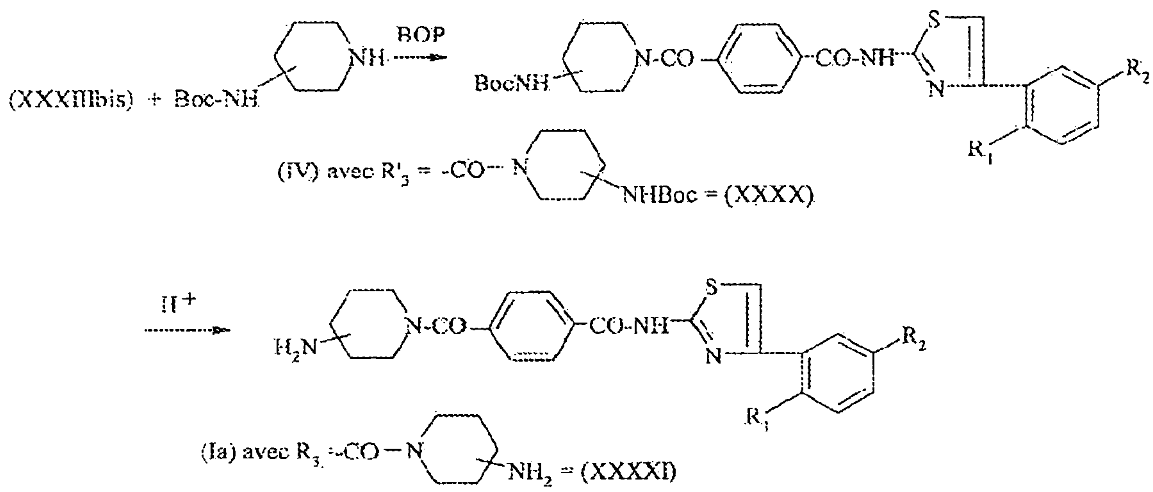


(IV) avec $R'_3$ = -CO- … = (XXXX)

(Ia) avec $R_3$ = -CO- … = (XXXXI)

A partir du composé (XXXXI) ainsi obtenu, on peut ensuite préparer différents composés de formule (Ia) dans laquelle $R_3 = f_2$) en substituant convenablement la fonction amine primaire.

Par exemple, par action de la tétrahydropyran-4-one, on prépare un composé de formule (Ia) dans laquelle $R_3$ est un groupe :

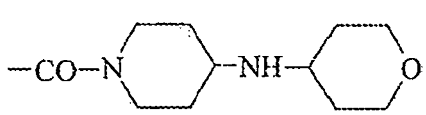

Pour préparer un composé de formule (Ia) dans laquelle $R_3$ = e) ou f), on peut procéder selon le schéma ci-après qui iilustrc le cas où $R_3 = f_2$) et G représente une pipéridine :

SCHEMA 15

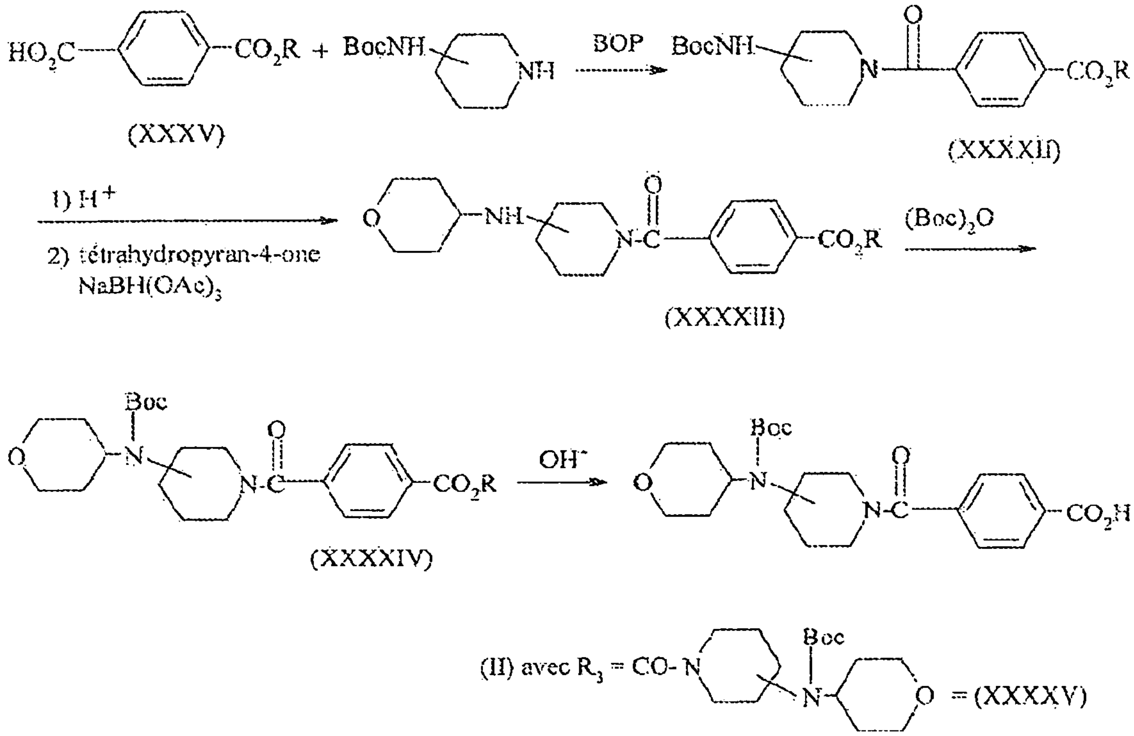


ou en inversant les premières étapes, on procède selon le schéma suivant :

SCHEMA 16

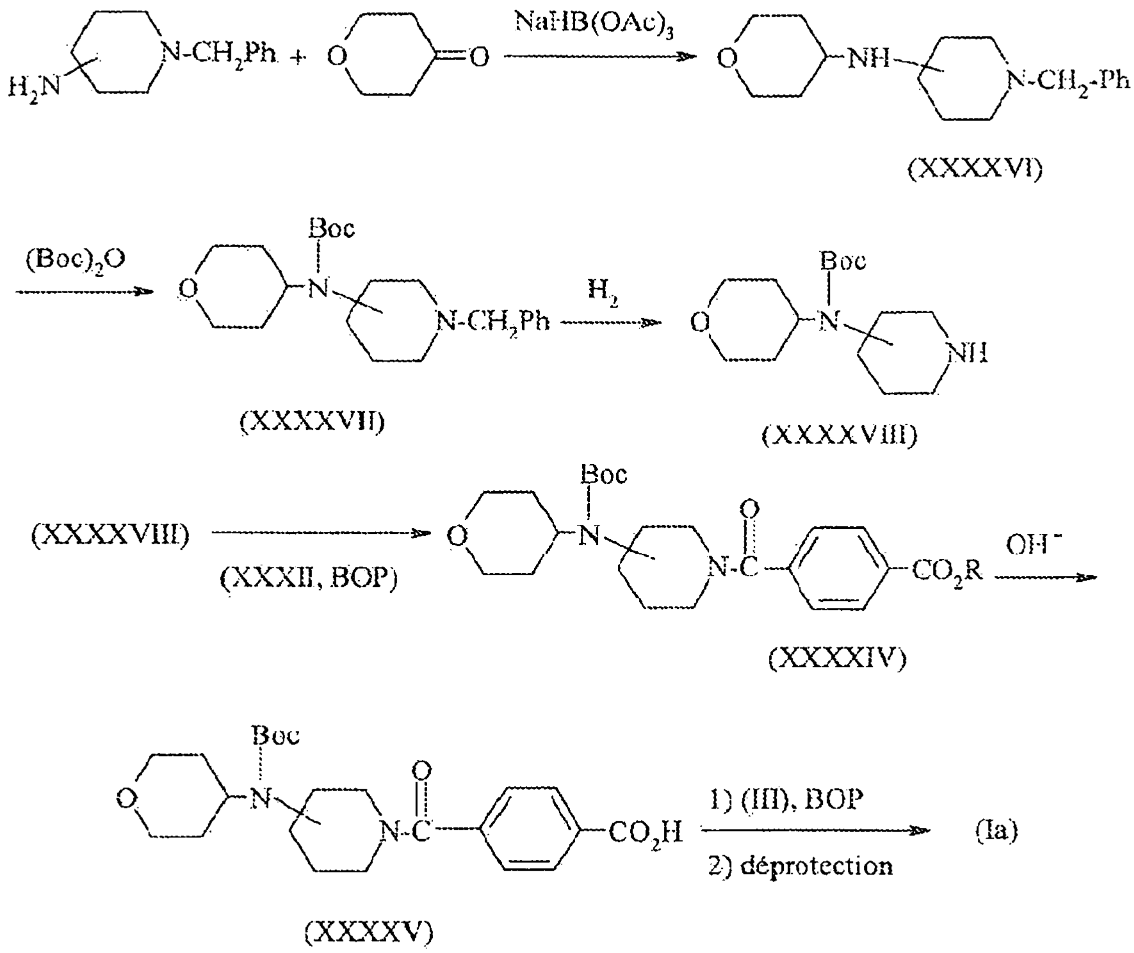


où, selon une autre alternative :

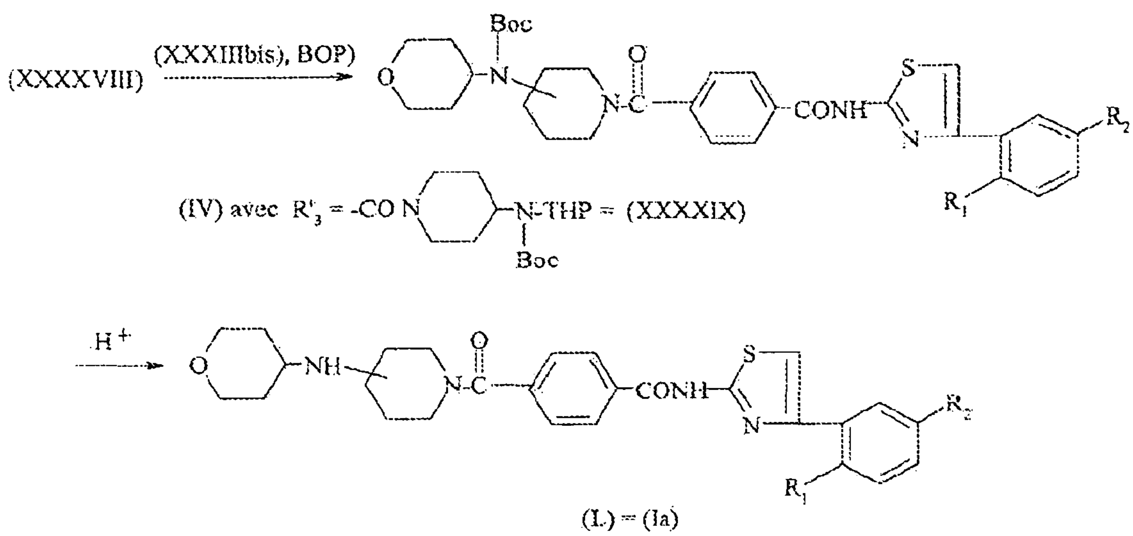


(I.) = (Ia)

Ph = phényle.

THP = tétrahydropyran-4-yle.

Dans les procédés représentés dans les Schémas 14, 15 et 16, comme groupe protecteur de l'azote, on peut utiliser le Boc, tel que représenté, ou tout autre groupe protecteur Gp approprié, par exemple un groupe alcanoyle tel que formyle ou acétyle, un groupe benzyle, un groupe 9-fluorénylméthoxycarbonyle (Fmoc) ou un groupe benzyloxycarbonyle ou *tert*-butoxycarbonyle (Boc).

Les aminothiazoles de formule (III) sont préparés par des méthodes connues telles que celles décrites dans le brevet EP 518 731, la demande de brevet EP 611 766 et la demande internationale WO 99/15525.

De façon générale, on fait réagir la thiourée avec une cétone halogénée de formule 4 selon le schéma réactionnel suivant :

SCHEMA 17

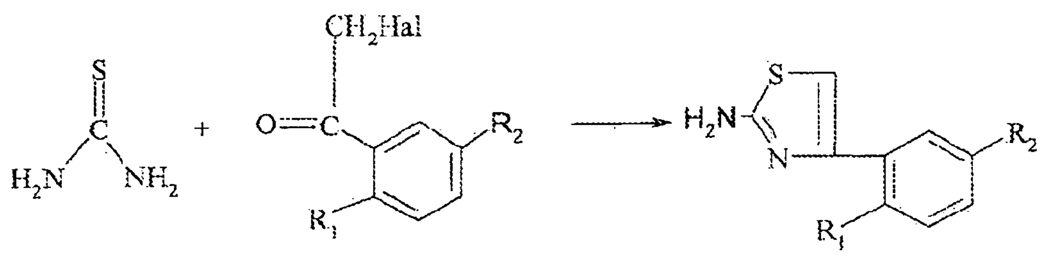

4

(III)

Les substituants $R_1$ et $R_2$ tels que définis pour (Ia) ; Hal représente un atome d'halogène, de préférence le brome, le chlore ou l'iode.

Les cétones halogénées de formule 4 peuvent être préparées par des procédés connus de l'homme de l'art. Par exemple, les bromocétones peuvent être obtenues par action du brome, du bromure cuivrique ou du phényltriméthylammoniumtribromure (PTT) sur un dérivé d'acétophénone de formule :

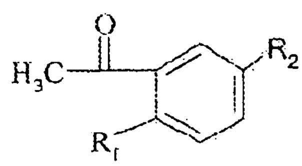

5

, dans un solvant organique tel que l'acétate d'éthyle, un solvant chloré ou leur mélange ou encore un alcool.

Lorsque le dérivé acétophénone de formule 5 n'est pas disponible commercialement, on peut le préparer par différentes méthodes :

- une réaction de Friedel-Crafts sur le benzène substitué par $R_1$ et $R_2$ que l'on fait réagir sur le chlorure d'acétyle ou l'anhydride acétique, en présence d'un acide de Lewis tél que $AlCl_3$ ou $TiCl_4$, par exemple;
- l'action du chlorure d'acétyle en présence de Palladium sur le benzène substitué par $R_1$ et $R_2$ après déprotonation du benzène, par exemple par action du butyllithium puis addition du chlorure de zinc ou de l'iodure de manganèse. Ce mode opératoire est utilisable pour préparer un dérivé d'acétophénone de formule 5 dans laquelle $R_2$ - $(C_1\text{-}C_4)$ perfluoroalkyle ;
- un réarrangement de Fries : à partir d'un dérivé d'acétoxybenzéne de formule :

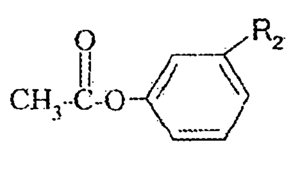

6

par action d'un acide de Lewis, on obtient un dérivé d'hydroxyacétophénone de formule :

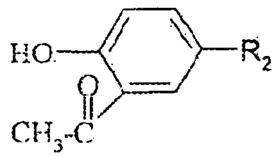

7

La fonction hydroxyle correspond à un groupement $R_1$ que l'on peut transformer dans une étape ultérieure en un groupement -O-Z tel que ($C_1$-$C_8$)alcoxy, trifluorométhoxy, trifluoroéthoxy, allyloxy, ($C_3$-$C_8$)cycloalkylmethoxy, ($C_3$-$C_8$) cycloalkyloxy.

Les dérivés du benzène substitués par $R_1$ et $R_2$ sont disponibles commercialement ou preparables par des méthodes connues de l'homme de l'art.

Par exemple, pour préparer un composé dans lequel $R_1$ est un groupement -O-Z tel que défini ci-dessus, on opère de la façon suivante :

SCHEMA 18

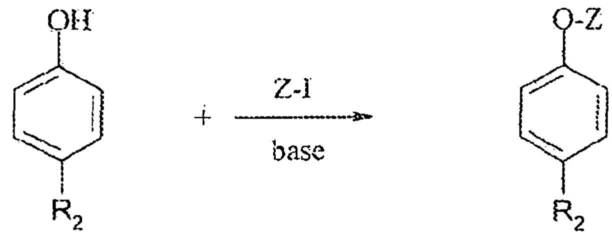


8 9

On peut également substituer un dérivé d'halogénobenzéne selon le schéma ci-après :

SCHEMA 19

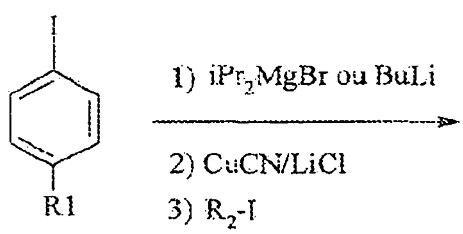


Dans le cas particulier où $R_2$ représente un ($C_1$-$C_4$)perfluoroalkyle, on peut également procéder selon le schéma réactionnel ci-après :

SCHEMA 20

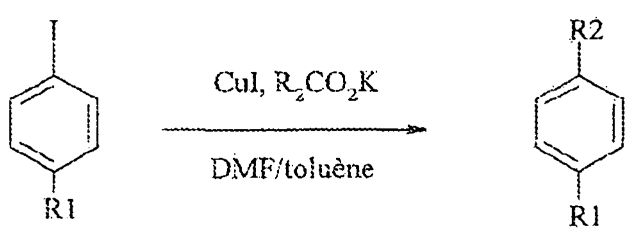


10 11

Des acides de formule (II) dans laquelle $R_3$ est un groupe éther que défini pour (Ia) par a) sont décrits, notamment dans Arch. Pharm., 1962, 295, 292-304 ; Eur. J. Med. Chem., 1994, 26 (9), 675-686 ; J. Med. Chem., 2002, 45 (16), 3406-3417 ; et dans des demandes de brevet : WO-02/53534 ; WO-01/00206 : WO-96/21656 ; WO-00/39087 ; EP-A-62504 ; EP-A-393607 ; EP-A-997465.

Les acides de formule (II) dans laquelle $R_3$ est un groupe b) ou c), tel que défini pour (Ia) sont généralement nouveaux. Les esters d'acide 4-((1-méthylpipéridin-4-yl) méthylbenzoïque et 4-((1-éthylpipéridin-4-yl)méthylbenzoïque sont décrits dans Pesticide Sciences, 1995, 44 (1), 96-102.

Les acides de formule (II) dans laquelle $R_3$' représente $R_3$ qui est un groupe e) ou f) tels que définis pour (Ia) sont généralement nouveaux. Des composés de formule (II) dans laquelle $R_3$ représente un groupe e1) sont décrits dans la demande de brevet WO-98/56760 et dans le brevet US 5 411 984.

Les 2-aminothiazoles de formule (III) sont généralement connus, notamment par les documents suivants : EP-A-819681, EP-A-44442 ou Indian J. Chem., section B, 1987, 26B (3), 287-289.

Les composées intermédiaires de formule (IV) à savoir les composés de formule X, XIII, XIV, XVII, XVIII, XIX, XXVI, XXVII, XXVII bis, XXIX, XXXIII, XXXIIIbis, XXXIV, XXXIV bis, XXXVIII, XXXX, XXXXIX sont nouveaux et constituent un objet ultérieur de la présente invention.

Par ailleurs, les composés intermédiaires de formule (IV) dans laquelle le groupement $R'_3$ comporte une fonction amine protégée, à savoir groupe -NGp au lieu et place d'un groupe $-NR_5$ sont également nouveaux.

Ainsi, l'invention a également pour objet les composés de formule :

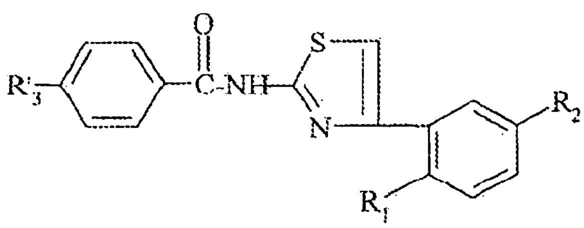
(IV)

dans laquelle:

- $R_1$ et $R_2$ sont tels que définis pour les composés de formule (Ia);
- $R'_3$ représente une groupe choisi parmi :

. -OPg, Pg étant un groupe protecteur tel que tert-butyle, benzoyle ou arylsulfonyle (phénylsulfonyle, tolylsulfonyle ou naphtylsulfonyle) ;
. $-O-(C_1-C_3)$alk-Q, Q étant un groupe diméthoxyméthyle, diéthoxyméthyle ou formyle ;
. $-O-(C_2-C_4)$alk-OX, X représentant un atome d'hydrogène, un groupe tétrahydropyranyle ou un groupe $SO_2R'$, R' étant un groupe méthyle ou tolyle ;
. $-(C_1-C_3)$alk-Q ;
. $-(C_1-C_4)$alk-Hal, Hal représentant un atome d'halogéne ;
. -I :
. -COOH ; -COOR avec R représentant un atome d'hydrogène, un $(C_1-C_4)$alkyle, ou un benzyle non substitué ou substitué sur le phényle par un groupe méthoxy ;
. $-CONH-(C_1-C_3)$alk-Q ;

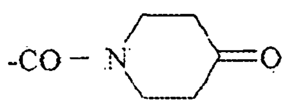
;

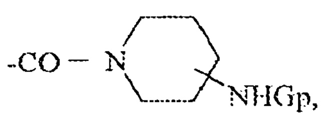
,

Gp représentant un groupe protecteur de l'azote tel que Fmoc, $(C_1-C_4)$alcanoyle, benzoyle, benzyloxycarbonyle , *tert*-butoxycarbonyle ;

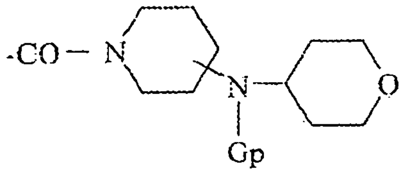

. a)

al) $-O-(C_2-C_4)alk-A'$;
a2) $-O-(C_1-C_4)alk-B'$ ;
a3) -O-E';

. b) $-(C_1-C_4)alk-A'$;
. c)-B' ;
. d)

d1) $-(C_1-C_4)alk-R_4-(C_2-C_3)alk-A'$;
d2) $-(C_1-C_4)alk-NR_4-(C1-C_3)alk-B'$;

. e)

e1) $-CONR_4-(C_2-C_4)alk-A'$;
e2) $-CONR_4-(C_2-C_4)alk-B'$;
e3) $-CONR_4-E'$;

. f)

f1) $-CO-D-(C_1-C_2)alk-A'$;

-2) -CO-G-A' ;

f3)

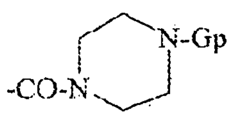

;

f4)

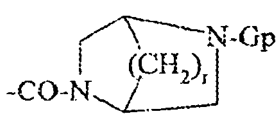

;

f5)

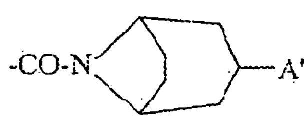

;

dans laquelle:

- A', B', E' représentent respectivement les groupes A, B, E tels que définis pour (1a) dans lesquels $R_5$ est remplacé par Gp ;
- Gp représenae un groupe protecteur de l'azote tel que : Boc, Fmoc, $(C_1-C_4)$alcanoyle, benzyloxcar-

bonyle ou benzyle.

Les préparations et exemples qui suivent illustrent la préparation de certains composés conformes à l'invention. Les numéros des composés exemplifiés l'envoient à ceux donnés dans les tableaux ci-après. Dans la description, les abréviations ci-après sont utilisées.

TA : température ambiante
déc, : décomposition
DCM : dichlorométhane
DMF : diméthylformamide
Nets : triéthylamine
BO.P : benzotriazol-1-yl-oxy-tris-(diméthylammo)phosphonium hexafluorophosphaté
Boc : *ter*butyloxycarbonyle
éther : éther éthylique
MTBE : méthyltertbutyléther
Me : méthyle
Et : méthyle
Pr : propyle
Bu : butyle
Pn Pentyle
kleex : hexyle
DIPEA : Diisopropyléthylamine
THP: -tétrahydropyran-4-yle.

Les composés sont caractérisés par:

Les spectres dé résonance magnétique du proton (RMN $^1H$) sont enregistrés à 200 MHz dans du DMSO-$d_6$, en utilisant le pic du DMSO-$d_6$ comme référence. Les déplacements chimiques $\delta$ sont exprimés en parties par million (ppm). Les signaux observés sont exprimés ainsi : s: singulet; se : singulet élargi ; d : doublet : d.d : doublet dédoublé ; t : triplet ; td : triplet dédoublé ; q : quadruplet ; m : massif : mt : multiplet.

Pour toues les composés synthétisés dans les Préparations et les Exemples qui suivent, on vérifie que les spectres RMN enregistrés sont conformes à la structure attendue.
Les composés selon l'invention sont analysés par couplage LC/UV/MS (chromatographie liquide/détection UV/spectométrie de masse). On mesure le pic moléculaire ($MH^+$) et le temps de rétention (t).
On utilise un appareil commercialisé par Waters et une colonne de 2,1 x 50 mm, avec des particules de 3,5 $\mu$m, à température ambiante, débit 0,4 mL/minute.
L'éluant est composé comme suit :

- solvant A : 0,005 % d'acide trifluoroacétique (TFA) dans l'eau
- solvant B : 0,005 % de TFA dans l'acétonitrile.

| Gradient | Temps (minutes) | % B |
|---|---|---|
| | 0 | 0 |
| | 10 | 90 |
| | 15 | 90 |
| | 15,5 | 0 |
| | 20 | 0 |

La détection UV est effectuée à 210 $\pm$ 8 nm et la détection de masse est effectuée après électroionisation (en anglais electrospray ionisation ou ESI) en mode positif.

Préparation des intermédiaires de formule (Im

Préparation 1.1

4-(2-Méthoxy-5-propoxyphényl)-1,3-thiazol-2-amine.

A) 1-(2-Hydroxy-5-propoxyphényl)éthanone.

Dans un ballon de 500 mL sont placés 10 g de 2,5-dihydroxyacétophénone en suspension dans 100 mL d'acétone, 9,14 g de $K_2CO_3$ anhydre sont ajoutés suivis par 12,4 g d'iodure de propyle. Le milieu réactionnel est chauffé à reflux pendant 30 heures. Après retour à température ambiante, le milieu est filtré sur Cérite puits concentré. L'huile brune obtenue est reprise dans lacet, filtrée, lavée à l'eau, avec une solution d'HCl 2M, puis avec une solution saturée de NaCl. La phase organique est évaporée pour donner une pâte noire. La pâte est reprise dans le chloroforme et filtrée. Le milieu est concentré pour donner 11,4 g d'un solide noir. Ce dernier est repris dans l'éthanol absolu. La solution est placée 10 minutes au congélateur, un solide précipite, il est collecté par filtration. Le filtrat est concentré, repris dans l'éthanol, refroidi au congélateur puis filtré à nouveau. Cette opération est répétée 4 fois pour donner 8,35 g du composé attendu sous forme d'une poudre. B) 1-(2-Méthoxy-5-propoxyphényl)éthanone.

A une solution de 35 g du solide précédent dans 350 mL de DMF, on ajoute 49,8 g de $K_2C0_3$, puis 22,4 mL d'iodure de méthyle. Le milieu réactionnel est chauffé pendant 12 heures à 60˚C. Après retour à température ambiante, le milieu est filtré sur Célite®, dilué dans l'éther et lavé avec une solution d'HCl 2M. La phase aqueuse est extraite 2 fois dans l'éther. Les phases organiques rassemblées sont lavées avec une solution de soude diluée, puis lavées à l'eau 2 fois et avec une solution saturée de NaCl. La phase organique est séchée sur $MgSO_4$, puis évaporée pour donner 35,55 g d'une huile brune. L'huile est distillée sous pression réduite à 1115˚C, pour donner 32,8 g du composé attendu sous forme d'une huile. C) 2-Bromo-1-(2-méthoxy-5-propoxyphényl)éthanone.

A une solution de 16,4 g de l'huile obtenue à l'étape précédente dans 100 mL de méthanol, on ajoute au goutte à goutte 4,8 mL de brome. Le milieu est agité 30 minutes à température ambiante, puis évaporé. L'huile obtenue est reprise dans le dichlorométhane, lavée 3 fois dans l'eau, puis séchée sur $MgSO_4$ et évaporée pour donner 24,5 g d'une huile brune. D) 4-(2-Méthoxy-5-propoxyphényl)-1,3-thiazol-2-amine.

A une solution de 42 g de la bromocétone préparée à l'étape précédente dans 200 mL d'éthanol, sont ajoutés 24,5 g de thiourée. Le milieu est porté à reflux pendant 1 heure 30 minutes. Le milieu est alors placé au réfrigérateur pendant 12 heures, puis filtré. Le solide ainsi collecté est rincé avec un peu d'éthanol froid, puis à l'éther. On récupère 25 g de bromhydrate.

Le solide est mis en suspension dans un mélange eau/dichlorométhane et on effectue un retour à la base par addition de soude. La phase aqueuse est extraite 2 fois dans le dichlorométhane. Les phases organiques rassemblées sont séchées sur $MgSO_4$ puis évaporées. L'huile obtenue est chromatographiée sur gel de silice pour donner 12 g du produit attendu sous forme de poudre. F = 76˚C

Préparation 1.2

4-(5-Butyl-2-méthoxyphényl)-1,3-thiazol-2-amine

A) 4-Butylphényl acétate.

Une solution de 10g de 4-n-butylphénol, 10 mL d'$Ac_2O$ et 8 mL de pyridine est agitée à reflux dans 10 mL de dichlorométhane. Après 2 heures, le milieu est refroidi à température ambiante, dilué dans le dichlorométhane, lavé à l'eau, lavé avec une solution d'HCl 1M, lavé dans une solution de $CuSO_4$ saturée, lavé à l'eau et séché sur $MgSO_4$. Après évaporation, on récupère 10,8 g du composé attendu sous forme d'une huile.

B) 1-(5-Butyl-2-hydroxyphényl)éthanone

A 5 g de l'huile précédente dans un ballon de 100 mL, on ajoute en plusieurs fois 3,22 g d'$AlCl_3$. Le milieu est chauffé à 130˚C pendant 1 heure. Après retour à température ambiante, une solution d'eau glacée acidifiée par du HCl 35 % est coulée sur le brut réactionnel. Le milieu est placé dans un bain à ultrasons. On additionne de l'AcOEt pour obtenir, après 15 minutes, la solubilisation du milieu. La phase aqueuse est extraite 3 fois dans fAcOEt, les phases organiques sont lavées à l'eau, puis avec une solution saturée de NaCl. Après séchage sur $MgSO_4$ et évaporation, on récupère 4,5 g d'une huile jaune.

C) 1-(5-Butyl-2-méthoxyphényl)éthanone

A une solution d'1 g de l'huile précédente dans 10 mL de DMF, on ajoute 1,44 g de $K_2CO_3$ puis 0,648 mL d'iodure de méthyle. Le milieu est chauffé à 60°C pendant une nuit. Après retour à température ambiante, le milieu est filtré sur Célite®, dilué dans l'éther et lavé avec une solution d'HCl 2M. La phase aqueuse est extraite 2 fois dans l'éthér. Les phases organiques rassemblées sont lavées avec une solution de soude diluée, puis lavées à l'eau deux fois et avec une solution saturée de NaCl. La phase organique est séchée sur $MgSO_4$, puis évaporée pour donner 1,27 g d'une huile brune. L'huile est purifiée par chromatographie pour donner 0,66 g du composé attendu.

D) 4-(5-Butyl-2-méthoxyphényl)-1,3-thiazol-2-amine.

A une solution de 0,66 g du produit de l'étape précédente dans 10 mL de méthanol, on additionne 0,19 mL de brome. Le milieu est agité pendant 10 minutes, puis évaporé et repris dans le dichlorométhane. La phase organique est lavée 3 fois à l'eau, puis séchée sur $MgSO_4$. On récupère 0,79 g du produit attendu après évaporation. Ce composé est dissout dans 5 mL d'éthanol en présence de 0,46 g de thiourée et le milieu est porté à reflux pendant 2 heures 30 minutes. Un solide précipite lors du retour à température ambiante. Le solide ainsi collecté est rincé avec un peu d'éthanol froid, puis à l'éther. On récupère ainsi 0,6 g du bromhydrate.
Le solide est mis en suspension dans un mélange eau/dichlorométhane et on effectue un retour à la base par addition de soude. La phase aqueuse est extraite 2 fois dans le dichlorométhane. Les phases organiques rassemblées sont séchées sur $MgSO_4$, puis évaporées pour donner 0,34 g d'une huile jaune qui cristallise lentement. Les eaux mères sont évaporées, puis agitées dans un mélange eau/dichlorométhane et on effectue un retour à la base par addition de soude. La phase aqueuse est extraite 2 fois dans le dichlorométhane. Les phases organiques rassemblées sont séchées sur $MgSO_4$, puis évaporées. L'huile obtenue est chromatographiée sur gel de silice pour donner 0,18 g du composé attendu. F = 48°C.

Préparation 1.3

4-(5-Cyclohexyl-2-méthoxyphényl)-1,3-thiazol-2-aminel-Cyclohexyl-4-méthoxybenzene.

A) A une solution de 5 g de 4-cyclohexylphénol dans 60 mL de DMF, on ajoute 7,84 g de $K_2CO_3$, puis 3,53 mL d'iodure de méthyle. Le milieu est chauffé à 60°C pendant une nuit. Après retour à température ambiante, le milieu est filtré sur Célite®, puis dilué dans l'éther et hydrolysé à l'eau. La phase aqueuse est acidifiée, puis extraite dans 3 fois 50 mL d'éther. Les phases organiques rassemblées sont lavées avec une solution de soude diluée, puis lavées à l'eau 2 fois et avec une solution saturée de NaCl. La phase organique est séchée sur $MgSO_4$, puis évaporée pour donner 4,31 g du composé attendu sous forme d'un solide. F = 67°C.
B) 1-(5-Cyclohexyl-2-méthoxyphényl)éthanone. Une suspension de 5,6 g d'$AlCl_3$ dans 40 mL de dichlorométhane est refroidie à -10°C. On ajoute 3 mL d'AcCl et 4 g du composé de l'étape précédente. Le milieu est agité 1 heure à -10°C, puis versé dans un bécher contenant de la glace mélangée à de l'HCl à 35%. Après décantation, les phases organiques rassemblées sont séchées sur $MgSO_4$ puis évaporées pour donner 4,54 g du produit attendu.
C) 4-(5-Cyclohexyl-2-méthoxyphényl)-1,3-thiazol-2-amine.

A une solution de 4,5 g du produit de l'étape précédente dans 25 mL de méthanol, on ajoute, au goutte à goutte 1,16 mL de brome. Le milieu est agité 30 minutes à température ambiante, puis devient très visqueux. 5 mL de méthanol sont rajoutés, suivis de 3,23 g de thiourée. Le milieu est chauffé à reflux pendant 2 heures. Après retour à température ambiante, un solide précipite. Le solide est collecté, puis rincé avec un peu de méthanol froid. Le solide est mis en suspension dans un mélange eau/dichlorométhane et on effectue un retour à la base par addition de soude. La phase aqueuse est extraite 2 fois dans le dichlorométhane. Les phases organiques rassemblées sont séchées sur $MgSO_4$ puis évaporées pour donner 3,33 g du composé attendu sous forme d'un solide. F = 113°C.

Préparation 1.24

4-(5-Pentafluoroethyl-2-methoxy-phenyl)-thiazol-2-ylamine

A) 1-Methoxy-4-pentafluoroethyl-benzene

Sous atmosphère inerte, dans un tricol de 500 ml équipé d'un Dean-Starck et d'un réfrigérant, on introduit 8,3 g de pentafluoropropionate de potassium et 9,8 g de CuI. On ajoute 90 ml de DMF et 1.0 ml de toluène, Le milieu est chauffé à 140°C sous azote, 80 ml de toluène sont distillés. Le milieu est alors refroidi à TA puis déoxygéné avec un bullage à l'azote. On ajoute ensuite 6 g de iodoanisolé puis on chauffe à 155°C pendant 20 h. Après retour à TA, le milieu est dilué dans 200 ml d'un i mélange eau / éther éthylique. Le milieu est alors filtré sur célite®, La phase organique est lavée 3 fois à l'eau, séchée sur $MgSO_4$ puis évaporée pour donner 4,3 g d'une huile marron.

B) 1-(2-Methoxy-5-pentafluoroethyl-phenyl)-ethanone A une solution de 3,5 g de 1-Methoxy-4-pentafluoroethyl-benzene dans 50 ml de THF anhydre, on additionne, à -70°C, 7,4 ml de BuLi à 2,5 M dans l'hexane. Le milieu est agité 30 min à -70°C puis 45 min à 0°C. On additionne alors 15,5 ml d'une solution de chlorure de zinc 1 M dans l'éther. Après 10 min d'agitation à 0°C, 1,33 ml de chlorure d'acétyle est ajouté. Le milieu est alors désoxygéné a l'azote et 332 mg de palladium benzyl(chloro)-bis(triphénylphosphine) dans 5 ml de THF anhydre sont introduites. Le milieu est agité 2 h 30 à 0°C: puis 72 heures à TA. Le milieu est coulé sur une solution d'HCl 2,5 M puis extrait dans l'éther, à phase organique est lavée au $NaHCO_3$ à 5 % dans l'eau, à l'eau puis avec une solution saturée de NaCl. Après séchage sur $MgSO_4$ et évaporation, le brut est purifié par flash-chromatographie sur silice pour donner 2,25 g d'un solide blanc.

F=47°C.

C) 4-(2-Methoxy-5-pentafluoroethyl-phenyl)-thiazo1-2-ylamine A une solution de 2,25 g du produit obtenu a l'étape précédente dans 10 ml de méthanol, on additionne 0,5 ml de brome en solution dans 8 ml de méthanol. Le milieu est agité pendant 10 min puis évaporé et repris dans le dichlorométhane. La phase organique est lavée 3 fois à l'eau puis séchée sur $MgSO_4$. On récupère 2,63 g du produit attendu après évaporation. Ce composé est dissout dans 1 5 ml de méthanol en présence de 1,25 g de thiourée et le milieu est porté à reflux pendant 2 h. Un solide précipite lors du retour à TA. Le solide ainsi collecté est rincé à l'éther éthylique. Le solide est mis en suspension dans un mélange eau / dichlotométhane et on effectue un retour à la base par addition de soude. La phase aqueuse est extraite 2 fois dans le dichlorométhane. Les phases organiques rassemblées sont séchées sur $MgSO_4$ puis évaporées pour donner 1,63 g d'un solide jaune.

F=125°C

Préparation 2.1

N-(Boc),N-(tétrahydro-2*H*-pyran-4-yl)pipéridin-4-aminé

A) 1-Benzyl-N-(tétrahydro-2*H*-pyran-4-yl)pipéridin-4-amine (XXXXVI).

Sous azote sec, on place 19 g de 4-amino-1-benzylpipéridine dans 50 mL de 1,2-dichloroéthane, on ajoute 10 g de tétrahydropyran-4-one dans 20 mL de 1,2-dichloroéthane ; après 10 minutes d'agitation, on ajoute 29,6 g de $NaBH(OAc)_3$, puis on laisse sous agitation pendant une journée. On ajoute au milieu réactionnel une solution de $Na_2CO_3$ à 10 % et de l'AcOEt, puis on décante. La phase organique est lavée par une solution de $Na_2CO_3$ à 10 % puis une solution saturée de NaCI puis séchée sur $MgSO_4$ et évaporée sous vide. On obtient 23,4 g du composé attendu. F = 60°C.

B) 1-Benzyl-N-Boc,-N-(tétrahydro-2*H*-pyran-4-yl)pipéridin-4-amine

On place 14,92 g du composé obtenu à l'étape précédente dans 100 mL d'AcOEt, puis on refroidit par un bain de glace et on ajoute 12,46 g de $(Boc)_2O$ dans 30 mL d'AcOEt. On chauffe à 50°C pendant 4 jours, puis on maintient sous agitation 2 jours à température ambiante. Le milieu réactionnel est lavé à l'eau (3 fois), séché sur $MgSO_4$ puis évaporé. L'huile obtenue cristallise, le solide est trituré dans du pentane, filtré et séché à 60°C sur $P_2O_5$. On obtient 15,9 g du composé attendu. F =104°C.

C) N-(Boc),N-(tétrahydro-2*H*-pyran-4-yl)plpéridin-4-amine.

On place 15,8 g du composé de l'étape précédente dans 100 mL de MeOH avec 1 g de Palladium sur charbon à 10 % et on hydrogène sous pression atmosphérique à 30°C pendant une journée. On filtre le milieu sur Célite® puis on rince par MeOH. Après évaporation du filtrat, on obtient 11,1 g du composé attendu qui cristallise. F = 125°C.

Préparation des intermédiaires de formule (II)

Préparation 3.1

Acide 4-(2,2-diéthoxyéthoxy)benzoïque

(II) : $R_3$' = 4-$OCH_2CH(OEt)_2$

A) Ester méthylique de l'acide 4-(2,2-diéthoxyéthoxy)benzoïque
On chauffe à 100°C pendant 5 minutes un mélange contenant 10 g de 4-hydroxybenzoate de méthyle et 22,71 g de $K_2CO_3$ dans 100 mL de THF, on revient à température ambiante et on ajoute 15,54 g de 2-bromo-1,1-diéthoxyéthane, et on laisse sous agitation 2 heures à température ambiante, puis 32 heures à 100°C, puis on laisse revenir à température ambiante. On filtre le minéral, puis on rince au DMF. Le filtrat est évaporé, puis repris au DCM, on lave à l'eau (3 fois), puis par une solution saturée de NaCl, on sèche sur $MgSO_4$ et on évapore. On obtient 16,68 g du composé attendu (supérieur à la masse théorique).
B) Acide 4-(2,2-diéthoxyéthoxy)benzoïque.
On place 8,08 g de l'ester obtenu à l'étape précédente dans 50 mL de méthanol avec 16 mL de NaOH 5N et on laisse sous agitation pendant 6 heures. Après évaporation du solvant, on reprend à l'eau puis on ajoute HCl 1,2 M jusqu'à pH = 3; on filtre et rince à l'eau pour obtenir un précipité qui est séché sous vide. Le filtrat est extrait 2 fois au DCM, puis séché sur $MgSO_4$. Au total, on obtient 7,29 g du composé attendu.

Préparation 3.2

Acide 4-((4-(N-Boc)tétrahydro-2*H*-pyran-4-yl)amino)pipéridin-1-yl)carbonyl) benzoïque, sel de triéthylamine.

(II), $NEt_3$ : (XXXXV) = $R'_3$ = 4 – 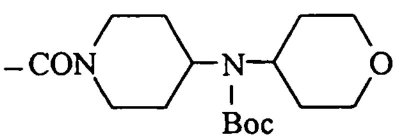

A) Ester méthylique de l'acide 4-((4-N-Bocamino)pipéridin-1-ylcarbonyl)benzoïque.
On laisse sous agitation à température ambiante pendant 4 jours un mélange contenant 5,39 g du monoester méthylique de l'acide téréphtalique, 40 mL de $CH_3CN$, 6 mL de $NEt_3$, 14,8 g de BOP et 5 g de 4-(N-Bocamino) pipéridine. On dilue le milieu réactionnel dans AcOEt, puis on lave 4 fois par une solution de $Na_2CO_3$ à 10%, puis 4 fois à l'eau. On sèche sur $MgSO_4$, puis on concentre à sec et on reprend dans un mélange $Et_2O$/cyclohexane. On filtre, puis sèche pour obtenir 6,66 g du composé attendu. F = 128-130°C.
B) Chlorhydrate de l'ester méthylique de l'acide 4-((4-aminopipéridin-1-yl) carbonyl)benzoïque.
On place sous agitation à température ambiante pendant 2 heures 17,66 g du composé de l'étape précédente dans 120 mL de solution d'HCl 4M dans le dioxane. On ajoute de l'éther, puis on prolonge l'agitation pendant 1 heure. On filtre, lave à l'éther, puis sèche pour obtenir 15 g du composé attendu. F = 236-238°C.
C) Ester méthylique de l'acide 4-((4-(tétrahydro-2*H*-pyran-4-ylamino)pipéridin-1-yl)carbonyl)benzoïque.
On place sous agitation pendant une nuit un mélange contenant 15 g du composé de l'étape précédente dans 50 mL de DMF, 5 g de tétrahydro-4*H*-pyran-4-one et 4 mL de $NEt_3$. On ajoute 1,36 mL d'AcOH, 12,7 g de $NaBH(OAc)_3$ et 50 mL de DMF et on maintient sous agitation pendant 3 heures. On concentre le milieu réactionnel à sec, puis on reprend par du DCM, on lave par une solution de $Na_2C0_3$ à 10%. On décante, puis on lave la phase organique à l'eau (3 fois), sèche sur $MgS0_4$ et concentre à sec. On obtient 9,5 g du composé attendu sous forme d'un solide. F =129°C.
D) Ester méthylique de l'acide 4-((4-((N-Boc)tétrahydro-2*H*-pyran-4-ylamino) pipéridin=l-yl)carbonyl)benzoïque.
On chauffe à reflux, pendant 10 heures, un mélange contenant 3,16 g du composé de l'étape précédente dans 12 mL de DCM en présence de 4,5 g de $Boc_20$ et de 1,65 mL de $NEt_3$. Après refroidissement du milieu réactionnel, on lave par une solution tampon à pH = 2 (3 fois), puis à l'eau (3 fois). On sèche la phase organique sur $MgS0_4$, puis on concentre à sec pour obtenir 4,63 g du composé attendu. E) Acide 4-((4-(N-Boc)tétrahydro-2*H* pyran-4-yl) amino)pipéridin-1-yl) carbonyl) benzoïque, sel de $NEt_3$.
On laisse sous agitation, pendant 48 heures, 4 g du composé de l'étape précédente dans 100 mL de NaOH 5N dans le méthanol. On élimine le MeOH par évaporation, on reprend le produit brut dans l'eau, on lave la phase aqueuse au DCM puis on l'acidifie par HCl jusqu'à pH = 3. On filtre et lave à l'éther, puis on reprend le solide obtenu par DCM/eau/$NEt_3$. La phase aqueuse est extraite au DCM, puis on sèche sur $MgSO_4$ et concentre à sec pour obtenir 700 mg du composé attendu sous forme solide. $MH^+$ = 433,3 ; t = 6,71.
On prépare l'acide libre correspondant : F = 190°C, $MH^+$ = 433,3 ; t = 6,70.

Préparation 3.3

Acide 4-(4-((R)-3-(N-Boc)-pyrrolidin-1-yl)piperidine-1-carbonyl) benzoïque

(II) R3' = 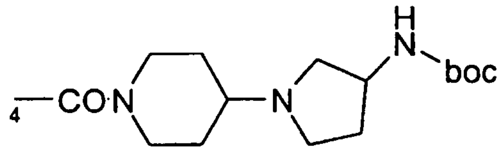

A) *tert*-butyl ester d'acide (1-(-Benzyl-piperidine-4-yl)-pyrrolidin-3-yl)-carbamique A une solution de 4,6 g de (3R)-3-(*tert*-butoxycarbonyl-amino)pyrrolidine dans 5 ml de dichloroéthane, on additionne 4,66 g de 1-benzyl-4-pipéridone dans 6 ml de dichloroéthane. Après 20 min à TA, on ajoute 7,31 g de $NaBH(OAc)_3$ en maintenant le milieu à une température inférieure à 20°C, puis 20 ml de dichloroéthane pour dissoudre le milieu qui prend en masse. Le milieu est agité à TA pendant 24 h, hydrolysé par addition d'une solution aqueuse de $Na_2CO_3$ à 10 % et dilué dans l'acétate d'éthyle. Après décantation, la phase organique est lavée dans une solution aqueuse de $Na_2CO_3$ à 10 % puis dans une solution saturée de NaCl puis séchée sur $MgSO_4$, puis évaporée. Le brut est trituré dans l'éther, filtré, rincé à l'éther puis séché pour donner 7,38 g d'un solide blanc.
F = 118°C
B) (*tert*-butyl ester d'acide 1-piperidine-4-yl-pyrrolidin-3-yl)-carbamique
Une solution de 7,37 g du composé précédemment décrit dans 50 ml de méthanol est hydrogénée à pression atmosphérique et à TA en présence d'1 g de Pd/C 10 % pendant 12 h. Le milieu est filtré sur Célite®, le solide est rincé au méthanol. Après évaporation du filtrat, on obtient 5,2 g d'une huile qui se solidifie après trituration, utlisée telle quelle pour l'étape suivante.
C) Ester méthylique de l'acide 4-(4-((R)-3-(N-Boc) pyrrolidin-1-yl)piperidine-1-carbonyl) benzoïque
A une solution de 4,75 g du composé obtenu à l'étape précédente dans 35 ml d'acétonitrile, on ajoute 3,8 g de monoester méthylique de l'acide téréphtalique, suivis de 9,4 g de BOP, puis de 2,2 g de triéthylamine. Le milieu est agité à TA pendant 24 h puis concentré. Le brut est repris dans l'acétate d'éthyle, lavé deux fois dans l'eau puis deux fois dans une solution aqueuse de $Na_2CO_3$ à 10 % puis dans une solution saturée de NaCl. Après séchage de la phase organique sur $MgSO_4$ et évaporation, on récupère 8,62 g de brut qui est trituré dans un mélange éther / acétate d'éthyle. Après filtration, on collecte 5,28 g du produit attendu.
D) Acide 4-(4-((R)-3-(N-Boc)-pyrrolidin-1-yl)piperidine-1-carbonyl) benzoïque
A une solution de 5,05 g de l'ester obtenu à l'étape précédente dans 20 ml de méthanol, on ajoute 1,17 g de soude. Le milieu est agité à TA pendant 12 h puis évaporé et repris dans l'eau. La phase aqueuse est lavée à l'éther puis acidifiée jusqu'à pH = 5 et extraite deux fois dans l'acétate d'éthyle. La phase organique est séchée sur $MgSO_4$ puis concentrée. La phase aqueuse est évaporée puis séchée par distillation azéotropique avec de l'éthanol. Le résidu est repris dans l'éthanol, filtré sur Célite® et le filtrat est évaporé. Les deux bruts sont réunis pour donner 3,65 g de l'acide attendu.
$MH^+$ = 418 à t = 4,7 mn

Préparation des intermédiaires de formule (IV)

Préparation 4.1.1 .

(XXXVIII): $R_1$ = 2-OMe ; $R_2$ = 5-nBu ;

$R'_3$ = 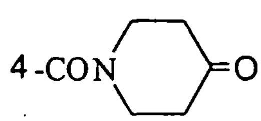

N-(4-(5-Butyl-2-méthoxyphényl)-1,3-thia2ol-2-yl)-4-((4-oxopipéridin-1-yl)carbonyl)benzamide.
On place sous agitation à TA pendant 3 jours une suspension contenant 0,86 g de l'aminothiazole de la préparation 1.2, 1 g d'acide 4-((4-oxopipéridin-1-yl)carbonyl)benzoïque et 0,6 mL de $NEt_3$ dans 8 mL d'acétonitrile et on ajoute 1,9 g de BOP. Le précipité formé est filtré puis lavé par 0,3 mL de $CH_3CN$ puis 1 mL d'éther, on obtient 0,85

g du composé attendu.
F = 184°C

Le composé (XXXVIII) peut être également préparé selon la méthode suivante.

A une solution de 10,4 g de l'acide préparé par la méthode décrite en 4.2.1 dans 50 ml d'acétonitrile, on ajoute 4,7 g de chlorhydrate de 4-pipéridone monohydrate puis 15,7 g de BOP. A 0°C, on additionne 13,3 ml de DIPEA et on laisse remonter la température à TA. Après 24 h d'agitation à TA, le milieu réactionnel est filtré, le solide est rincé à l'acétonitrile puis le filtrat est concentré et repris dans le dichlorométhane. La phase organique est lavée dans $Na_2CO_3$ à 10 % puis dans HCl 0,5 M puis dans une solution saturée de NaCl. Après séchage de la phase organique sur $MgSO_4$, on récupère 11,33 g du produit attendu.

En procédant de manière similaire, on a préparé les composés décrits dans le tableau ci-après.

TABLEAU 2

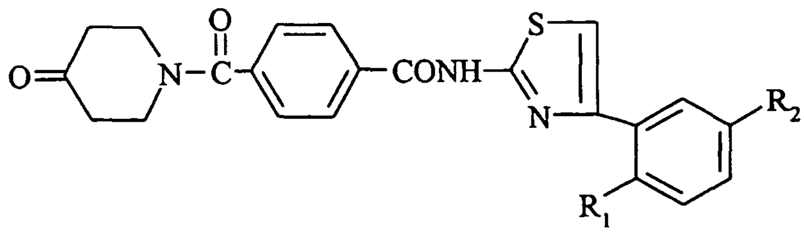

(XXXVIII)

| Préparations n° | $R_1$ | $R_2$ | Caractérisation |
|---|---|---|---|
| 4.1.1 | MeO | nBu | F = 184°C |
| 4.1.2 (composé n°242) | MeO | nPrO | F = 196°C |
| 4.1.3 | MeO | Me | F = 183°C |
| 4.1.4 | MeO | Et | F = 180°C |
| 4.1.5 | MeO | nPr | F = 172°C |
| 4.1.6 (composé n°243) | MeO | Cyclohexyle | F = 186°C déc. |
| 4.1.7 (composé n°244) | EtO | Cyclohexyle | F = 236°C |
| 4.1.8 | EtO | Et | F = 160°C |
| 4.1.9 | MeO | Cyclopentyle | $MH^+$ = 504,5 t = 10,55 |
| 4.1.10 | -OEt | -nBu | MH = 506 t = 10,3 |

Préparation 4.2.1 .

Acide 4-(((4-(5-butyl-2-méthoxyphényl)-1,3-thiazol-2-yl)amino)carbonyl) benzoïque

(XXXIIIbis) : $R_1$ = 2-OMe; $R_2$ = 5-nBu; $R'_3$ = 4-COOH

On mélange 5 g d'aminothiazole de la Préparation 1.2, 4,12 g de 4-carboxybenzoate de méthyle, puis 1,85 mL de $NEt_3$, 25 mL de $CH_3CN$ et 10,13 g de BOP et on laisse 4 jours sous agitation à température ambiante. On filtre le précipité formé, puis on le lave à l'acétonitrile. Le précipité est ensuite repris par un mélange d'AcOEt et d'une solution saturée de $Na_2C03$. Après décantation, la phase aqueuse est extraite au DCM. Les phases organiques réunies sont séchées sur $MgS0_4$ et concentrées. On obtient 3,18 g du composé attendu sous forme d'un ester méthylique. Cet ester est mis en suspension dans 34 mL de MeOH, puis on ajoute 5,3 mL de soude 5N. Après 5 jours sous agitation à température ambiante, le milieu réactionnel est concentré à sec. Le solide obtenu est solubilisé dans 5 mL d'eau, puis lavé 2 fois par 50 mL d'AcOEt. La phase aqueuse est acidifiée par HCl 1M jusqu'à pH = 2, le précipité formé est filtré puis lavé à l'éther. Après séchage, on obtient 2,78 g du composé attendu sous forme d'une gomme. F = 160°C.

En procédant comme décrit à la préparation ci-dessus, on a préparé les composés intermédiaires décrits dans le tableau suivant.

TABLEAU 3

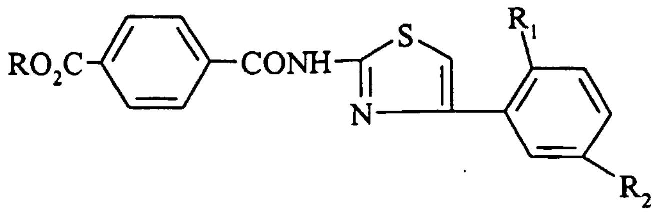

(XXXIII) et (XXXIIIbis)

| Préparations | $R_1$ | $R_2$ | R | Sel | Caractérisation |
|---|---|---|---|---|---|
| 4.2.1 | MeO- | nBu | H | - | $MH^+$ = 410 t=21,53 |
| 4.2.2 | MeO- | nPrO | Me | - | F = 180°C |
| 4.2.3 | MeO- | nPrO | H | HCl | $MH^+$ = 412 t=6,11 |
| 4.2.4 | MeO- | nBu | Me | - | $MH^+$ = 424 t = 21,35 |
| 4.2.5 | MeO- | nPr | Me | - | - |
| 4.2.6 | MeO- | nPr | H | - | $MH^+$ = 396 t = 8,17 |
| 4.2.7 | EtO- | Et | H | TFA | $MH^+$ = 396 t = 14,45 F = 250°C |
| 4.2.8 | EtO- | Et | tBu | - | RMN |
| 4.2.9 | MeO- | cyclohexyle | Me | - | $MH^+$ = 440 t=8,11 |
| 4.2.10 | MeO- | cyclohexyle | H | - | $MH^+$ = 436 t = 10,49 F > 260°C |
| 4.2.11 | EtO- | cyclohexyle | Me | - | - |
| 4.2.12 | EtO- | cyclohexyle | H | - | $MH^+$ = 450 t = 11,05 |
| 4.2.13 | MeO- | Et | Me | - | RMIV |
| 4.2.14 | MeO- | Et | H | - | RMN |
| 4.2.15 | MeO- | nHex | Me | - | $MH^+$ = 453 t = 11,9 |
| 4.2.16 | MeO- | nHex | H | - | $MH^+$ = 439 t = 11,0 |
| 4.2.17 | EtO- | nBu | Me | - | $MH^+$ = 439 t = 12,1 |
| 4.2.18 | EtO- | nBu | H | - | $MH^+$ = 425 t = 8,6 |

RMN : Préparation 4.2.8. : 1,2 ppm : t : 3H ; 1,6 ppm : t : 3H ; 1,7 ppm : s : 9H ; 2,7 ppm : m : 2H ; 4,2 ppm : q : 2H ; 7,0-7,2 ppm : m : 2H ; 7,8 ppm : s : 1H ; 8,0-8,3 ppm : m : 5H ; 12,9 ppm : s.e. : 1H.

RMN : Préparation 4.2.13. : 1,2 ppm : t : 3H ; 2,6 ppm : q : 2H ; 3,9 ppm : s : 6H ; 7,0-7,2 ppm : m : 2H ; 7,7 ppm : s : 1H ; 8,0-8,4 ppm : m : 5H ; 12,8 ppm : s.e. : 1H.

RMN : Préparation 4.2.14. : 1,2 ppm : t : 3H ; 2,7 ppm : q : 2H ; 3,9 ppm : s : 3H ; 7,0-7,2 ppm : m : 2H ; 7,7 ppm : s : 1H ; 8,0-8,4 ppm : m : 5H ; 12,6-13 ppm : s.e. : 1H ; 13,2-13,5 ppm : s.e. : 1H.

Préparation 4.3.1

N-(4-(S-Butyl-2-méthoxyphényl)-1,3-thiazol-2-yl)-4-(2-chloroéthyl) benzamide.

(XXIX): $R_1$ = 2-OMe; $R_2$ = 5-nBu; $R'_3$ = 4-$(CH_2)_2$Cl

On prépare un mélange contenant 3 g d'aminothiazole de la Préparation 1.2, 10 mL de $CH_3CN$, 2,54 g d'acide 4-(2-chloroéthyl)benzoïque, 1,11 mL de $NEt_3$ et 6,1 g de BOP et on laisse sous agitation pendant 48 heures. Le milieu

réactionnel est dilué par AcOEt, puis lavé par une solution saturée de $Na_2CO_3$ (2 fois) et par une solution saturée de NaCl, puis on sèche sur $MgSO_4$ et on concentre à sec. Après trituration dans l'EtOH, l'insoluble formé est éliminé par filtration, puis le filtrat est concentré à sec pour donner 7,7 g du composé attendu. Celui-ci est purifié par chromatographie sur silice en éluant par un gradient toluène/cyclohexane (9/1; v/v) jusqu'au toluène pur.

En procédant comme décrit ci-dessus, on prépare les composés de la formule (XXIX) décrits dans le tableau ci-après.

TABLEAU 4

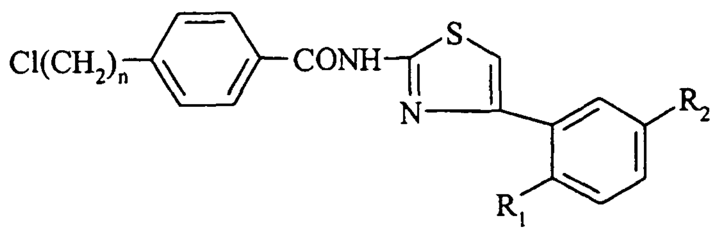

(XXIX)

| Préparations n° | n | $R_1$ | $R_2$ | Caractérisation |
|---|---|---|---|---|
| 4.3.1 | 2 | MeO- | nBu | RMN |
| 4.3.2 | 2 | MeO- | Et | RMN |
| 4.3.3 | 2 | MeO- | nPrO | $MH^+$ = 431,2 t = 9,91 |
| 4.3.4 | 1 | MeO- | nRu | - |
| 4.3.5 | 1 | MeO- | Et | F = 141°C |

RMN : Préparation 4.3. t. : 0,85 ppm : t : 3H ; 1,05-1.6 ppm m : 4H ; 2,5-2,6 ppm : m : 2H : 3.0 ppm : m : 2H ; 3,8-4,0 ppm : m : 5H ; 7.0 ppm : m : 2H : 7.4-7,6 ppm : d : 2H ; 7,7 ppm : s : 1H ; 8,0 ppm : m : 3H ; 12,6 ppm : s.e. : 1H.

RMN : Préparation 4.3.2. : 1,2 ppm : t 3H ; 2,7 ppm : q : 2H ; 3,2 ppm : t : 2H : - 4,0 ppm : m : 5H ; 7,0-7,2- ppm : m : 2H ; 7,6 ppm : d : 2H ; 7,8 ppm : s : 1H ; 8,0-8,3 ppm : m : 3H : 12,7 ppm : s.c. : 1H.

préparation 4.4.1

N-(4-(5-Butyl-2-méthoxyphényl)-1,3-thiazol-2-yl)-4-(3-oxopropyl)benzmide.

$R_1$ = 2-OMe; $R_2$ = 5-nBu. $R'_3$ = 4-$(CH_2)_2$CHO

A) N-(-4-(5-Butyl-2-memoxyphényl)-1,3-thiazol-2-yl)-4-iodobenzamide.

On laisse sous agitation à température ambiante pendant 8 heures un mélange contenant 6,62 g d'aminothiazole de la Préparation 1.2, 75 mL de $CH_3CN$, 7,53 g d'acide 4-iodobenzoïque, 4.2 mL de $NEt_3$ et 13,45 g de BOP. On filtre le produit formé puis on rince au $CH_2CN$. On redissout le précipité dans le DCM, lave par une solution de NaOH à 7% (2 fois), puis sèche sur $MgSO_4$ et évapore. Parallèlement, le filtral dans $CH_3CN$ est évaporé, repris au DCM, puis lavé 4 fois par use solution de NaOH à 7% et séché sur $MgSO_4$. On obtient ainsi au total 16,81 g de composé attendu. $MH^+$ = 492; t = 12,01 minutes.

B) N-(-4-(5-Butyl-2-memoxyphényl)-1,3-thiazol-2-yl)-4-(3-oxopropyl)benzamide.

On- place sous azote 2 g du composé de l'étape précédente dans 15 mL de DMP avec 0,79 g de tamis moléculaire 4Å, 0,42 mL d'alcool allylique, 1,3 g de bromure de tetra-(nbutyl)ammonium séché et 0;85 g de $NaHCO_3$ séché. Après 2 heures sous agitation à température ambiante, on ajoute 50 mg de Pd $(OAc)_2$ et on maintient sous agitation à température ambiante pendant 20 heures, sous azote sec. On filtre sur Célite®, on rince au DMF, puis on ajoute de l'eau et de l'éther. Après décantation, on extrait à nouveau 3 fois à l'éther. On sèche sur $MgSO_4$ et évapore sous-vide. On obtient 2 g du composé attendu.

En procédant comme décrit ci-dessus, on prépare les composés de formule (XXVII) et leurs précurseurs iodés tels que décrits à l'étape A de la préparation ci-dessus.

TABLEAU 5

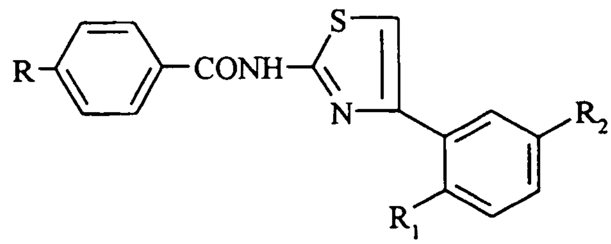

(XXVII) : R = $(CH_2)_2CHO$ (XXVII bis) : R = I

| Préparations n° | $R_1$ | $R_2$ | R | Caractérisation |
|---|---|---|---|---|
| | OMe | nBu | 1 | $MH^+$ = 493,2 t = 12,01 |
| 4.4.1 | OMe | nBu | $-(CH_2)_2CHO$ | - |
| | OEt | Et | 1 | F = 180°C |
| 4.4.2 | OEt | Et | $-(CH_2)_2CHO$ | - |
| | OMe | Et | I | $MH^+$ = 465,1 t = 11,35 |
| 4.4.3 | OMe | Et | $-(CH_2)_2)CHO$ | - |

Préparation 4.5

Chlorhydrate de N-(4-(2-Méthoxy-5-propoxyphényl)-1,3-thiazol-2-yl)-N'-(2-oxoéthyl)téréphtalamide

(XXXIVbis), HCl : $R_1$ =2-OMe; $R_2$= 5-OnPr ; $R'_3$ =4-$CONHCH_2CHO$

A) N-(-2,2-Diméthoxyéthyl)-N'-(4-(2-méthoxy-S-propoxyphényl)-1,3-thiazol-2-yl) téréphtalamide.
On laisse sous agitation à température ambiante pendant 3 heures et demie un mélange contenant 2,6 g du composé de la Préparation 4.2.3, 30 mL de $CH_3CN$, 1,61 mL de $NEt_3$, 0,53 mL d'aminoacétaldehyde diméthylacétal et 2,56 g de BOP. Après filtration, on lave au $CH_3CN$ puis au. DCM. On évapore le filtrat, triture le résidu avec $CH_3CN$, filtre et on obtient 2 g du composé attendu.
B) Chlorydrate de N-(4-(2-Mérhoxy-5-propoxyphényl)-1,3-thiazol-2-yl)-N-(2-oxocthyl) téréphtalàtnicie.
Sous azote sec, on place 0,2 g du composé obtenu à l'étape précédente dans 2 mL de dioxane, on chauffe à reflux pour dissoudre et on refroidit à TA, puis or ajoute 3 mL d'HCl 4M dans le dioxane et on laisse sous agitation 7 heures. On filtre sous azote le précipité formé pour obtenir 0,15 g du composé attendu.

Préparation 4.6

4-(2-Hydioxyéthoxy)-N-(4-(2-méthoxy-5-propoxyphényl)-1,3-thiazol-2-yl) benzamide.

(XVIII) : $R_1$ = 2-OMe; $R_2$= 5-OnPr ; $R'_3$ =4=$O(CH_2)_2OH$.

A) 4-(2-(Tetrtihydro-2*H*-pyran-2-yloxy)éthoxy)benxoate de méthyle.
On chauffe à 100°C un mélange contenant 40 g de 4-hydroxybenzerate de méthyle et 90,84 g de $K_2CO_3$ dans 400 mL, de DMF et l'on ajoute lentement 71,47 g de 2-(2-bromooéthoxy)tétrahydro-2*H*-pyrane puis on maintient le chauffage pendant 8 heures. Le mineral est filtré, rincé au DMF. Le filtrat est évaporé sous vide puis repris au DCM, lavé 3 Fois à l'eau, séché sur $MgSO_4$ puis évaporé. On obtient 77,66 g du composé attendu.
B) Acide 4-(2-(tétrahydro-2*H*-pyran-2-yloxy)éthoxy)benzoïque.
On laisse sous agitation à TA, sous azote sec, pendant une journée, un mélange contenant 77,66 g du produit de

l'étape précédente dans 400 mL de MeOH et 135 mol de NaOH 5M. Le milieu réactionnel est évaporé puis repris par de l'eau et à pH = 5 par addition d'HCl On filtre puis on rince à l'eau. Le filtrat est extrait 2 fois au DCM, puis séché sur $MgSO_4$ et évaporé. On obtient 72 g du composé attendu.

C) N-(4-(2-methoxy-3-propoxyphényl)-1,3-thiazol-2-yl)-4-(tétrahydro-2*H*-pyran-2-yloxy)éthoxy)benxamide.

(XVII) : $R'_3$ = 4-O$(CH_2)_2$-OTHP.

On laisse sous agitation à TA pendant 4 jours un mélange contenant 3,83 g du composé de la Préparation 1.1, 30 mL de $CH_3CN$, 4,63 g d'acide obtenu à l'étape précédente, 7,69 g de BOP et 2,4 mL de $NEt_3$. On filtre le précipité forme, rince par $CH_3CN$ et sèche sur $P_2O_5$ à 60˚C. On obtient 5,5 g du composé attendu, F = 114˚C. D) 4-(2-Hydroxyéthoxy)-N-(4-(2-méthoxy-3-propoxyphényl)-1,3-thiazol-2-yl) benzamide.

On place 5,41 g du composé de l'étape précédente dans 25 ml, d'HCl 4M dans le dioxine et on laisse sous agitation pendant 30 minutes. On filtre le précipité formé puis on le rince au dioxane et à l'éther et sèche sur $P_2O_5$ à 60˚C. On obtient 4,6 g du composé attendu.

Préparation 4.7

N-(4-(2-méthoxy-5-propoxyphényl)-1,3-thiazol-2-yl)-4-(2-oxoéthoxy)benzamide.

(XIV) : $R_1$ = OMe; $R_2$ = OnPr; $R'_3$ = 4-$OCH_2CHO$.

A) 4-(2,2-Diéthoxyéthoxy)-N-(4-(2-méthoxy-5-propoxyphényl)-1,3-thiazol-2-yl) benzamide.

On laisse sous agitation pendant 3 jours un mélange contenant 4,56 g du composé de la Préparation 1.1, 5,26 g du dérivé d'acide benzoïque de la Préparation 3.1 dans 40 mL de $CH_3CN$ avec 9,16 g de BOP et 2,9 mL de $NEt_3$. Après évaporation, on reprend au DCM, puis on lave 3 fois à l'eau, on sèche sur $MgSO_4$ et on évapore. Le produit obtenu est chromatographié sur silice en éluant par un mélange toluène/AcOEt (95/5; v/v). On reprend à l'éther, filtre, rince puis sèche à 60˚C sur $P_2O_5$ pour obtenir 6,12 g du composé attendu. F = 107˚C

B) N-(4-(2-Méthoxy-5-propoxyphényl)-1,3-thiazol-2-yl)-4-(2-oxoéthoxy)benzamide.

On place 1,52 g du composé de l'étape précédente dans 3,8 mL d'acide formique et on chauffe à 50˚C pendant 3 heures. Après retour à température ambiante, on ajoute de l'eau puis on filtre, rince à l'eau et sèche à 60˚C sur $P_2O_5$. On obtient 1,25 g du composé attendu.

Préparation 4.7 bis

N-(4-(2-méthoxy-5-propoxyphényl)-1,3-thiazol-2-yl)-4-(2-oxoéthoxy)benzamide.

On refroidit à -60˚C sous azote sec un mélange contenant 0,4 g du composé de la Préparation 4.6 dans 0,36 mL de DMSO et 5 mL de DCM et on laisse sous agitation pendant 2 heures. On ajoute 0,83 mL de $NEt_3$ et on laisse la température revenir à TA. Le minéral est filtré puis rincé par du DCM. Le filtrat est lavé à l'eau, par une solution de $Na_2CO_3$ à 10 % puis 2 fois par une solution saturée de NaCl. On sèche sur $MgSO_4$ et évapore. On obtient 0,39 g du composé attendu.

Préparation 4.8

2-(4-(((4-(2-Méthoxy-5-propoxyphényl)-1,3-thiazol-2-yl)amino)carbonyl) phénoxy)éthyl méthanesulfonate.

On prépare un mélange contenant 0,4 g du composé de la Préparation 4.6 dans 5 mL de DCM, 0,28 mL de $NEt_3$ et 120 µl de $CH_3SO_2Cl$. On laisse sous agitation pendant 1 heure et demie en refroidissant dans un bain de glace. On filtre l'insoluble que l'on rince au DCM. Le filtrat est lavé 3 fois à l'eau. On sèche la phase organique sur $MgSO_4$, filtre, rince au DCM et évapore pour obtenir 0,38 g du composé attendu.

EXEMPLE 1 Composé : 65

*N*-[4-(5-butyl-2-méthoxyphényl)-1,3-thiazol-2-yl]-4-((4-[2-(S)-(hydroxyméthyl) pyrrolidin-1-yl]pipéridin-1-yl) carbonyl)benzamide (I) : $R_1$ = 2-OMe ; $R_2$ = 5-nBu ;

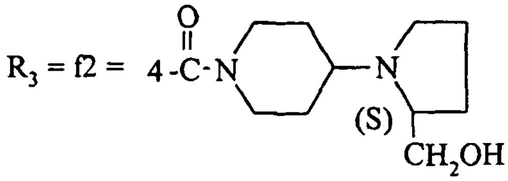

On place sous agitation pendant 30 minutes une solution contenant 82 mg du composé de la Préparation 4.1.1 et 45 mg de (S) prolinol dans 2 mL de DCM et 5 gouttes de $CH_3CO_2H$, puis on ajoute 100 mg de $NaBH(OAc)_3$ et on laisse sous agitation pendant 12 heures. On dilue le milieu par 200 mL d'AcOEt, puis on lave 2 fois par $Na_2CO_3$, on sèche sur $MgSO_4$ et on évapore. Le produit obtenu est agité aux ultrasons en présence de 5 mL d'éther puis filtré pour donner 70 mg du composé attendu. F = 199°C.

Spectre de RMN : 0,85 ppm : t : 3H ; 1,1-2 ppm : m : 12H ; 2,2-4,6 ppm : m : 15H ; 6,8-7,2 ppm : dd : 2H ; 7,45 ppm : d : 2H ; 7,65 ppm : s : 1H ; 7,95 ppm : s : 1H ; 8,1 ppm : d : 2H ; 12,6 ppm : se : 1H.

EXEMPLE 2 : Composé 120

4-((4-[3-(R)-(acétylamino)pyrrolidin-1-yl]pipéridin-1-yl)carbonyl)-N-[4-(5-butyl-2-méthoxyphényl)-1,3-thiazol-2-yl]benzamide. (I) : $R_1$ = 2-OMe ; $R_2$ = 5-nBu ;

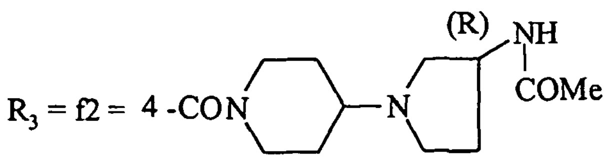

On place sous azote sec un mélange contenant 0,25 g du composé de la Préparation 4.1.1, 0,13 g de (3R) 3-acétamidopyrrolidine et 2 mL de DCM et on agite 15 minutes, puis on ajoute 0,22 g de $NaBH(OAc)_3$ et 8 gouttes d'AcOH et on maintient sous agitation à température ambiante pendant 3 heures 50 minutes. On ajoute une solution de $Na_2CO_3$ à 10 % et AcOEt. Après décantation, on lave la phase organique par $Na_2CO_3$ à 10 % puis on sèche sur $MgSO_4$ et évapore. Le résidu est trituré dans l'éther puis on filtre, rince à l'éther et sèche à 60°C sur $P_2O_5$. On obtient 0,25 g du composé attendu. F > 200°C dec.

Spectre de RMN : 0,85 ppm : t : 3H ; 1-2,1 ppm : m : 13H ; 2,1-4,5 ppm : m : 15H ; 6,8-7,2 ppm : dd : 1H ; 7,45 ppm : d : 2H ; 7,65 ppm : s : 1H ; 7,85 ppm : d : 1H ; 8,15 ppm : d : 2H ; 12,65 ppm : se : 1H.

EXEMPLE 3 : Composé 107

Dichlorhydrate de *N*-[4-(5-butyl-2-méthoxyphényl)-1.3-thiazol-2-yl]-4-((4-pyrrolidin-1-ylpipéridin-1-yl)carbonyl) benzamide (Ia), 2HCl : $R_1$ = 2-OMe ; $R_2$ = 5-nBu ;

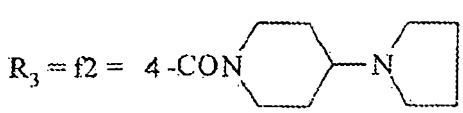

On laisse sous agitation à température ambiante pendant 9 jours un mélange contenant 0,6 g du composé de la Préparation 4.2.1, 6 mL de $CH_3CN$, 0,31 mL d'éthyldiisopropylamine, 0,27 g de 4-(pyrrolidin-1-yl)pipéridine dans 2 mL de $CH_3CN$ et 0,78 g. de BOP. Après évaporation des solvants, le milieu est repris par AcOEt puis lavé par une solution aqueuse de $Na_2CO_3$ à 10 % (3 fois), puis une solution saturée de NaCl, puis séché sur $MgSO_4$ et évaporé sous vide. Le résidu est chromatographié sur silice en éluant par DCM/MeOH (100/3;v/v) pour obtenir 0,43 g de base, F = 128°C, $MH^+$ = 547,4 ; t=6,83.

La base obtenue est reprise dans du DCM, puis on ajoute de l'éther chlorhydrique. On obtient 0,44 g du composé attendu après filtration et séchage.

Spectre de RMN: 0,85 ppm : t : 3H ; 1,05-2 ppm : m : 12H ; 2,25 ppm : m : 1H ; 2,5 ppm : t : 2H ; 2,8-4,40 ppm : m: 12H ; 6,95 ppm : d : 1H ; 7,05 ppm : d de d : 1H ; 7,5 ppm : d : 2H ; 7,65 ppm : s : 1H ; 7,95 ppm : d : 1H ; 8,15 ppm : d : 1H ; 12,55 ppm : se : 1H.

EXEMPLE 4 : Composé 68

Dichlorhydrate de *N*-4-(5-butyl-2-méthoxyphényl)-1,3-thiazol-2-yl]-4-((4-(tetrahydro-2*H*-pyran-4-ylamino)pipéridin-1-yl)carbonyl)benzamide.

(Ia), 2HCl : $R_1$ = 2-OMe ; $R_2$ = 5-nBu ;

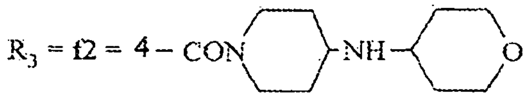

(IV) : $R_1$ = 2-OMe ; $R_2$ = 5-nBu ;

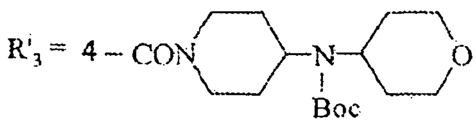

On place 0,3 g du composé de la Préparation 4.2.1 dans 3 mL de $CH_3CN$ et on ajoure 153 µl d'éthyldiisopropylamine, 0,25 g d'amine de la Préparation 2.1, et 0,39 g de BOP. On laisse sous agitation à température ambiante pendant une nuit puis on filtre, rince à l'éther et sèche sur $P_2O_5$ à 60°C. On obtient 0,36 g du composé attendu. F= 152°C.
B)

On place 0,35 g du composé de l'étape précédente dans 3 mL d'HCl 4M dans le dioxane et on laisse 35 minutes sous agitation à température ambiante. On ajoute de l'éther, filtre, lave à l'éther puis on sèche sur $P_2O_5$ à 60°C. On obtient 0,32 g du composé attendu, F = 181°C.

Spectre de RMN : 0,85 ppm : t : 3H ; 1,25 ppm : sext : 2H; 1,15-1,90 ppm : m : 10H ; 2.5 ppm : t : 2H ; 2,65-4,70 ppm : m : 15H ; 6,95 ppm : d : 1H; 7,05 ppm : dd : 1H ; 7,45 ppm : d : 2H ; 7,65 ppm : s : 1H ; 7,95 ppm : d 1H ; 8,1 ppm d : 2H ; 9,3 ppm : se : 1H ; 12,65 ppm : se : 1H.

A partir des composés intermédiaires décrits dans le tableau ci-après, on opère selon l'Exemple 4, étape B par traitement en milieu acide pour préparer des composer de formule (Ia) selon l'invention.

TABLEAU 6

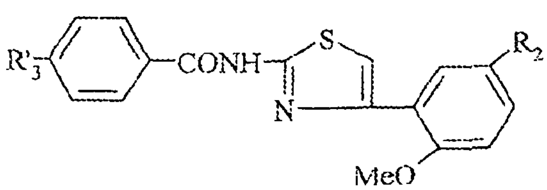

(IV)

| Préparations | $R_2$ | R'$_3$ | Caractérisation |
|---|---|---|---|
| 5.1 | -nBu | 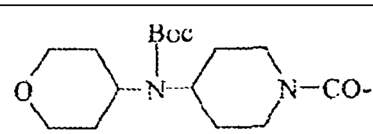 | F = 152°C |
| 5-2 | -Et | Boc-NH-$(CH_2)_2$-NHCO- | F- 221°C |
| 5.3 | -OnPr | 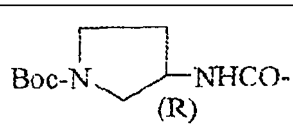 | $MH^+$ = 581,4 t = 10,12 |
| 5.4 | -OnPr | 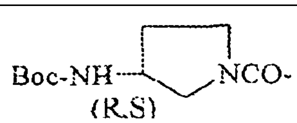 | $MH^+$ = 581.4 t = 9,74 |

(suite)

| Préparations | $R_2$ | $R'_3$ | Caractérisation |
|---|---|---|---|
| 5.5 | -nBu | 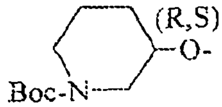 | $MH^+$ = 566,4 t = 12,11 |
| 5.6 | -nBu | 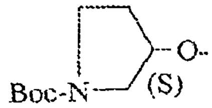 | $MH^+$ = 552,5 t= 11,84 |
| 5.7 | -nBu | 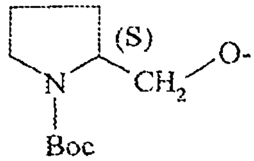 | $MH^+$ = 566,5 t = 12,37 |
| 5.8 | -nBu | 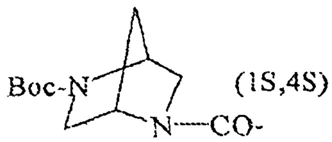 | $MH^+$ = 591,4 t = 10.63 F = 115°C |
| 5.9 | -nBu | 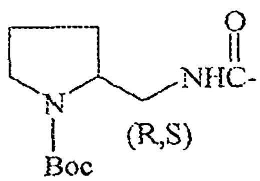 | F = 98°C |
| 5.10 | cyclopentyle | 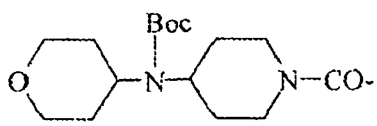 | F = 180°C |
| 5.11 | -nBu | 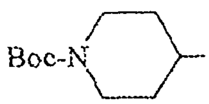 | F = 82°C |
| 5.12 | -nPr | 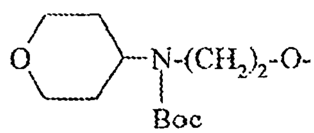 | $MH^+$ = 596,3 t = 10,74 |
| 5.13 | -nBu | 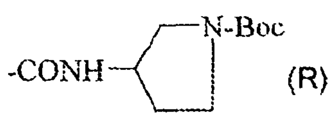 | $MH^+$ = 579 t = 10.6 |
| 5.14 | -nBu | 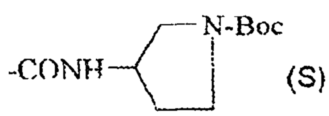 | $MH^+$ = 579 t = 10,6 |

EXEMPLE 4 bis : Composé 68

Dichlorhydrate de *N*-[4-(5-butyl-2-méthoxyphényl)-1,3-thiazol-2-yl]-4-((4-(tetrahydro-2*H*-pyran-4-ylamino)pipéradin-1-yl)carbonyl)benzamide.
(Ia), 2HCl : $R_1$ = 2-OMe ; $R_2$ = 5-nBu ;

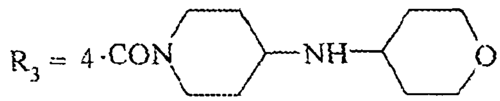

A)

On place sous agitation a température ambiante, pendant 48 heures, un mélange contenant 370 mg du composé de la Préparation 1.2, 3 mL de $CH_3CN$, 378 mg de BOP et 151 mg du compose de la Préparation 3.2. On dilue le milieu réactionnel par AcOEt, puis on lave 3 fois par une solution aqueuse de $Na_2CO_3$ à 10%, puis 3 fois par une solution aqueuse de NaCl. La phase organique est séchée sur $MgSO_4$, puis concentrée à sec. On reprend par de l'éther isopropylique, puis on filtre et sèche pour obtenir 325 mg du composé attendu. F = 152°C, B)

On place sous agitation, pendant 1 heure, 320 mg du composé de l'étape précédente dans 3 mL d'HCl 4M dans le dioxane. On ajoute de l'éther, filtre, puis on lave le solide à l'éther et on sèche pour obtenir 259 mg du composé attendu. F = 181°C.

EXEMPLE 5 : Composé 161

4-[3-(3-(acétylamino)pyrrolidin-1-yl)propanoyl]-*N*-[4-(5-butyl-2-méthoxyphényl)-1,3-thiazol-2-yl]-benzamide.
(Ia) : $R_1$ = 2-$OCH_3$ ; $R_2$ = 5-nBu ;

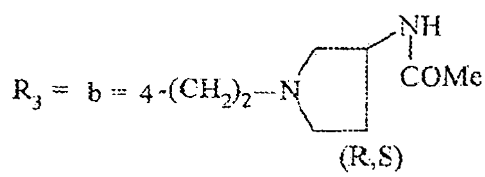

On place sous agitation à 50°C, pendant 2 jours, un mélange contenant 0,3 g du composé de la Préparation 4.3.1, 0,12 g de KI, 0,06 g de $NaHCO_3$ dans 3 mL de DMF et 897,3 mg de pyrrolidine-3-acétamide. Le milieu est concentré à sec, puis repris par du DCM. La phase organique est lavée 2 fois par une solution saturée de NaCl, puis séchée sur $MgSO_4$ et concentrée à sec. Le produit cristallise en présence de MTBE. Après filtration et séchage, on obtient 0,02 g du composé attendu, F = 112°C.

EXEMPLE 6 : Composé 185 Trichlorhydrate de *N*-[4-(5-éthyl-2-méthoxyphényl)-1,3-thiazol-2-yl]-4-[*N*-((1

-éthylpyrrolidin-2-yl)methyl)glycyl]benzamide
(Ia) : 3HCl : $R_1$ = 2-OMe ; $R_2$ = 5-Et ; $R_3$ = d2=

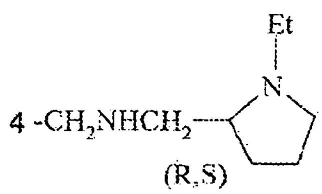

On chauffe à reflux, pendant 5 heures et demie, un mélange contenant 0,25 g du composé de la Préparation 4.3.5 dans 3 mL de $CH_3CN$ et 0,21 g de 2-aminométhyl-1-éthylpyrrolidine dans 1 mL de $CH_3CN$. Après une nuit sous agitation à température ambiante, on évapore, reprend au DCM, puis lave 3 fois à l'eau, puis on sèche sur $MgSO_4$ et évapore. Le produit obtenu est chromatographié sur silice en éluant par DCM/MeOH (100/5; v/v).

Le composé obtenu est repris au DCM, on ajoute de l'éther chlorhydrique, puis on filtre le précipite formé et rince à l'éther. On sèche sur $P_2O_5$ à 60°C pour obtenir 0,17 g du composé attendu, F = 163°C (déc),

EXEMPLE 7 : Composé 182

N-[4-(5-butyl-2-méthoxyphényl)-1,3-thiazol-2-yl]-4-[3-(tétrahydro-2*H*-pyran-4-ylamino)propyl]benzamide
(Ia) : $R_1$ = 2-OMe ; $R_2$ = 5-nBu ;

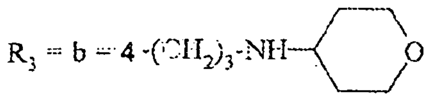

On place sous azote un mélange contenant 0,38 g du composé de la Préparation 4.4.1 dans 3 mL de DCM et 0,15 g de 4-aminotetrahydropyrane dans 1 mL de DCM. Après 15 minutes d'agitation, on ajoute 0,31 g de NaBH $(OAc)_3$ et on maintient l'agitation pendant 6 heures. On ajoute une solution de $Na_2CO_3$ à 10 %, et l'AcOEt puis on décante, on lave par une solution de $Na_2CO_3$ à 10 % puis on sèche sur $Na_2SO_4$ et évapore. Le produit obtenu est chromatographié sur silice en éluant par DCM/MeOH (100/4; v/v). On obtient 0,1 g du composé attendu.

EXEMPLE 8 ; Composé 95

*N*-[4-(5-éthyl-2-méthoxyphenyl)-1,3-thiazol-2-yl]-*N'*-(2-pyrrolidin-1-yléthyl) téréphtalamide
(Ia) : $R_t$ = 2-OMe ; $R_2$ = 5-Et ;

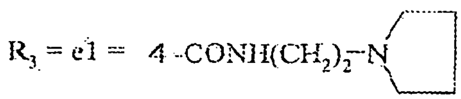

On laisse sous agitation à température ambiante pendant 2 jours un mélange contenant 0,3 g du composé de la Préparation 4.2.14, 3 mL de $CH_3CN$, 0,25 mL d'éthyldiisopropylamine, 98 mg de 1-(2-aminoéthyl)pyrrolidine dans 2 mL de $CH_3CN$, 0,38 g de BOP et 1 mL de DMF. On filtre, rince par $CH_3CN$ puis sèche à 60°C sur $P_2O_5$. On obtient 0,22 g du composé attendu. F = 170°C.

EXEMPLE 9 : Composé 9

4-[2-(2-(S)-(hydroxyméthyl)pyrrolidin-1-yl)éthoxy]-*N*-[4-(2-méthoxy-5-propoxyphényl)-1,3-thiazol-2-yl]benzamide.
(Ia) : $R_1$ = 2-OMe ; $R_2$ = 5-OnPr ;

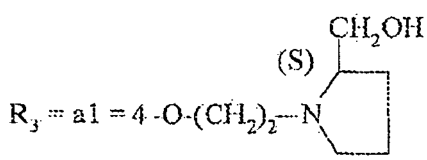

On laisse sous agitation pendant 18 heures un mélange contenant 0,2 g de la Préparation 4.7, 3 mL de DCM, 112 µl de (S)-(+)-2-pyrrolidineméthanol, 0,2 g de $NaBH(OAc)_3$ et 5 gouttes de $CH_3CO_2H$. On ajoute au milieu réactionnel une solution saturée de $Na_2CO_3$, de l'eau, du DCM puis on décante. La phase organique est lavée par de l'eau, puis séchée sur $MgSO_4$ et évaporée. On purifie par chromatographie sur silice en éluant par un mélange DCM/MeOH (100/2, v/v). On obtient 100 mg du composé attendu.

$MH^+$ = 511 ; t = 5,72 minutes $[\alpha]_D^{20} = -11,2°$ ( c = 0,848, DMSO)

EXEMPLE 10 : Composé 183

*N*-[4-(5-butyl-2-méthoxyphényl)-1,3-thiazol-2-yl]-4-[(4-tétrahydro-2*H*-pyran-4-ylamino)pipéridin-1-yl)méthyl] benzamide.
(Ia) ; $R_1$ = 2-OMe ; $R_2$ = 5-nBu ;

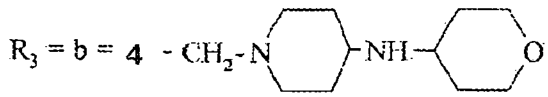

A)

(IV) : $R_1$ = 2-OMe ; $R_2$ = 5-nBu ;

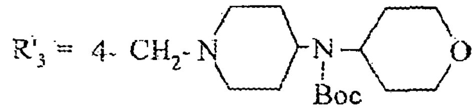

On chauffe à reflux pendant une heure un mélange contenant 0,25g du composé de la Préparation 4.3.4 et 0,34 g du composé de la Préparation 2.1 dans 1 mL de $CH_3CN$. On filtré, rince par $CH_3CN$, puis à l'éther et sèche à 60°C sur $P_2O_5$. On obtient 0,35 g du composé attendu.

B)

On place 0,34 g du composé obtenu à l'étape précédente dans 3 mL d'HCl/dioxane 4M et on laisse 5 heures sous agitation à température ambiante. On ajoute de l'eau, filtre puis rince à l'éther et sèche sur $P_2O_5$ à 60°C. On reprend par un mélange AcOEt/$Na_2CO_3$ à 10%. Après décantation, on lave par $Na_2CO_3$ à 10%, puis à l'eau et sèche sur $MgSO_4$ puis évapore. On purifie par chromatographie sur silice en éluant par DCM/MeOH (90/3 ; v/v). On obtient 93 mg du composé attendu.

$MH^+$ = 562, t = 5,8 minutes.

EXEMPLE 11 : Composé 17

*N*-[2-(2-(S)-(hydroxyméthyl)pyrrolidin-1-yl)éthyl]-*N*-[4-(2-méthoxy-5-propoxyphényl)-1,3-thiazol-2-yl]téréphtalamide.

n(I) : $R_1$ = 2-OMe ; $R_2$ = 5-OnPr ;

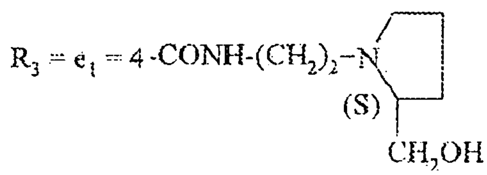

Sous azote sec, on mélange 0,13 g du composé de la Préparation 4.5 dans 2 mL de DCM avec 54 mg de (S)-(+)-2-pyrrolidineméthanol. Après 15 minutes d'agitation, on ajoute 1.07 mg de $NaBH(OAc)_3$ et 5 gouttes de AcOH puis on maintient l'agitation pendant 4 heures. On ajoute au milieu réactionnel une solution saturée de $Na_2CO_3$, de l'eau et du DCM puis on décante. On extrait à nouveau au DCM puis on lave 2 fois par une solution de NaCl saturée. Les phases organiques sont rassemblées et séchées sur $MgSO_4$. Après évaporation partielle, on ajoute $CH_3CN$ et de l'éther, on filtre le précipité formé et sèche à 45°C sur $P_2O_5$. On obtient 57 mg du composé attendu sous forme solide. F = 168°C (déc.).

EXEMPLE 12 : Composé 14 bis Dichlorhydrate de N-(4-(2-méthoxy-5-propoxyphényl)-1,3-thiazol-2-yl)-4-(2-pipéridin-1-yléthoxy)benzamide.

(Ia), 2HCl : $R_1$ = 2-OMe ; $R_2$ = 5-OnPr ;

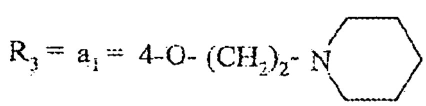

On chauffe à 80°C pendant 2 heures 40 minutes un mélange contenant 0,19 g du composé de la Préparation 4.8, 62 mg de $K_2CO_3$ et 38 mg de pipéridine dans 3 mL de DMF. On laisse revenir à TA puis on ajoute du DCM et de l'eau. Le milieu réactionnel est lavé à l'eau puis avec une solution diluée de NaOH. On sèche sur $MgSO_4$ et évapore. Le résidu

est chromatographié sur silice en éluant par DCM/MeOH (100/2 ; v/v). On reprend au DCM puis on ajoute de l'éther chlorhydrique, filtre et rince à l'éther, Après séchage sur $P_2O_5$ à 60°C, on obtient 54 mg du composé attendu.

Spectre de RMN : Composé 14 bis : 0,97 ppm : t : 3H ; 1,2-2,0 ppm : m : 8H ; 2,9-3,1 ppm : m : 2H ; 3,4-3,6 ppm : m : 4H ; 3,8-4,0 ppm : m : 5H ; 4,49 ppm : t : 2H ; 6,88 ppm ; dd : 1H ; 7,02 ppm : d : 1H ; 7,13 ppm : d : 2H ; 7,74 ppm : s : 1H ; 7,77 ppm : d : 1H ; 8,17 ppm : d : 2H ; 10,4 ppm : se : 1H ; 12,5 ppm : se : 1H.

EXEMPLE 13 : Composé N° 186

Ester éthylique {1-(1(4-(4-(5-Butyl-2-methoxy-phenyl)-thiazol-2-ylcarbamoyl)-piperidin-4-yl)-pyrrolidin-3-yl)-de l'acide carbamique

(Ia) $R_1$ = 2-OMe, $R_2$ = 5-nBu,

$R_3$ = f2 = 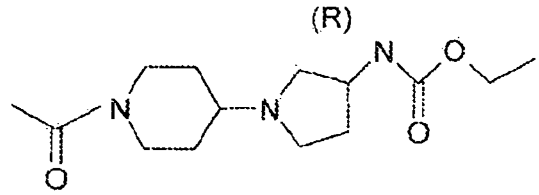

<u>A</u>) Ester *tert*-butylique (1-(1-(4-(4-(5-Butyl-2-methoxy-phenyl)-thiazol-2-ylcarbamoyl)-piperidin-4-yl)-pyrrolidin-3-yl)-de l'acide carbamique.

A une solution de 0,4 g du composé de la préparation 4.1.1 dans 3 ml de dichloroéthane, on ajoute 0,3 g de (3R)-3-(tert-butoxycarbonylamino)pyrrolidine, suivis de 0,35 g de $NaHB(Oac)_3$. 3 gouttes d'acide acétique sont ajoutées dans le milieu réactionnel qui est ensuite agité à TA pendant 2h. Le milieu est hydrolysé par addition d'eau puis dilué dans le dichloromethane et lavé 3 fois avec de la soude 1 M. La phase organique est lavée avec une solution saturée de NaCl, séchée sur $MgSO_4$ puis évaporée pour donner 0,37 g du composé attendu. Le brut ainsi obtenu peut être utilisé tel quel dans l'étape suivante.

F = 104°C pour une fraction de brut purifiée par flash chromatographie.

<u>B</u>) 4-(4-(3-Amino-pyrrolidin-1-yl)-piperidine-1-carbonyl)-N-(4-(5-butyl-2-methoxy-phényl)-thiazol-2-yl)-benzamide.

A une solution de 2,7 g du composé N° 151 dissous dans 9 ml de dichlorométhane, on ajoute à 0°C, 3 ml d'acide trifluoroacétique. On laisse le milieu revenir à TA. Après 2 h à TA, le milieu est évaporé puis repris trois fois dans le dichlorométhane et évaporé. L'huile obtenue est reprise dans le dichlorométhane puis traitée par une solution de $Na_2CO_3$ à 10 %. La phase organique est lavée dans une solution de $Na_2CO_3$ à 10 %, puis dans une solution saturée de NaCl, puis séchée sur $MgSO_4$ et évaporée pour donner 2,18 g d'un solide beige. La base libre est purifiée par trituration dans un mélange dichlorométhane / méthanol pour donner 1,9 g du produit attendu.

Le chlorhydrate est obtenu par addition lente d'une solution 2M d'acide chlorhydrique dans l'éther à une solution de la base libre dans un mélange chloroforme/méthanol. La suspension est évaporée puis séchée sous vide pour donner 2 g du chlorhydrate.

F = 188°C.

C) Ester éthylique (1-(1-(4-(4-(5-Butyl-2-methoxy-phenyl)-thiazol-2-ylcarbamoyl)-piperidin-4-yl)-pyrrolidin-3-yl)-de l'acide carbamique.

A une solution de 2 g du compose N° 152 sous sa forme basique dans 7 ml de dichloromethane, un ajoute 0,46 g d'ethylchloroformate. Le milieu est refroidi à 0°C. On ajoute ensuite, au goutte à goutte, 0,7 g de diisopropyléthylamine. Le milieu est agité jusqu'à retour à TA. Le milieu est hydrolysé avec de l'eau puis dilué dans le dichloromethane. La phase organique est lavée avec une solution de soude molaire puis à l'eau puis avec une solution saturée de NaCl. La phase organique est séchée sur $MgSO_4$ puis évaporée. Le brut est purifié par flash chromatographie pour donner 1,7 g du produit attendu.

F = 126°C.

Le solide obtenu est dissout dans le dichlorométhane puis salifié par addition lente d'une solution 2M d'acide chlorhydrique dans l'éther. Après évaporation, on collecte 1,72 g du chlorhydrate.

F = 178°C.

EXEMPLE 14 : Composé N° 187

N-(4-(5-butyl-2-methoxy-phenyl)-thiazol-2-yl)-4-(4-(3-methanesulfonylamino-pyrroldin-1-yl)-piperidine-1-carbonyl)-benzamide.

(Ia) $R_1$ = 2-OMe, $R_2$ = 5-nBu,

$R_3$ = f2 = 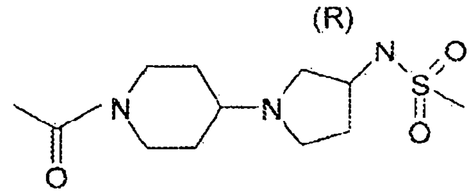

A une solution dans 2 ml d'un mélange 1/1 d'acétate d'éthyle, de DMF et de 0,14 g du composé n° 152 sous sa forme basique, on ajoute 57 mg de chlorure de méthane sulfonyl et 0,14 ml de triéthylamine. Après 24 h à TA, le milieu est filtré puis évaporé et repris dans l'acétate d'ethyle. La phase organique est lavée deux fois dans une solution saturée de NaCl, séchée sur $MgSO_4$ et évaporée. Le brut est purifié par flash chromatographie pour donner 65 mg d'un solide blanc.
F = 125°C.

EXEMPLE 15 : Composé N° 153

N-(4-(5- butyl- 2- methoxy- phenyl)-thiazol- 2- yl)- 4-(4-(3- propionylamino- pyrrolidin- 1- yl)-piperidine- 1- carbonyl)-benzamide.
(Ia) $R_1$ = 2-OMe, $R_2$ = 5-nBu,

$R_3$ = f2 = 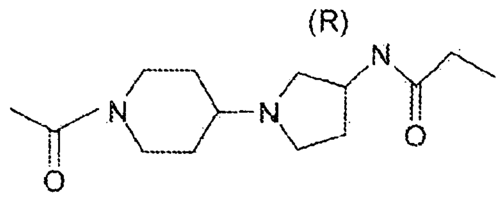

A une solution dans 0,5 ml d'acétate d'éthyle et de 0,1 g du composé N° 152 sous sa forme basique, on ajoute 0,04 g d'anhydride propionique et 0,05 ml de triethylamine. Après 2 h à TA, le milieu est dilué dans le dichlorométhane. La phase organique est lavée dans l'eau, dans une solution saturée de NACl, séchée sur $MgSO_4$ et évaporée. Le brut est purifié par flash chromatographie pour donner 67 mg d'un solide blanc.
F = 128°C

EXEMPLE 16 : Composé N° 188

Ester 1-(1-(4-(4-(5-Butyl-2-methoxy-phenyl)-thiazol-2-ylcarbamoyl)-benzoyl)-piperidin-4-yl)-pyrrolidin-3-yl de l'acide acétique.
(Ia) $R_1$ = 2-OMe, $R_2$ = 5-nBu,

$R_3$ = f2 = 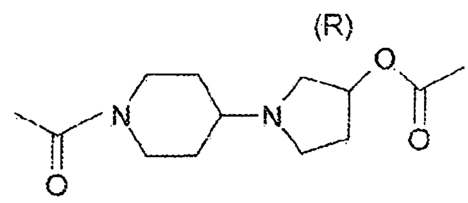

A une solution de 0,2 g du composé N° 123 en solution dans 1 ml de dichlorométhane, on ajoute 73 mg d'anhydride acétique. Après 2 h à TA, on rajoute 73 mg d'anhydride acétique et on laisse agiter 12 h à TA. Le milieu est dilué dans le dichlorométhane. La phase organique est lavée 3 fois dans une solution de $Na_2CO_3$ à 10 %, puis dans l'eau puis dans une solution saturée de NaCl, puis séchée sur $MgSO_4$ et évaporée pour donner 194 mg d'un solide blanc.
F = 99°C.

EXEMPLE 17 : Composé N° 189

4-(4-(1-Acetyl-pyrrolidin-3-ylamino)-piperidine-1-carbonyl)-N-4-(5-butyl-2-methoxy-phenyl)-thiazol-2-yl)-ben-

zamide.
(Ia) $R_1$ = 2-OMe, $R_2$ = 5-nBu,

$R_3$ = f2 = 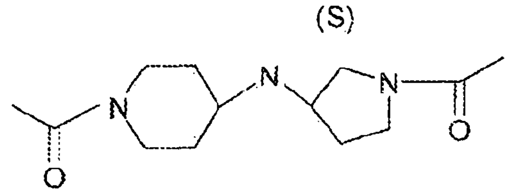

A) Ester *tert*-butylique 3-(1-(4-(4-(5-Butyl-2-methoxy-phenyl)-thiazol-2-ylcarbamoyl)-benzoyl)-piperidin-4-ylamino)-pyrrolidin-1-acide carboxylique

Une solution de 0,23 g de (S)-3-amino-1-N-Boc-pyrrolidine et de 0.5 g du composé de la préparation 4.1.1 dans 2 ml de dichlorométhane est agitée pendant 3 0 min à TA. On ajoute 0,43 g de $NaHB(OAc)_3$ au mélange réactionnel et 8 gouttes d'acide acétique. Le milieu réactionnel est ensuite agité à TA pendant 4 h. Le milieu est hydrolysé par addition d'eau puis dilué dans le dichlorométhane et lavé 3 fois avec de la soude molaire. La phase organique est lavée avec une solution saturée de NaCl, séchée sur $MgSO_4$ puis évaporée pour donner 0,65 g du composé attendu. Le brut ainsi obtenu peut être utilisé tel quel dans l'étape suivante.

$MH^+$ = 662 à t = 7,3 mn.

B) N-4-(5-butyl-2-methoxy-phenyl)-thiazol-2-yl)-4-(4-(pyrrolidin-3-ylamino)-piperidine-1-carbonyl)-benzamide A une solution de 0,61 g du composé obtenu à l'étape précédente dans 2 ml de dichlorométhane, on ajoute 2 ml d'éther chlorhydrique. Après 4 h 30 à TA, le milieu est évaporé. Le brut est trituré dans l'éther, filtré, rincé à l'éther et séché pour donner 0,51 g du composé attendu.

MH+ = 562 à t = 5.83 mn.

C) 4-(4-(1-Acetyl-pyrrolidin-3-ylamino)-piperidine-1-carbonyl)-N-4-(5-butyl-2-methoxy-phenyl)-thiazol-2-yl)-benzamide

Une suspension de 0,5 g du composé obtenu à l'étape précédente dans un mélange dichlorométhane / acétate d'éthyle est traitée par une solution de $Na_2CO_3$ à 10 %. Après décantation, la phase aqueuse est extraite dans l'acétate d'éthyle, puis dans le dichlorométhane. Les phases organiques rassemblées sont séchées sur $MgSO_4$ puis évaporées.

On récupère ainsi 0,37 g de la base libre qui est mise en solution dans 2 ml de dichlorométhane. On ajoute 0,062 ml d'anhydride acétique et on agite pendant 3 h 30 min à TA. Le milieu est évaporé puis chromatographié sur silice pour donner 0,3 g du produit attendu.

$MH^+$ = 604 à 6,68 mn.

Les tableau qui suivent illustrent les structures chimiques et les propriétés physiques de quelques exemples de composés selon l'invention. Dans ces tableaux :

- dans la colonne "sel", "-" représente un composé sous forme de base libre, alors que "HCl" représente un composé sous forme de chlorhydrate ;
- Me, Et, nPr, iPr, nBu, iBu, tBu, nHex représentent respectivement les groupes méthyle, éthyle, n-propyle, *iso*-propyle, n-butyle; *iso*-butyle, *tert*-butyle, n-hexyle ;
- Ph et Bn représentent respectivement les groupes phényle et benzoyle.
- THP représente le tétrahydropyran-4-ylc.

Les composés sont caractérisés soit par leur spectre de résonance magnétique nucléaire (RMN), placé à la fin du tableau, soit par leur point de fusion (F), soit par leur spectre de masse : $MH^+$ et temps de rétention (t), exprimé en minutes.

TABLEAU 7

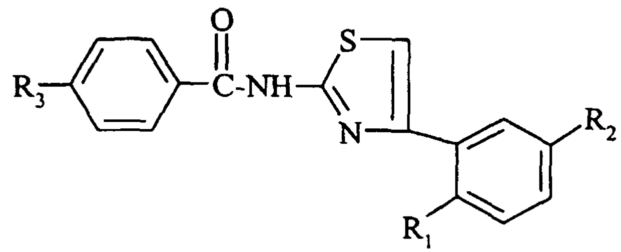

(Ia)

| Composés n° | $R_1$ | $R_2$ | $R_3$ | Sel | Caractérisation |
|---|---|---|---|---|---|
| 1 | -OMe | -OnPr | $-O(CH_2)_2NEt_2$ | 2HCl | RMN |
| 2 | -OMe | -OnPr | 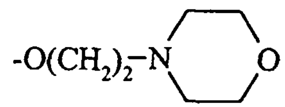 | 2HCl | $MH^+$ = 498,4 t = 6,64 |
| 3 | -OMe | -OnPr | $-O(CH_2)_2NH(CH_2)_2OH$ | - | $MH^+$ = 472,4 t = 6,45 |
| 4 | -OMe | -OnPr | 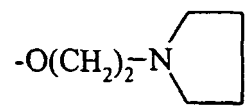 | 2HCl | $MH^+$ = 482,3 t = 5,87 |
| 5 | -OMe | -OnPr | $-O(CH_2)_2NHiPr$ | 2HCl | $MH^+$ = 470,2 t = 5,84 |
| 6 | -OMe | -OnPr | 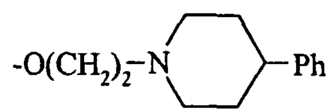 | 2HCl | $MH^+$ = 572,5 t = 7,59 |
| 7 | -OMe | -OnPr | 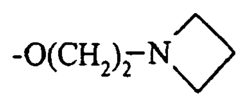 | - | $MH^+$ = 468,4 t = 5,73 |

(suite)

| Composés n° | $R_1$ | $R_2$ | $R_3$ | Sel | Caractérisation |
|---|---|---|---|---|---|
| 8 | -OMe | -OnPr | 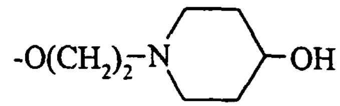 | - | $MH^+$ = 512,4 t = 5,64 |
| 9 | -OMe | -OnPr | 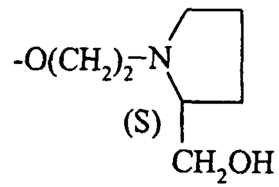 | - | $MH^+$ = 512,4 t = 5,72 |
| 10 | -OMe | -OnPr | 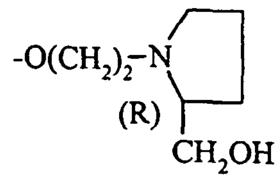 | 2HCl | $MH^+$ = 512,3 t = 5,71 |
| 11 | -OMe | -OnPr | $-O(CH_2)_2NH$-THP | 2HCl | $MH^+$ = 512,3 t = 5,75 |
| 12 | -OEt | -OEt | $-O(CH_2)_2NEt_2$ | 2HCl | $MH^+$ = 484,4 t = 5,94 |
| 13 | -OMe | -OnPr | 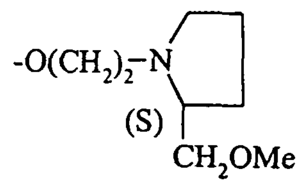 | 2HCl | F = 122°C (dec) $MH^+$ = 525,5 t = 6,84 |
| 14 | -SEt | -nBu | $-O(CH_2)_2NEt_2$ | 2HCl | $MH^+$ = 512,5 t = 7,64 |
| 14 bis | -OMe | -OnPr | 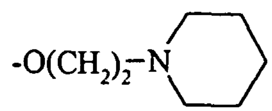 | 2HCl | RMN |
| 15 | -OMe | -OnPr | $-CONH(CH_2)_2NEt_2$ | - | F = 204°C $MH^+$ = 511,4 t = 5,70 |
| 16 | -OMe | -nPr | $-CONH(CH_2)_3NEt_2$ | - | $MH^+$ = 525,3 t = 5,73 |

(suite)

| Composés n° | $R_1$ | $R_2$ | $R_3$ | Sel | Caractérisation |
|---|---|---|---|---|---|
| 17 | -OMe | -OnPr | 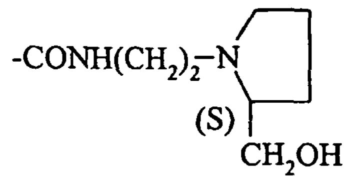 | - | F = 168°C $MH^+$ = 539,3 t = 5,68 |
| 18 | -OMe | -OnPr | 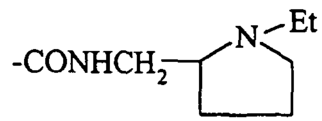 | - | F =166°C $MH^+$ = 523,3 t = 5,86 |
| 19 | -OMe | -OnPr | 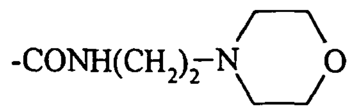 | 2HCl | $MH^+$ = 525,5 t = 6,55 |
| 20 | -OMe | -OnPr | 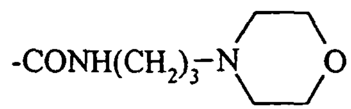 | 2HCl | $MH^+$ = 539,5 t = 6,55 |
| 21 | -OMe | -OnPr | 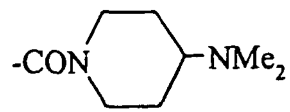 | - | $MH^+$ = 523,3 t = 5,56 |
| 22 | -OMe | -OnPr | 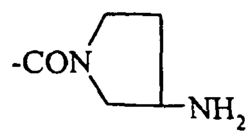 | $2CF_3$-$CO_2H$ | $MH^+$ = 481,5 t = 6,28 |
| 23 | -OMe | -OnPr | 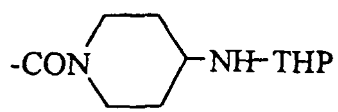 | - | F = 153°C $MH^+$ = 579,5 t = 6,32 |

(suite)

| Composés n° | $R_1$ | $R_2$ | $R_3$ | Sel | Caractérisation |
|---|---|---|---|---|---|
| 24 | -OMe | -OnPr | 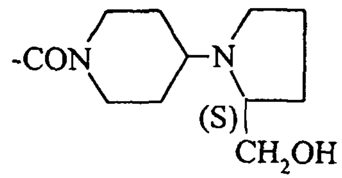 | - | F = 205°C $MH^+$ = 579,5 t = 6,31 |
| 25 | -OMe | -OnPr | 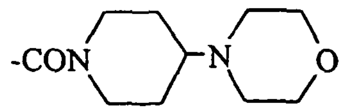 | - | F = 205°C |
| 26 | -OMe | -OnPr | 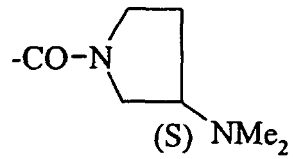 | - | F = 178°C |
| 27 | -OMe | -OnPr | 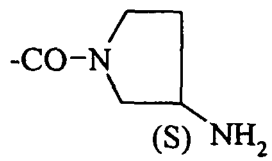 | $2CF_3$-$CO_2$ H | $MH^+$ = 481,2 t = 6,30 |
| 28 | -OMe | -OnPr | 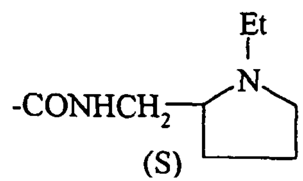 | - | F = 152°C $MH^+$ = 523,4 t = 6,52 |
| 29 | -OMe | -OnPr | 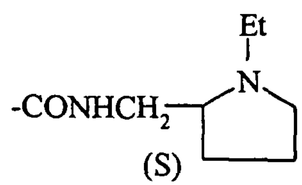 | 2HCl | $MH^+$ = 523,3 t = 6,58 |

(suite)

| Composés n° | $R_1$ | $R_2$ | $R_3$ | Sel | Caractérisation |
|---|---|---|---|---|---|
| 30 | -OMe | -OnPr | 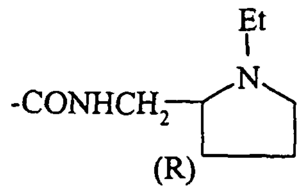 | - | F=150°C $MH^+$ = 523,4 t = 6,52 |
| 31 | -OMe | -OnPr | 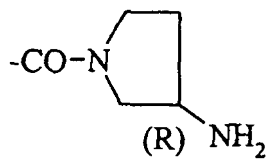 | $2CF_3$-$CO_2H$ | $MH^+$ = 481,5 t = 6,10 |
| 32 | -OMe | -OnPr | 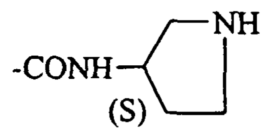 | - | $MH^+$ = 481,4 t = 6,25 |
| 33 | -OMe | -OnPr | $-(CH_2)_2-NEt_2$ | $2(HO_2C-CO_2H)$ | RMN |
| 34 | -OMe | -OnPr | 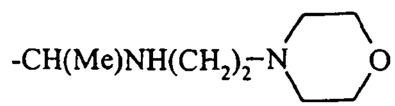 | - | F= 141°C |
| 35 | -OMe | -nBu | $-O(CH_2)_2NEt_2$ | 2HCl | $MH^+$ = 482,4 t = 6,46 |
| 36 | -OMe | -nPr | $-O(CH_2)_2NEt_2$ | 2HCl | $MH^+$ = 468,3 t = 6,17 |
| 37 | -OMe | -OEt | $-O(CH_2)_2NEt_2$ | 2HCl | $MH^+$ = 454,2 t = 5,84 |
| 38 | -OMe | -nPr | 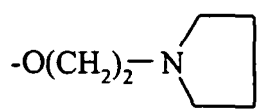 | - | $MH^+$ = 466,3 t = 6,12 |
| 39 | -OMe | -nPr | $-O(CH_2)_2NH$-THP | $2CF_3$-$CO_2H$ | $MH^+$ = 496,3 t = 6,03 |
| 40 | -OMe | -nPr | $-O(CH_2)_2NMe$-THP | - | $MH^+$ =·510,4 t=6,13 |
| 41 | -OMe | -iPr | $-O(CH_2)_2NEt_2$ | 2HCl | $MH^+$ = 468,4 t=6,16 |

(suite)

| Composés n° | $R_1$ | $R_2$ | $R_3$ | Sel | Caractérisation |
|---|---|---|---|---|---|
| 42 | -OMe | -Ph | $-O(CH_2)_2NEt_2$ | 2HCl | $MH^+$ = 502,2 t = 7,16 |
| 43 | -OMe | 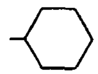 | $-O(CH_2)_2NEt_2$ | 2HCl | $MH^+$ = 508,5 t = 7,41 |
| 44 | -OEt | -Et | $-O(CH_2)_2NEt_2$ | 2HCl | F = 180°C $MH^+$ = 468,4 t = 6,89 |
| 45 | -OMe | $-CF_3$ | $-O(CH_2)_2NEt_2$ | 2HCl | $MH^+$ = 494,5 t = 6,68 |
| 46 | -OMe | -nBu | 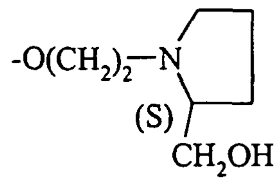 | - | $MH^+$ = 510,4 t = 7,11 |
| 47 | -OMe | 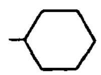 | $-O(CH_2)_2NH$-THP | 2HCl | $MH^+$ = 536,4 t = 7,31 |
| 48 | -OEt | *tert*-Pentyle | $-O(CH_2)_2NEt_2$ | - | F = 116°C $MH^+$ = 510,5 t = 7,60 |
| 49 | -OMe | -secBu | $-O(CH_2)_2NEt_2$ | 2HCl | F = 150-156°C $MH^+$ = 482,5 t = 7,24 |
| 50 | -OMe | -tertBu | $-O(CH_2)_2NEt_2$ | 2HCl | F = 149-151°C $MH^+$ = 482,4 t = 6,05 |
| 51 | -OMe | -nBu | 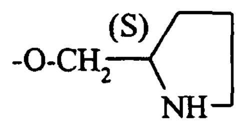 | 2HCl | $MH^+$ = 466,3 t = 7,09 |
| 52 | -OMe | -nBu | 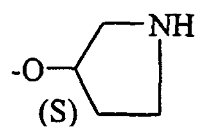 | 2HCl | $MH^+$ = 452,4 t = 7,01 |
| 53 | -OMe | 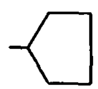 | $-O(CH_2)_2NEt_2$ | 2HCl | $MH^+$ = 494,4 t = 7,23 |

(suite)

| Composés n° | $R_1$ | $R_2$ | $R_3$ | Sel | Caractérisation |
|---|---|---|---|---|---|
| 54 | -OMe | -nBu | 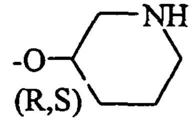 | 2HCl | $MH^+$ = 466,4 t=7,12 |
| 55 | -OMe | $-CHEt_2$ | $-O(CH_2)_2NEt_2$ | 2HCl | $MH^+$ = 496,4 t = 7,29 |
| 56 | -OMe | -nBu | 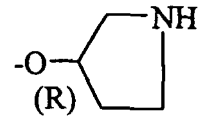 | 2HCl | $MH^+$ = 452,4 t = 6,98 |
| 57 | -OMe | 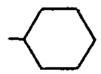 | 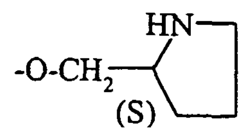 | 2HCl | $MH^+$ = 492,4 t = 7,33 |
| 58 | -OMe | -CH-(nPr)$_2$ | $-O(CH_2)_2NEt_2$ | 2HCl | $MH^+$ = 524,4 t = 7,84 |
| 59 | 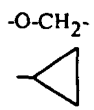 | -nBu | $-O(CH_2)_2NEt_2$ | 2HCl | $MH^+$ = 522,4 t = 7,81 |
| 60 | -OEt | 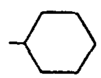 | 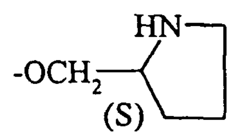 | 2HCl | $MH^+$ = 506,3 t = 7,53 |
| 61 | -OMe | -nBu | 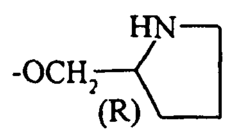 | 2HCl | $MH^+$ = 466,3 t = 6,93 |
| 62 | -OEt | -nBu | $-O(CH_2)_2NEt_2$ | 2HCl | $MH^+$ = 496,3 t = 7,56 |
| 63 | -OnPr | -nBu | $-O(CH_2)_2NEt_2$ | 2HCl | $MH^+$ = 510,3 t = 7,90 |

(suite)

| Composés n° | $R_1$ | $R_2$ | $R_3$ | Sel | Caractérisation |
|---|---|---|---|---|---|
| 64 | -OMe | -nBu | 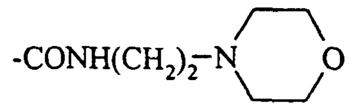 | - | $MH^+ = 523,3$ t = 6,22 |
| 65 | -OMe | -nBu | 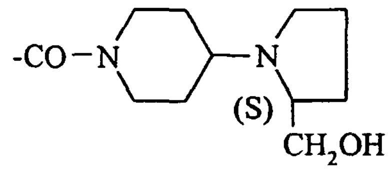 | - | F = 199°C $MH^+ = 577,5$ t = 6,83 |
| 66 | -OMe | -nBu | 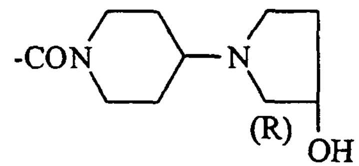 | - | $MH^+ = 563,3$ t = 6,84 |
| 67 | -OMe | -nBu | 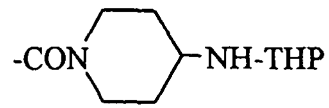 | - | F 160°C dec $MH^+ = 577,5$ t = 6,84 ; RMN |
| 68 | -OMe | -nBu | 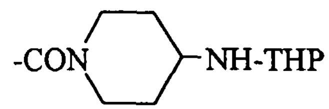 | 2HCl | F =181-189°C dec $MH^+ = 577,5$ t = 6,80 |
| 69 | -OMe | -Et | 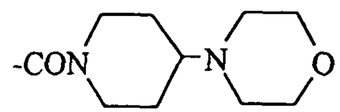 | - | RMN |

(suite)

| Composés n° | $R_1$ | $R_2$ | $R_3$ | Sel | Caractérisation |
|---|---|---|---|---|---|
| 70 | -OMe | -Et | 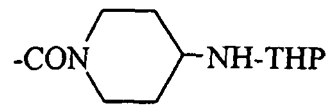 | - | RMN |
| 71 | -OMe | -Et | 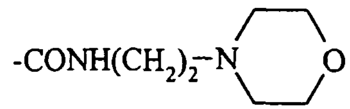 | - | F = 208°C $MH^+$ = 494 t = 6,39 |
| 72 | -OMe | -Et | 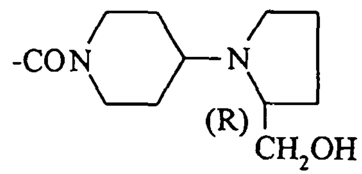 | - | $MH^+$ = 549,5 t = 6,35 |
| 73 | -OMe | -Et | 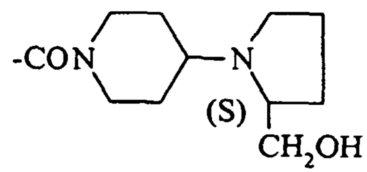 | - | $MH^+$ = 549,5 t = 6,35 |
| 74 | -OMe | -nBu | 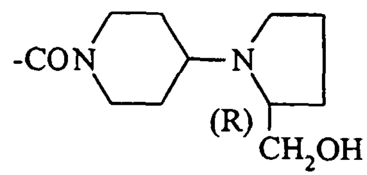 | - | F = 190°C $MH^+$ = 577,5 t = 6,98 |
| 75 | -OMe | -Et | 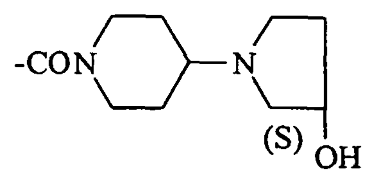 | - | F ≃ 140°C $MH^+$ = 535,4 t = 6,27 |

(suite)

| Composés n° | $R_1$ | $R_2$ | $R_3$ | Sel | Caractérisation |
|---|---|---|---|---|---|
| 76 | -OMe | -Et | 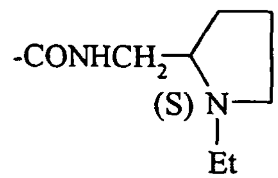 | - | F = 90°C $MH^+$ = 493,4 t = 6,45 |
| 77 | -OMe | -Et | 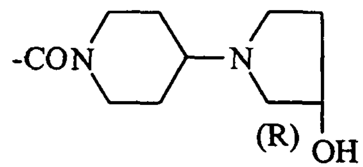 | - | F ≃ 143°C $MH^+$ = 535,4 t = 6,22 |
| 78 | -OMe | -Et | 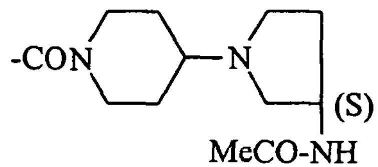 | - | F = 225°C $MH^+$ = 576,4 t = 6,16 |
| 79 | -OMe | -Et | 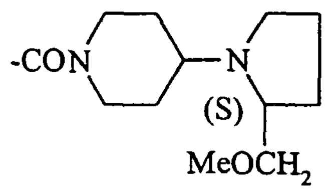 | - | F = 165°C $MH^+$ = 563,4 t = 6,42 |
| 80 | -OMe | -nBu | 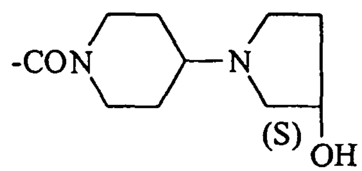 | - | F ≃ 129°C $MH^+$ = 563,5 t = 6,68 |

(suite)

| Composés n° | $R_1$ | $R_2$ | $R_3$ | Sel | Caractérisation |
|---|---|---|---|---|---|
| 81 | -OMe | -nBu | 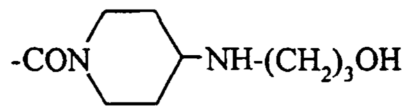 | - | F = 140°C $MH^+$ = 551,5 t = 6,73 |
| 82 | -OMe | -Me | 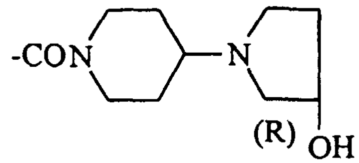 | - | F ≃ 155°C $MH^+$ = 521,4 t = 5,87 |
| 83 | -OMe | -nBu | 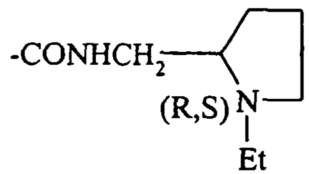 | - | F ≃ 76°C $MH^+$ = 521,4 t=7,13 |
| 84 | -OMe | -Me | 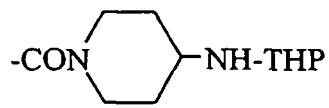 | - | F = 198°C $MH^+$ = 535,5 t = 5,96 |
| 85 | -OMe | -Me | 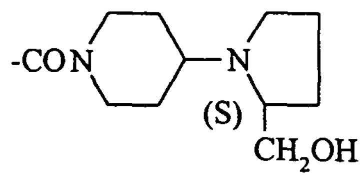 | - | F = 160°C $MH^+$ = 535,5 t = 5,93 |
| 86 | -OMe | -nBu | 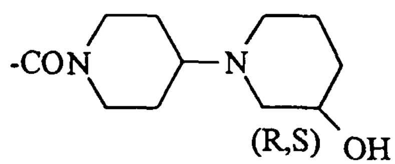 | - | F = 170°C $MH^+$ = 577,5 t = 6,80 |

(suite)

| Composés n° | $R_1$ | $R_2$ | $R_3$ | Sel | Caractérisation |
|---|---|---|---|---|---|
| 87 | -OMe | -nPr | 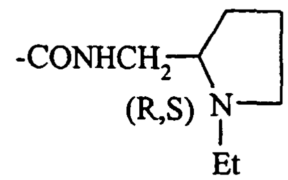 | - | F = 158°C $MH^+$ = 507,5 t = 6,76 |
| 88 | -OMe | -nPr | 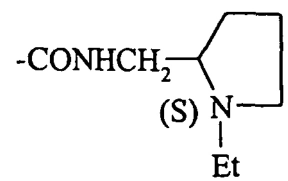 | - | F = 102°C $MH^+$ = 507,4 t = 6,68 |
| 89 | -OMe | -nPr | 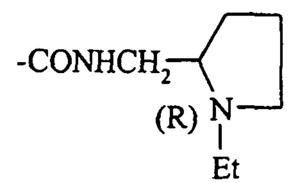 | - | F = 160°C $MH^+$ = 507,4 t = 6,79 |
| 90 | -OMe | -nBu | 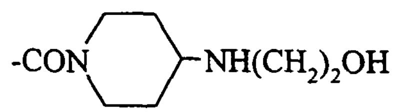 | - | $MH^+$ = 537,5 t = 6,67 |
| 91 | -OMe | -nBu | 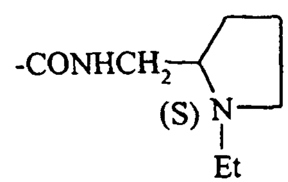 | - | F = 208°C $MH^+$ = 521,5 t = 7,30 |

(suite)

| Composés n° | $R_1$ | $R_2$ | $R_3$ | Sel | Caractérisation |
|---|---|---|---|---|---|
| 92 | -OMe | -nBu | 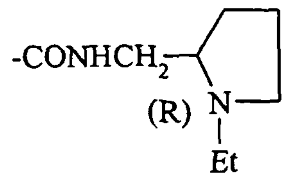 | - | F = 132°C $MH^+$ = 521,3 t = 7,04 |
| 93 | -OMe | -Et | 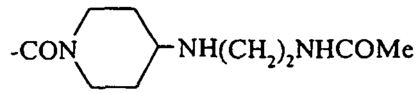 | - | $MH^+$ = 550,4 t = 6,30 |
| 94 | -OMe | -Et | 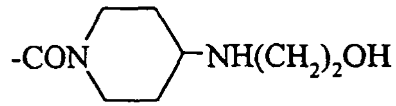 | - | $MH^+$ = 509,4 t = 6,28 |
| 95 | -OMe | -Et | 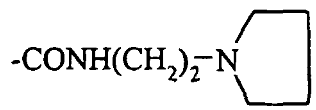 | - | F = 170°C $MH^+$ = 479,5 t = 6,44 |
| 96 | -OMe | -Et | $-CONH(CH_2)_2NH2$ | 2HCl | $MH^+$ = 425,4 t = 6,32 |
| 97 | -OMe | -Et | $-CONH(CH_2)_2NEt_2$ | 2HCl | F = 130°C dec $MH^+$ = 481,4 t = 6,55 |
| 98 | -OMe | -Et | 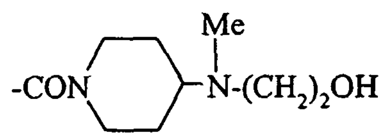 | - | F = 146°C $MH^+$ = 523,5 t = 6,29 |
| 99 | -OMe | -Et | $-CONH(CH_2)_2NHCOMe$ | - | F > 260°C $MH^+$ = 467,4 t = 8,04 |
| 100 | -OMe | -nBu | 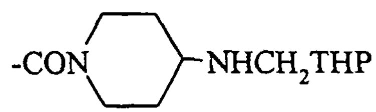 | - | F = 177°C $MH^+$ = 591,4 t = 5,95 |

(suite)

| Composés n° | $R_1$ | $R_2$ | $R_3$ | Sel | Caractérisation |
|---|---|---|---|---|---|
| 101 | -OMe | -Et | 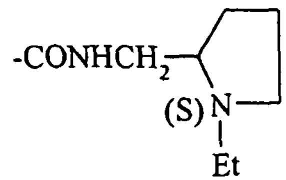 | 2HCl | $MH^+$ = 493,5 t = 6,59 |
| 102 | -OMe | -Et | 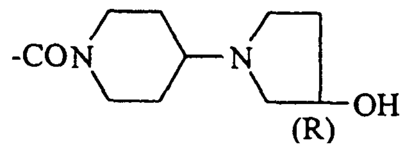 | 2HCl | F = 187°C $MH^+$ = 535,4 t = 6,12 |
| 103 | -OMe | -nBu | 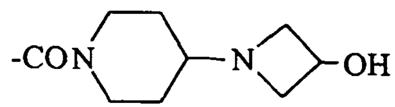 | 2HCl | $MH^+$ = 549,5 t=6,75 |
| 104 | -OMe | -nBu | 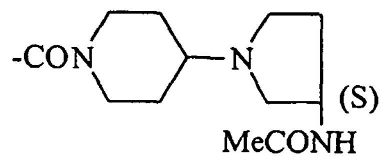 | - | F = 207°C $MH^+$ = 604,4 t = 6,74 |
| 105 | -OMe | -nBu | 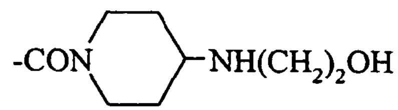 | 2HCl | $MH^+$ = 537,4 t = 6,69 |
| 106 | -OMe | -nBu | 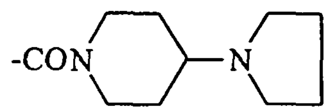 | - | $MH^+$ = 547,4 t = 6,83 |

(suite)

| Composés n° | $R_1$ | $R_2$ | $R_3$ | Sel | Caractérisation |
|---|---|---|---|---|---|
| 107 | -OMe | -nBu | 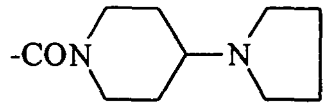 | 2HCl | $MH^+$ = 547,3 t = 6,93 |
| 108 | -OMe | -nBu | 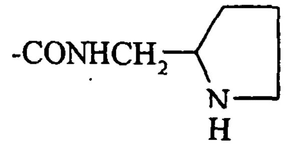 | 2HCl | F = 196°C $MH^+$ = 493,3 t = 6,84 |
| 109 | -OMe | -nBu | 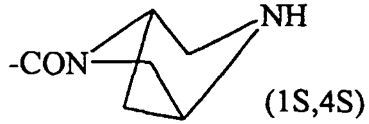 (1S,4S) | 2HCl | F = 192°C $MH^+$ = 492,4 t = 6,65 |
| 110 | -OMe | -nBu | 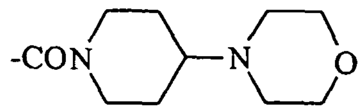 | - | MH + = 563,3 t = 6,84 |
| 111 | -OMe | -nBu | 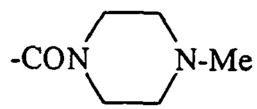 | - | F = 180°C $MH^+$ = 494,3 t = 6,80 |
| 112 | -OMe | -nBu | 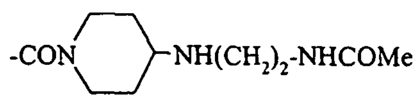 | - | $MH^+$ = 578,4 t = 6,80 |
| 113 | -OMe | -nBu | 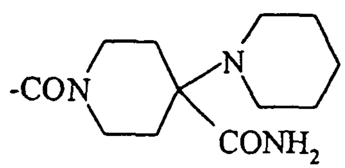 | - | F = 150°C $MH^+$ = 604,4 t = 6,91 |

(suite)

| Composés n° | $R_1$ | $R_2$ | $R_3$ | Sel | Caractérisation |
|---|---|---|---|---|---|
| 114 | -OEt | -Et | 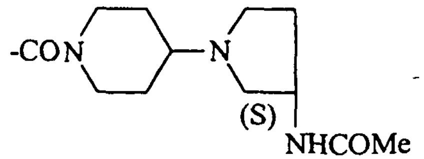 | - | F = 153°C $MH^+$ = 590,4 t = 6,41 |
| 115 | -OMe | -nBu | 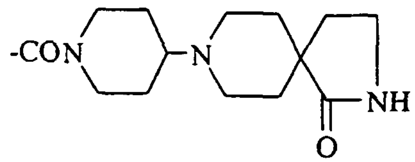 | - | $MH^+$ = 630,4 t = 6,74 |
| 116 | -OEt | -Et | 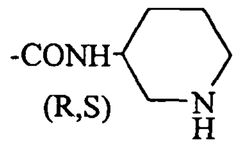 | 2HCl | F= 171°C $MH^+$ = 479,4 t = 6,57 |
| 117 | -OEt | -Et | 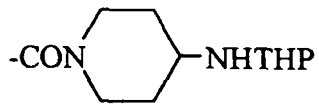 | - | F = 171°C $MH^+$ = 563,4 t = 6,50 |
| 118 | -OMe | -nBu | 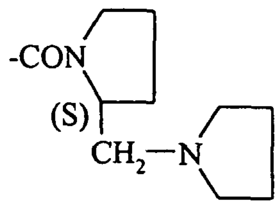 | - | $MH^+$ = 547,4 t = 7,18 |

(suite)

| Composés n° | $R_1$ | $R_2$ | $R_3$ | Sel | Caractérisation |
|---|---|---|---|---|---|
| 119 | -OEt | -Et | 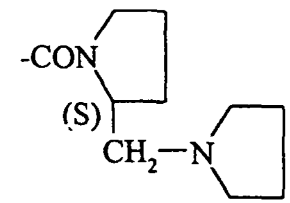 | 2HCl | $MH^+$ = 533,4 t = 6,75 |
| 120 | -OMe | -nBu | 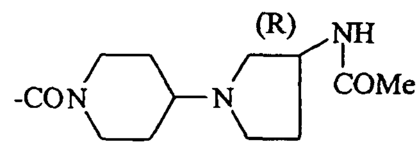 | - | F = 200°C dec $MH^+$ = 604,5 t = 6,65 |
| 121 | -OEt | -Et | 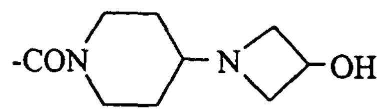 | - | F = 136°C $MH^+$ = 535,3 t = 6,37 |
| 122 | -OEt | -Et | 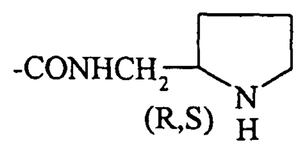 | 2HCl | F = 145°C $MH^+$ = 479,3 t = 6,52 |
| 123 | -OEt | -Et | 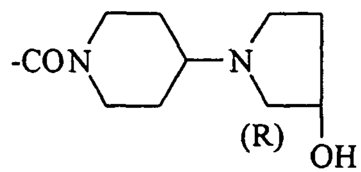 | - | F = 134°C $MH^+$ = 549,3 t = 6,34 |
| 124 | -OMe | -nBu | 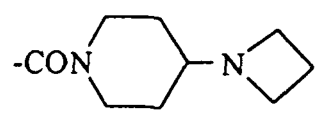 | - | $MH^+$ = 533,4 t = 6,91 |

(suite)

| Composés n° | $R_1$ | $R_2$ | $R_3$ | Sel | Caractérisation |
| --- | --- | --- | --- | --- | --- |
| 125 | -OEt | -Et | 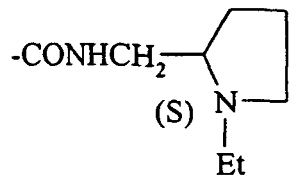 | 2HCl | F = 136°C $MH^+$ = 507,4 t = 6,75 |
| 126 | -OMe | -nBu | 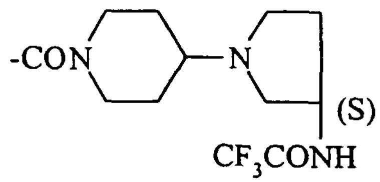 | - | $MH^+$ = 658,3 t = 7,14 |
| 127 | -OMe | -nBu | 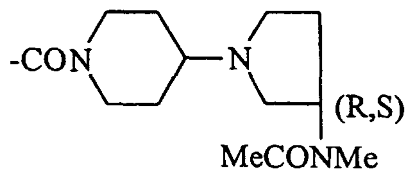 | 2HCl | $MH^+$ = 618,4 t = 6,79 |
| 128 | -OMe | -nBu | 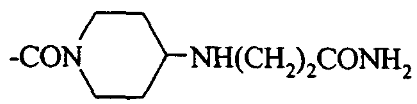 | 2HCl | $MH^+$ = 564,6 t = 6,69 |
| 129 | -OEt | -Et | 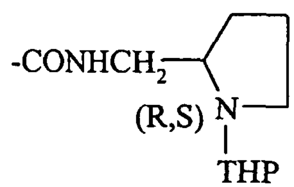 | - | F = 170°C $MH^+$ = 563,4 t = 6,74 |

(suite)

| Composés n° | $R_1$ | $R_2$ | $R_3$ | Sel | Caractérisation |
|---|---|---|---|---|---|
| 130 | -OMe | -nBu | 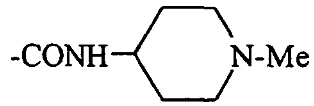 | - | F = 222°C $MH^+$ = 507,3 t = 6,80 |
| 131 | -OEt | 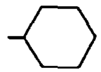 | 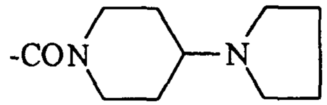 | - | F = 143°C $MH^+$ = 487,5 t = 7,53 |
| 132 | -OEt | 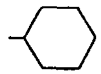 | 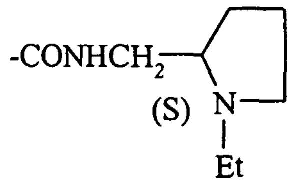 | - | F = 97°C $MH^+$ = 561,4 t = 7,74 |
| 133 | -OMe | -nBu | 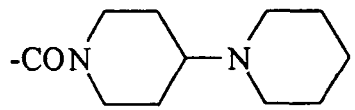 | - | F = 188°C $MH^+$ = 561,4 t = 6,99 |
| 134 | -OMe | 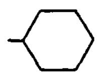 | 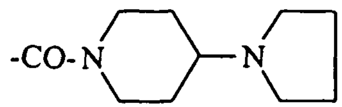 | - | F = 192°C $MH^+$ = 573,4 t = 7,16 |
| 135 | -OEt | -Et | 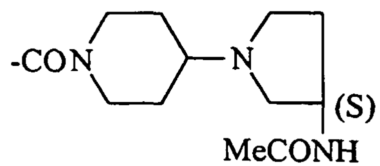 | 2HCl | $MH^+$ = 590,3 t = 6,49 |

(suite)

| Composés n° | $R_1$ | $R_2$ | $R_3$ | Sel | Caractérisation |
|---|---|---|---|---|---|
| 136 | -OMe | 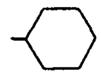 | 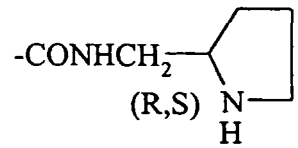 | 2HCl | $MH^+$ = 519,3 t = 7,03 |
| 137 | -OMe | 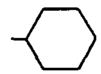 | 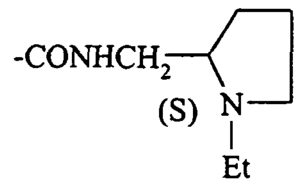 | - | F = 104°C $MH^+$ = 547,3 t = 7,18 |
| 138 | -OMe | -nBu | 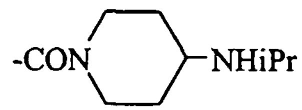 | 2HCl | $MH^+$ = 494,3 t = 6,96 |
| 139 | -OEt | 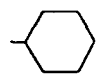 | 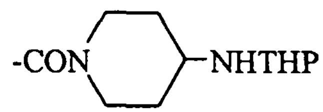 | 2HCl | $MH^+$ = 617,3 t = 7,32 |
| 140 | -OMe | 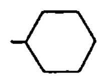 | 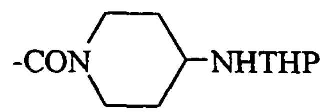 | 2HCl | $MH^+$ = 603,3 t = 7,03 |
| 141 | -OMe | -nPr | 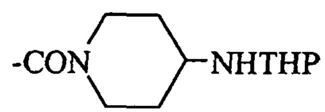 | - | $MH^+$ = 563,4 t = 6,42 |

(suite)

| Composés n° | $R_1$ | $R_2$ | $R_3$ | Sel | Caractérisation |
|---|---|---|---|---|---|
| 142 | -OMe | -nBu | 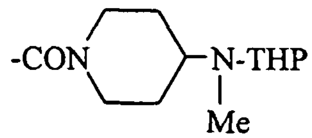 | - | $MH^+$ = 591,3 t = 6,84 |
| 143 | -OMe | -nBu | 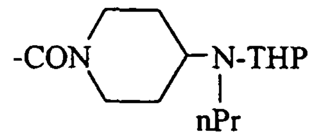 | - | $MH^+$ = 619,4 t = 7,07 |
| 144 | -OMe | 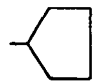 | 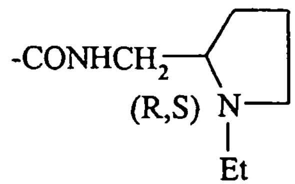 | - | F = 134°C $MH^+$ = 533,3 t = 7,03 |
| 145 | -OMe | -nBu | 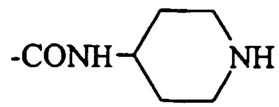 | 2HCl | $MH^+$ = 493,3 t = 6,81 |
| 146 | -OMe | -nPr | 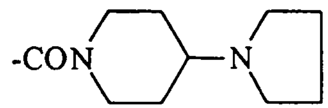 | - | F = 163°C $MH^+$ = 533,3 t = 6,56 |
| 147 | -OMe | -nBu | 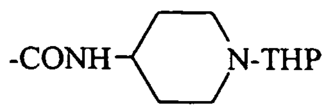 | - | F ≃ 170°C $MH^+$ = 577,3 t = 6,82 |

(suite)

| Composés n° | $R_1$ | $R_2$ | $R_3$ | Sel | Caractérisation |
|---|---|---|---|---|---|
| 148 | -OMe | 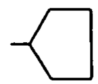 | 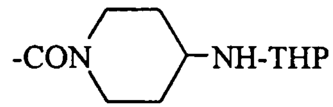 | 2HCl | F ≃ 180°C $MH^+$ = 589,3 t = 6,76 |
| 149 | -OMe | -nHex | 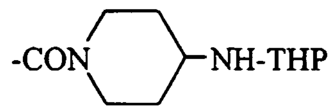 | 2HCl | $MH^+$ = 605,3 t = 7,35 |
| 150 | -OMe | 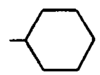 | 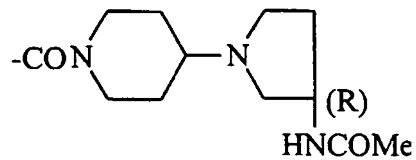 | - | F = 164°C dec $MH^+$ = 630,4 t = 6,97 |
| 151 | -OMe | -nBu | 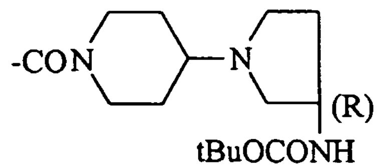 | - | F = 104°C $MH^+$ = 662,3 t = 7,33 |
| 152 | -OMe | -nBu | 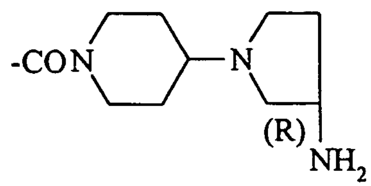 | 3HCl | F =188°C $MH^+$ = 561,9 t = 5,82 |
| 153 | -OMe | -nBu | 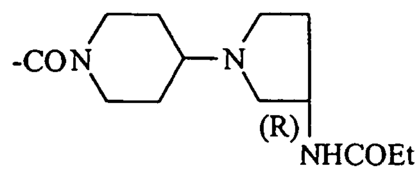 | - | F = 128°C $MH^+$ = 618,3 t = 6,84 |

(suite)

| Composés n° | $R_1$ | $R_2$ | $R_3$ | Sel | Caractérisation |
|---|---|---|---|---|---|
| 154 | -OMe | -nBu | 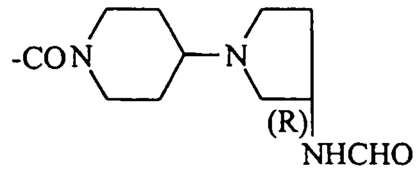 | - | F = 148°C $MH^+$ = 590,3 t = 6,73 |
| 155 | -OMe | 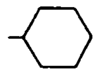 | 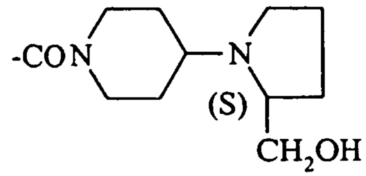 | 2HCl | $MH^+$ = 603,3 t = 7,08 |
| 156 | -OMe | -nBu | 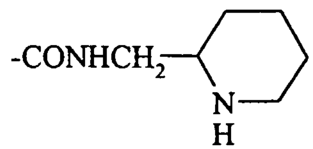 | - | F = 182°C $MH^+$ = 507,4 t = 6,89 |
| 157 | -OMe | -nPr | 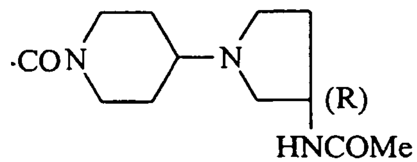 | - | F = 230°C $MH^+$ = 590,3 t = 6,39 |
| 158 | -OMe | -nPr | 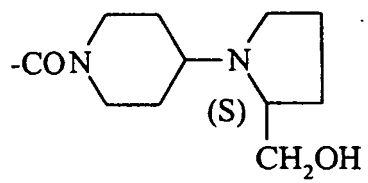 | - | F = 226°C $MH^+$ = 563,2 t = 6,51 |

(suite)

| Composés n° | $R_1$ | $R_2$ | $R_3$ | Sel | Caractérisation |
|---|---|---|---|---|---|
| 159 | -OMe | -nBu | 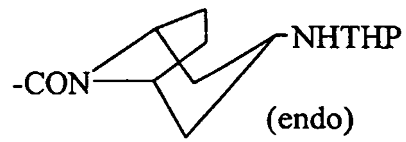 | - | F = 94°C dec $MH^+$ = 603,3 t = 6,86 |
| 160 | -OMe | 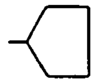 | 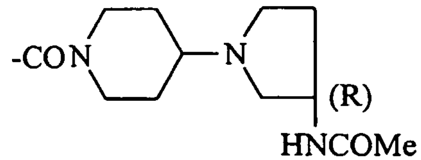 | - | F = 148°C $MH^+$ = 616,3 t = 6,66 |
| 161 | -OMe | -nBu | 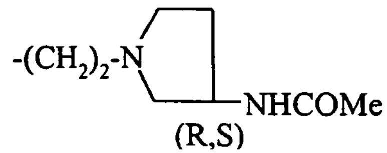 | - | F = 112°C $MH^+$ = 521,4 t = 6,98 |
| 162 | -OMe | -Et | $-(CH_2)_3NEt_2$ | 2HCl | $MH^+$ = 452,5 t = 6,76 |
| 163 | -OMe | -Et | 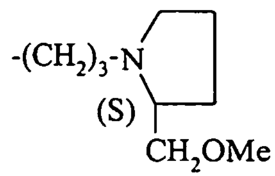 | - | $MH^+$ = 494,5 t = 6,95 |
| 164 | -OMe | -nBu | 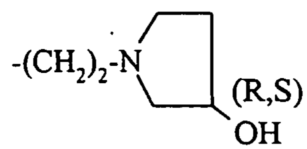 | - | $MH^+$ = 480,4 t=7,18 |

(suite)

| Composés n° | $R_1$ | $R_2$ | $R_3$ | Sel | Caractérisation |
|---|---|---|---|---|---|
| 165 | -OMe | -nBu | 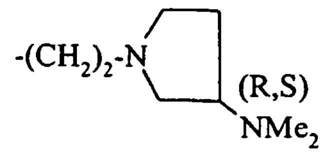 | - | $MH^+$ = 507,5 t = 6,25 |
| 166 | -OMe | -Et | 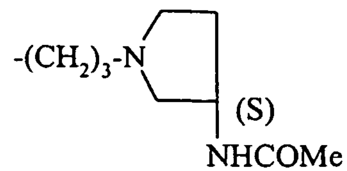 | - | $MH^+$ = 507,6 t = 6,62 |
| 167 | -OMe | -nBu | 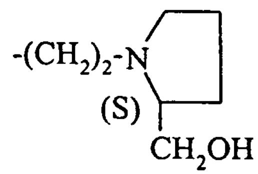 | - | $MH^+$ = 494,4 t = 7,76 |
| 168 | -OMe | -Et | 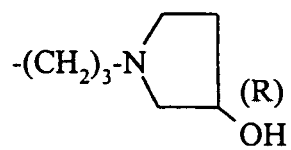 | - | $MH^+$ = 466,4 t = 6,57 |
| 169 | -OMe | -Et | 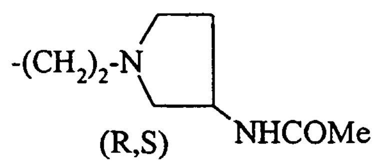 | - | $MH^+$ = 493,5 t = 6,93 |
| 170 | -OMe | -Et | 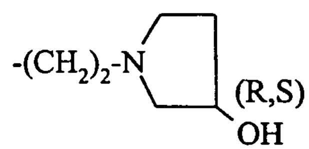 | - | $MH^+$ = 452,4 t = 6,94 |

(suite)

| Composés n° | $R_1$ | $R_2$ | $R_3$ | Sel | Caractérisation |
|---|---|---|---|---|---|
| 171 | -OMe | -Et | $-(CH_2)_3NH(CH_2)_2OH$ | - | F = 140°C dec $MH^+$ = 440,4 t = 7,11 |
| 172 | -OMe | -Et | $-(CH_2)_3NH$-THP | 2HCl | $MH^+$ = 480,5 t = 6,54 |
| 173 | -OMe | -Et | 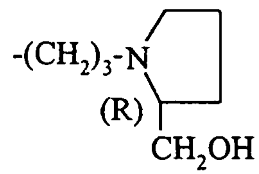 | - | $MH^+$ = 480,4 t = 6,49 |
| 174 | -OMe | -Et | 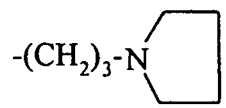 | 2HCl | $MH^+$ = 450,5 t = 6,54 |
| 175 | -OMe | -Et | 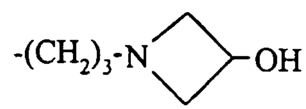 | 2HCl | $MH^+$ = 452,4 t = 6,35 |
| 176 | -OMe | -nBu | 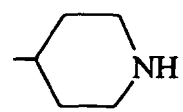 | 2HCl | F = 173°C $MH^+$ = 450,4 t=7,18 |
| 177 | -OMe | -nBu | 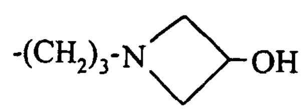 | - | $MH^+$ = 480,4 t = 7,13 |

(suite)

| Composés n° | $R_1$ | $R_2$ | $R_3$ | Sel | Caractérisation |
|---|---|---|---|---|---|
| 178 | -OMe | -nBu | 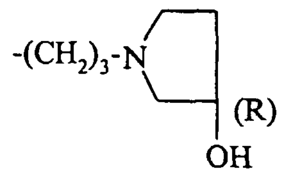 | - | $MH^+$ = 494,4 t = 7,04 |
| 179 | -OMe | -nBu | 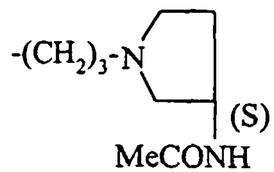 | - | $MH^+$ = 535,4 t = 6,99 |
| 180 | -OMe | -nBu | 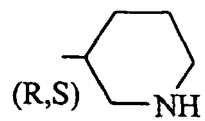 | 2HCl | F = 220°C $MH^+$ = 450,4 t = 7,19 |
| 181 | -OEt | -Et | 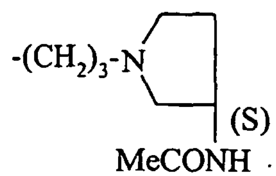 | - | $MH^+$ = 521,4 t = 6,63 |
| 182 | -OMe | -nBu | $-(CH_2)_3NH-THP$ | - | F = 150°C $MH^+$ = 508,4 t=7,10 |
| 183 | -OMe | -nBu | 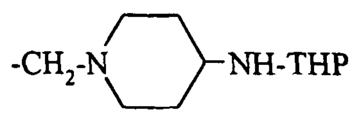 | - | F = 80°C dec $MH^+$ = 563,4 t = 5,80 |

(suite)

| Composés n° | $R_1$ | $R_2$ | $R_3$ | Sel | Caractérisation |
|---|---|---|---|---|---|
| 184 | -OMe | -nBu | 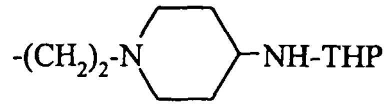 | - | F = 80°C dec $MH^+$ = 577,3 t = 5,94 |
| 185 | -OMe | -Et | 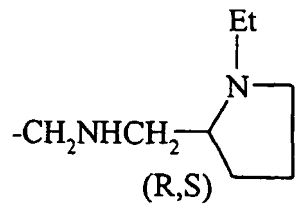 | 2HCl | F = 163°C dec $MH^+$ = 479,4 t = 6,14 |
| 186 | -OMe | -nBu | 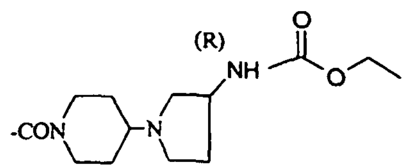 | HCl | F = 178°C $MH^+$ = 634 t = 7,03 |
| 187 | -OMe | -nBu | 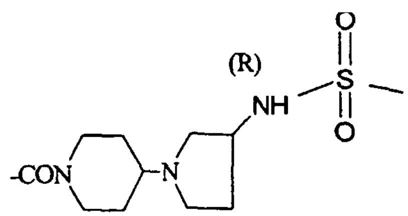 | - | F = 125°C $MH^+$ = 640 t = 6,88 |
| 188 | -OMe | -nBu | 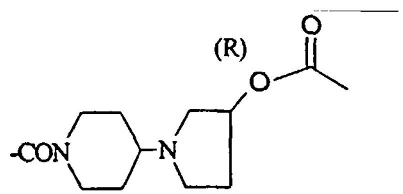 | - | F = 99°C $MH^+$ = 605 t = 6,92 |

(suite)

| Composés n° | $R_1$ | $R_2$ | $R_3$ | Sel | Caractérisation |
|---|---|---|---|---|---|
| 189 | -OMe | -nBu | 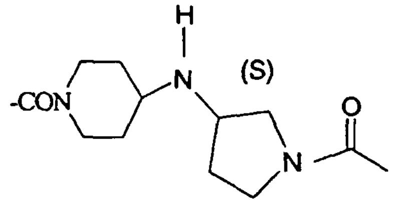 | - | $MH^+ = 604$ t = 6,68 |
| 190 | -OMe | -OnPr | $-CH_2NH$ 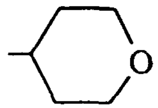 | - | F = 210°C |
| 191 | -OMe | -F | $-O(CH_2)_2NEt_2$ | HCl | F = 228°C |
| 192 | -OMe | -nHex | $-O(CH_2)_2NEt_2$ | HCl | $MH^+ = 510$ t = 7,73 |
| 193 | -OEt | -nHex | $-O(CH_2)_2NEt_2$ | HCl | F = 182°C $MH^+ = 524$ t = 8,05 |
| 194 | -OMe | 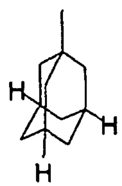 | $-O(CH_2)_2NEt_2$ | HCl | $MH^+ = 560$ t = 7,93 |
| 195 | -OEt | -nPr | $-O(CH_2)_2NEt_2$ | - | $MH^+ = 482$ t = 7,11 |
| 196 | -OMe | $-CF_2CF_3$ | $-O(CH_2)_2NEt_2$ | - | $MH^+ = 544$ t = 6,79 |
| 197 | -OMe | 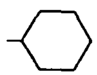 | 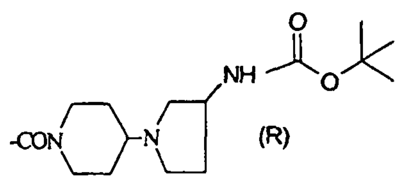 | - | $MH^+ = 688$ t = 7,53 |

(suite)

| Composés n° | $R_1$ | $R_2$ | $R_3$ | Sel | Caractérisation |
|---|---|---|---|---|---|
| 198 | -OMe | -nBu | 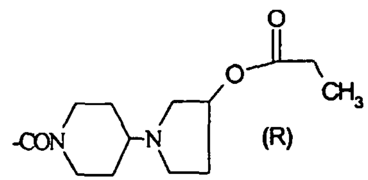 | - | F = 158°C $MH^+$ = 619 t = 7,09 |
| 199 | -OMe | 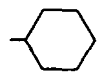 | 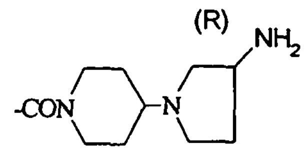 | - | $MH^+$ = 588 t = 6,06 |
| 200 | -OMe | 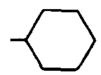 | 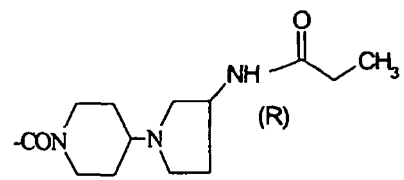 | - | $MH^+$ = 643 t = 7,06 |
| 201 | -OMe | -nBu | 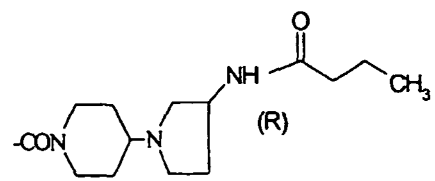 | - | $MH^+$ = 632 t=7,01 |
| 202 | -OMe | 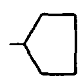 | 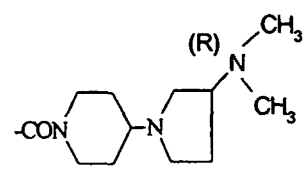 | - | $MH^+$ = 602 t = 5,94 |

(suite)

| Composés n° | $R_1$ | $R_2$ | $R_3$ | Sel | Caractérisation |
|---|---|---|---|---|---|
| 203 | -OMe | -nPr | 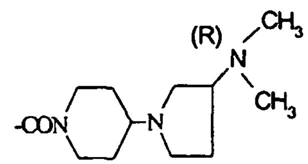 | - | $MH^+$ = 576 t = 5,64 |
| 204 | -OMe | -nBu | 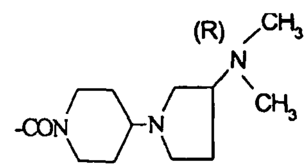 | - | $MH^+$ = 590 t = 5,91 |
| 205 | -OMe | 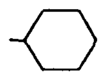 | 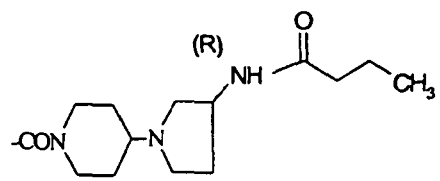 | - | $MH^+$ = 658 t = 7,28 |
| 206 | -OEt | 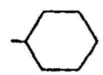 | 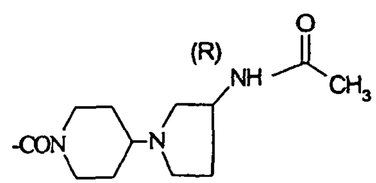 | - | F = 156°C $MH^+$ = 644 t = 7,33 |
| 207 | -OMe | 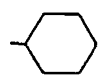 | 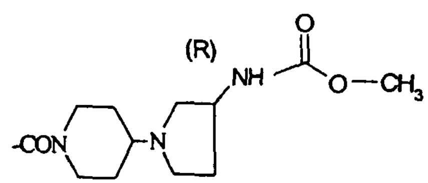 | - | F = 121°C $MH^+$ = 646 t = 7,24 |

(suite)

| Composés n° | $R_1$ | $R_2$ | $R_3$ | Sel | Caractérisation |
|---|---|---|---|---|---|
| 208 | -OMe | -nBu | 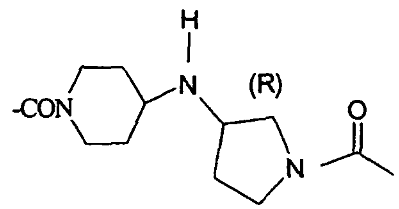 | - | F = 158°C $MH^+$ = 604 t = 6,78 |
| 209 | -OEt | 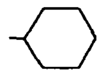 | 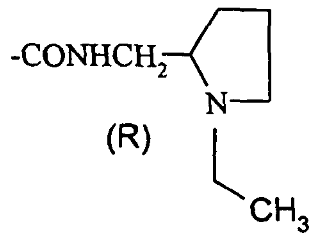 | - | $MH^+$ = 561 t = 7,77 |
| 210 | -OEt | -Et | 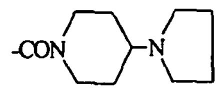 | - | F = 125°C $MH^+$ = 533 t = 6,53 |
| 211 | -OEt | -Et | 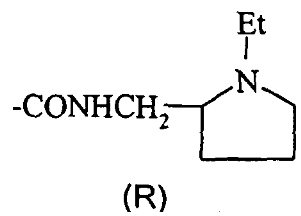 | - | F =151°C $MH^+$ = 507 t=6,75 |
| 212 | -OEt | -Et | 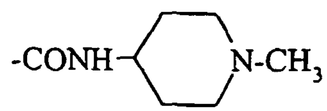 | - | F = 230°C $MH^+$ = 493 t = 6,69 |

(suite)

| Composés n° | $R_1$ | $R_2$ | $R_3$ | Sel | Caractérisation |
|---|---|---|---|---|---|
| 213 | -OMe | -nBu | 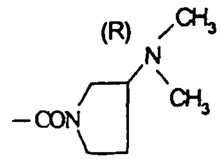 | - | F = 97°C $MH^+$ = 507 t = 6,76 |
| 214 | -OMe | -nBu | 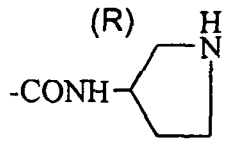 | - | F = 230°C $MH^+$ = 479 t = 6,84 |
| 215 | -OMe | -nBu | 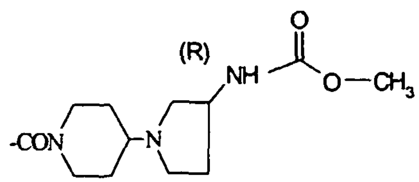 | - | F = 112°C $MH^+$ = 620 t = 6,89 |
| 216 | -OMe | -nBu | 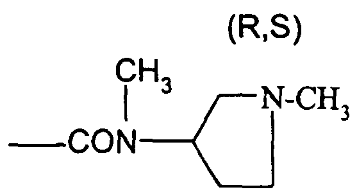 | - | F = 195°C $MH^+$ = 507 t = 6,23 |
| 217 | -OMe | -nBu | 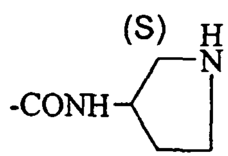 | - | F=221°C $MH^+$ = 479 t = 6,79 |

(suite)

| Composés n° | $R_1$ | $R_2$ | $R_3$ | Sel | Caractérisation |
|---|---|---|---|---|---|
| 218 | -OMe | -nBu | 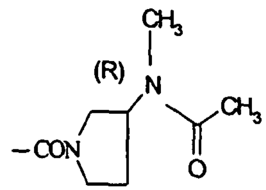 | - | F =142°C $MH^+$ = 535 t = 9,04 |
| 219 | -OMe | 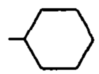 | 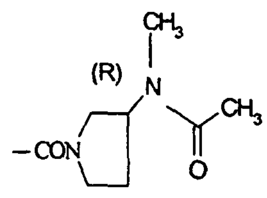 | - | F = 190°C $MH^+$ = 561 t = 9,8 |
| 220 | -OMe | 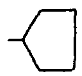 | 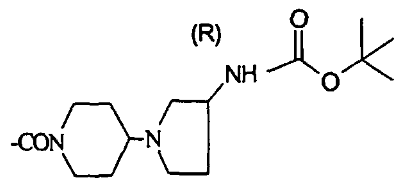 | - | F = 172°C $MH^+$ = 674 t = 7,38 |
| 221 | -OMe | -nHex | 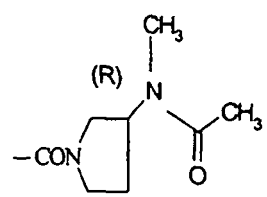 | - | F = 124°C $MH^+$ = 563 t = 9,96 |
| 222 | -OEt | 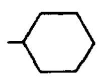 | 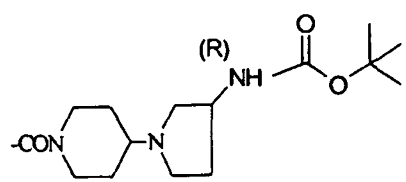 | - | F =176°C $MH^+$ = 702 t = 7,98 |

(suite)

| Composés n° | $R_1$ | $R_2$ | $R_3$ | Sel | Caractérisation |
|---|---|---|---|---|---|
| 223 | -OMe | 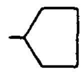 | 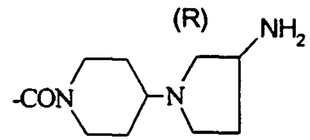 | - | F = 189°C $MH^+$ = 574 t=5,16 |
| 224 | -OEt | 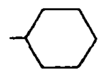 | 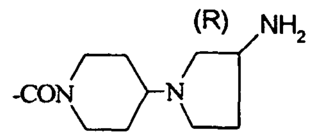 | - | F = 161°C $MH^+$ = 602 t = 6,39 |
| 225 | -OMe | 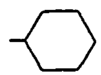 | 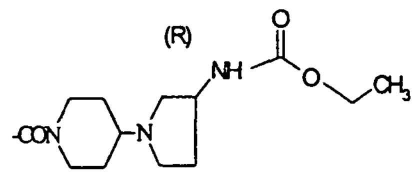 | - | F = 144°C $MH^+$ = 660 t = 7,37 |
| 226 | -OEt | 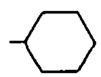 | 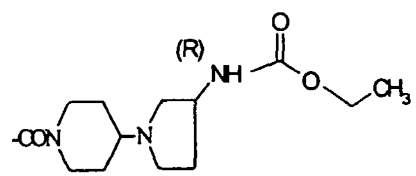 | - | F = 136°C $MH^+$ = 674 t = 7,59 |
| 227 | -OEt | 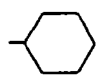 | 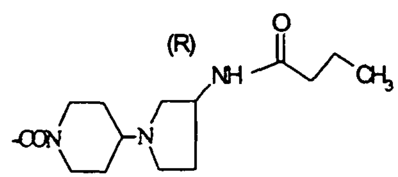 | - | F = 131°C $MH^+$ = 672 t=7,52 |
| 228 | -OEt | 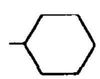 | 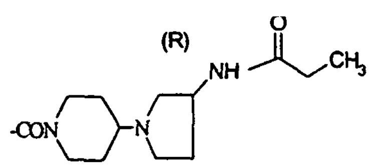 | - | F = 152°C $MH^+$ = 658 t = 7,47 |

(suite)

| Composés n° | $R_1$ | $R_2$ | $R_3$ | Sel | Caractérisation |
|---|---|---|---|---|---|
| 229 | -OMe | 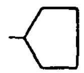 | 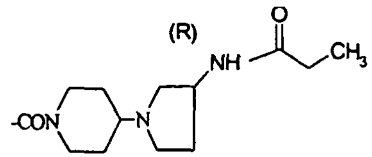 | - | F = 163°C $MH^+$ = 630 t = 7,16 |
| 230 | -OMe | 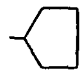 | 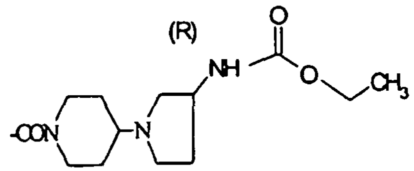 | - | F = 136°C $MH^+$ = 646 t = 7,09 |
| 231 | -OMe | 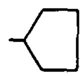 | 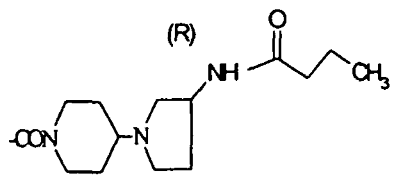 | - | F = 182°C $MH^+$ = 643 t = 7,29 |
| 232 | -OEt | -nBu | 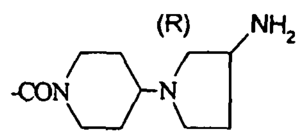 | - | F = 138°C $MH^+$ = 576 t = 6,06 |
| 233 | -OEt | -nBu | 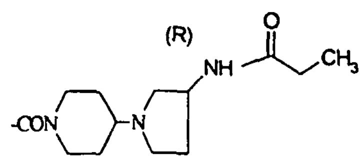 | - | F = 126°C $MH^+$ = 632 t=7,12 |
| 234 | -OEt | 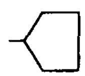 | 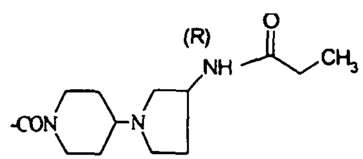 | - | $MH^+$ = 644 t = 7,33 |

(suite)

| Composés n° | $R_1$ | $R_2$ | $R_3$ | Sel | Caractérisation |
|---|---|---|---|---|---|
| 235 | -OEt | 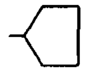 | 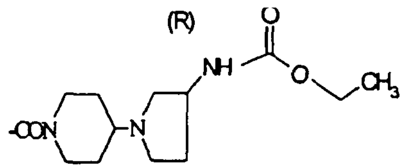 | - | $MH^+$ = 660 t = 7,51 |
| 236 | -OEt | 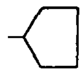 | 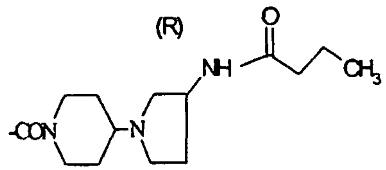 | - | F = 105°C $MH^+$ = 658 t = 7,27 |
| 237 | -OEt | 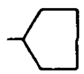 | 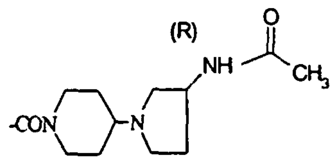 | - | F = 121°C $MH^+$ = 630 t = 7,04 |
| 238 | -OEt | -nBu | 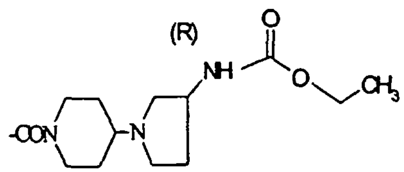 | - | F = 102°C $MH^+$ = 648 t = 7,48 |
| 239 | -OEt | -nBu | 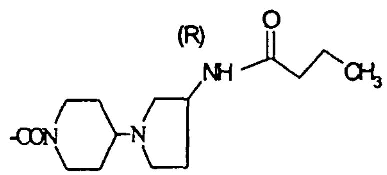 | - | F = 129°C $MH^+$ = 646 t = 7,42 |

(suite)

| Composés n° | $R_1$ | $R_2$ | $R_3$ | Sel | Caractérisation |
|---|---|---|---|---|---|
| 240 | -OMe | -nBu | 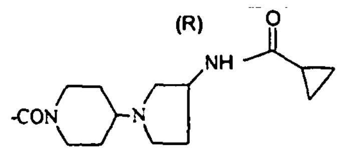 | - | F = 124°C $MH^+$ = 630 t = 7,59 |
| 241 | -OMe | -nBu | 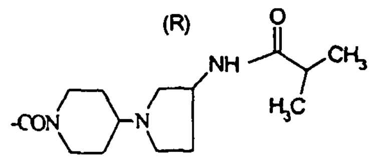 | - | F = 135°C $MH^+$ = 632 t = 7,62 |
| 242 (4.1.2.) | -OMe | -OnPr | 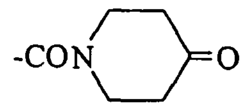 | - | F = 196°C |
| 243 (4.1.6.) | -OMe | 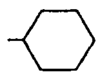 | 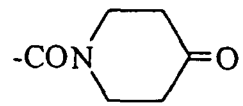 | - | F = 186°C |
| 244 (4.1.7.) | -OEt | 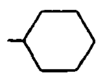 | 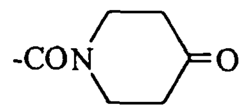 | - | F = 236°C |
| 245 | -OMe | 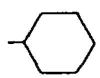 | 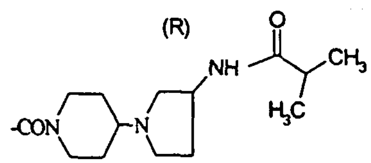 | - | F = 154°C |

(suite)

| Composés n° | $R_1$ | $R_2$ | $R_3$ | Sel | Caractérisation |
|---|---|---|---|---|---|
| 246 | -OMe | 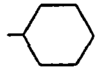 | 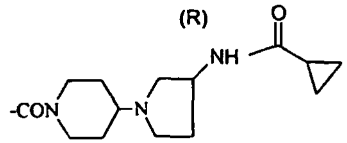 | - | F = 158°C |
| 247 | -OEt | -nBu | 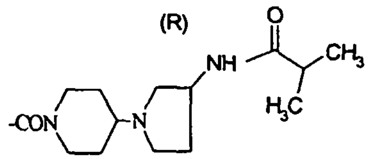 | - | F = 116°C |
| 248 | -OEt | -nBu | 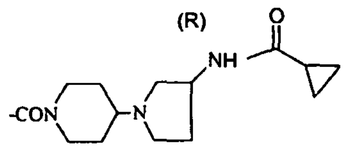 | - | F = 118°C |
| 249 | -OEt | 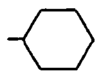 | 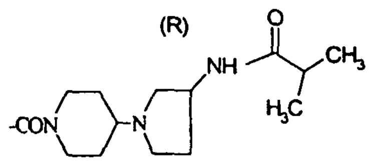 | - | F = 135°C |
| 250 | -OEt | 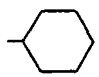 | 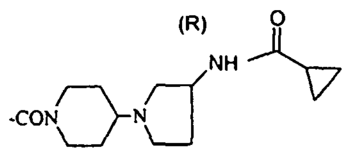 | - | F = 141°C |
| 251 | -OMe | -nBu | 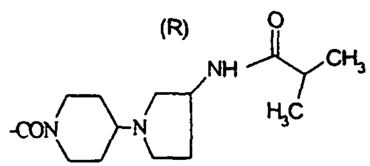 | - | F = 119°C |

(suite)

| Composés n° | $R_1$ | $R_2$ | $R_3$ | Sel | Caractérisation |
|---|---|---|---|---|---|
| 252 | -OMe | -nBu | 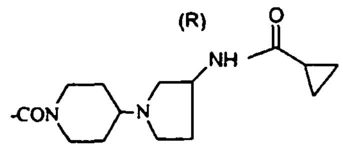 | - | F = 109°C |
| 253 | -OMe | -nBu | 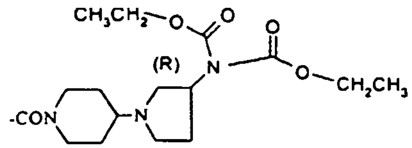 | HCl | F = 148°C |
| 254 | -OMe | -nBu | 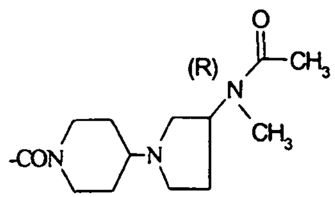 | - | F = 118°C |
| 255 | -OMe | -nBu | 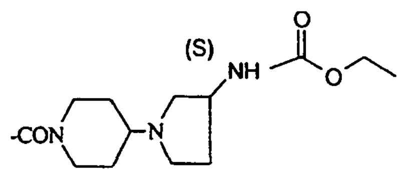 | HCl | F = 176°C |

Spectre de RMN : Composé 1 : 0,98 ppm : t : 3H ; 1,24 ppm : t : 6H ; 1,72 ppm : sext : 2H ; 3,1-3,3 ppm : m : 4H ; 3,4-3,6 ppm : m : 2H ; 3,8-4,0 ppm : m : 5H ; 4,50 ppm : t : 2H ; 6,87 ppm : d.d : 1H ; 7,04 ppm : d : 1H ; 7,16 ppm : d : 2H ; 7,7-7,8 ppm : m : 2H ; 8,16 ppm : d : 2H ; 10,6 ppm : se : 1H ; 12,5 ppm : se : 1H.

Spectre de RMN : Composé 33 : 1,00 ppm : t : 3H ; 1,26 ppm : t : 6H ; 1,74 ppm : sext : 2H ; 2,9-3,5 ppm : m : 8H ; 3,8-4,0 ppm : m : 5H ; 6,88 ppm : d.d : 1H ; 7,04 ppm : d : 1H ; 7,5 ppm : d : 2H ; 7,7-7,8 ppm : m : 2H ; 8,14 ppm : d : 2H.

Spectre de RMN : Composé 67 : 0,80 ppm : t : 3H ; 0,95-1,95 ppm : m : 12H ; 2,45 ppm : t : 2H ; 2,55-4,4 ppm : m : 13H ; 6,95 ppm : d : 1H ; 7,05 ppm : d de d : 1H ; 7,45 ppm : d : 2H ; 7,65 ppm : s : 1H ; 7,90 ppm : s : 1H ; 8,1 ppm : d : 2H.

Spectre de RMN : Composé 69 : 1,1 ppm : t : 3H ; 1,15-1,90 ppm : m : 4H ; 2,20-4,50 ppm : m : 17H ; 6,95 ppm : d : 1H ; 7,05 ppm : dd : 1H ; 7,45 ppm : d : 2H ; 7,60 ppm : s : 1H ; 7,95 ppm : s : 1H ; 8,1 ppm : d : 2H ; 12,65 ppm : se : 1H.

Spectre de RMN : Composé 70 : 0,85-1,95 ppm : m : 11H ; 2,30-4,4 ppm : m : 15H ; 6,95 ppm : d : 1H ; 7,05 ppm : dd : 1H ; 7,40 ppm : d : 2H ; 7,60 ppm : s : 1H ; 7,90 ppm : s : 1H ; 8,1 ppm : d : 2H.

Les composés selon l'invention ont fait l'objet d'essais pharmacologiques permettant de déterminer leur effet modulateur de l'activité des récepteurs aux chimiokines.

Les chimiokines sont des protéines de bas poids moléculaire qui appartiennent à la famille des cytokines proinflammatoires et sont impliquées dans le chimiotactisme des leucocytes et des cellules endothéliales. Les chimiokines contrôlent de nombreux processus biologiques et sont associées à des désordres inflammatoires apparaissant lors des états de stress, lors de blessures ou d'infections ; la modulation des effets des chimiokines permet de prévenir ou de traiter des pathologies comme l'asthme, l'arthrite, les allergies, les maladies auto-immunes, l'athérosclérose ou l'angiogénèse (C.D. Paavola et al., J. Biol. Chem., 1998, 273, (50), 33157-33165).

Parmi les chimiokines, on distingue le hMCP-1 (de l'anglais human Monocyte Chemotactic Protein) qui appartient au groupe des CC chimiokines et dont les actions sont médiées par le récepteur CCR2b.

On a mesuré l'activité inhibitrice des composés selon l'invention sur des cellules exprimant le récepteur CCR2b humain. La concentration d'agoniste naturel hMCP-1 qui inhibe 50 % ($CI_{50}$) de l'activité du récepteur CCR2b est de 0,57 nM. Les composés selon l'invention présentent une $CI_{50}$ généralement inférieure à 0,1 $\mu$M.

Par exemple, le composé n° 12 a présenté une $CI_{50}$ de 0,081 $\mu$M;

le composé n° 244 a présenté une $CI_{50}$ de 0,088 $\mu$M;

le composé n° 203 a présenté une $CI_{50}$ de 0,093 $\mu$M.

On a également mesuré l'inhibition du chimiotactisme sur des cellules monocytaires THP-1 humaines (commercialisées par DSMZ - Allemagne) en utilisant une technique adaptée de celle décrite par A. Albini et al., Cancer Res., 1987, 47, 3239-3245. Dans ces conditions le hMCP-1 présente une $CI_{50}$ de 6 nM. Les composés selon l'invention présentent une $CI_{50}$ généralement inférieure à 1 $\mu$M.

L'inhibition du chimiotactisme par les composés selon l'invention est le signe de leur activité antagoniste sur les récepteurs des chimiokines et en particulier du CCR2b.

Il apparaît donc que les composés selon l'invention sont des antagonistes de l'effet des chimiokines, en particulier du hMCP-1.

On a également mesuré l'activité inhibitrice des composés selon l'invention sur des PBMC (Peripheral Blood Mononuclear Cells) infectés par le virus VIH-1 Bal, selon une technique adaptée de celle décrite par V. Dolle et col., J. Med. Chem., 2000, 43, 3949, 3962. Selon cette technique, les PBMC sont infectés par le VIH-1 Bal puis les composés à tester sont ajoutés dans le milieu de culture pendant 5 jours. Au terme de cette exposition, on mesure dans le surnageant le taux de transcriptase reverse qui est corrélé avec le niveau de réplication virale dans les cellules.

Dans ces conditions, l'AZT, molécule de référence qui inhibe la réplication virale présente une CI50 inférieure à 0,1 $\mu$M. Les composés selon l'invention présentent également des CI50 généralement inférieures à 0,1 $\mu$M. Par exemple, le composé n° 104 a montré une CI50 de 0,063 $\mu$M.

Les composés selon l'invention peuvent donc être utilisés pour la préparation de médicaments, en particulier de médicaments antagonistes de l'effet des chimiokines.

Ainsi, selon un autre de ses aspects, la présente invention a pour objet des médicaments qui comprennent un composé de formule (I), ou un sel d'addition de ce dernier à un acide pharmaceutiquement acceptable, ou encore un hydrate ou un solvat.

Ces médicaments trouvent leur emploi en thérapeutique, notamment dans la prévention et le traitement de différentes pathologies telles que :

- les maladies et les syndromes immuno-inflammatoires aigus et chroniques comme l'athérosclérose, les resténoses, les maladies pulmonaires chroniques, en particulier COPD (de l'anglais chronic obstructive pulmonary disease) ; le syndrome de détresse respiratoire ; l'hyperactivité bronchique; les colites; la silicose ; les pathologies fibreuses, les fibroses pulmonaires, les fibroses kystiques ; les infections virales ou bactériennes, SIDA, méningite, malaria,

lèpre, tuberculose, herpes, infections par cytomégalovirus; les chocs septiques, la septicémie, les chocs endotoxiques ; les rejets de greffes ; les pathologies osseuses telles que l'ostéoporose, les ostéoarthrites ; les conjonctivites ; les dermatites atypiques ou de contact; les eczémas ; les glomérulonéphrites ; les pancréatites ; les colites ulcéreuses, les maladies auto-immunes comme la polyarthrite rhumatoïde, la sclérose en plaques, la sclérose amyotrophique latérale, la maladie de Crohn, le lupus érythémateux, la sclérodermie, le psoriasis; la maladie de Parkinson; la maladie d'Alzheimer; le diabète ; la cachexie; l'obésité;

- le traitement de la douleur, en particulier neuropathique et inflammatoire;
- les maladies allergiques comme les maladies respiratoires allergiques, l'asthme, les rhinites, l'hypersensibilité pulmonaire, l'hypersensibilité retardée;
- les maladies et les désordres dans lesquels les processus angiogéniques sont impliqués comme les cancers (angiogénèse intratumorale), les maladies rétiniennes (dégénérescence maculaire liée à l'age : DMLA);
- les pathologies cardiaques : choc hémodynamique ; les ischémies cardiaques ;

les attaques de réinfusion post-ischémique ; l'infarctus du myocarde, la thrombose coronarienne, l'insuffisance cardiaque, l'angine de poitrine.

Selon un autre de ses aspects, la présente invention concerne des compositions pharmaceutiques comprenant, en tant que principe actif, un composé selon l'invention, Ces compostions pharmaceutiques contiennent une dose efficace d'au moins un composé selon l'invention, ou un sel pharmaceutiquement acceptable, un hydrate ou solvat dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, infra-veineuse, topique, local, intratrachéale, intranasale, transdermique ou rectale, le principe actif de formule (Ia) ci-dessus, ou son sel, solvat ou hydrate éventuel, peut être administré sous forme unitaire d'administration, en mélange avec des excipients pharmaceutiques classiques, aux animaux et aux êt.res humains pour la prophylaxie ou le traitement des troubles ou des maladies ci-dessus

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Par voie orale, la dose de principe actif administrée par jour peut atteindre 0,1 à 1000 mg/kg, en une ou plusieurs prises.

Il peut y avoir des cas particuliers où des dosages plus élevés ou pas faibles sont appropriés: de tels dosages ne sortent pas du cadre de l'invention. Selon la pratique habituelle, le dosage approprie à chaque patient est détermine par le médecin selon le mode d'administration, le poids et la réponse dudit patient.

## Revendications

1. Composés répondant à la formule (Ia) :

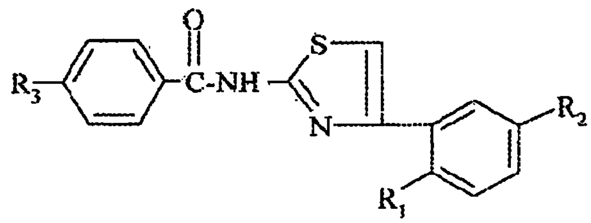

(Ia)

dans laquelle:

- $R_1$ représente un atome d'halogène, un groupe $(C_1-C_4)$alkyle, hydroxyle, $(C_1-C_4)$alcoxy, $(C_3-C_8)$cycloalkyloxy, allyloxy, cyclopropylméthoxy, $(C_1-C_4)$alkylthio ; et/ou
- $R_2$ représente un atome d'halogène, un groupe $(C_1-C_8)$alkyle, trifluoromethyle, $(C_3-C_{10})$-cycloalkyle, phényle, $(C_1-C_8)$alcoxy, allyloxy, $(C_3-C_8)$cycloalkylméthoxy, $(C_3-C_8)$cycloalkyloxy, $(C_3-C_8)$cycloalkylméthyle; et/ou
- $R_3$ représente un groupe choisi parmi :

g)

a1) -O-$(C_2-C_4)$alk-A;
a2) -O-$(C_1-C_4)$alk-B;
a3) -O-E ;

h) -$(C_1-C_4)$alk-A ;
i) -B ;
j)

d1) -$(C_1-C_4)$alk-$NR_4$-$(C_2-C_3)$alk-A ;
d2) -$(C_1-C_4)$alk-$NR_4$-$(C_1-C_3)$alk-B ;

k)

e1) -$CONR_4$-$(C_2-C_4)$alk-A ;
e2) -$CONR_4$-$(C_1-C_4)$alk-B ;
e3) -$CONR_4$-E ;

l)

f1) -CO-D-$(C_1-C_2)$alk-A ;
f2) -CO-G-A ;
f3)

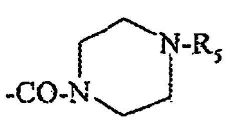

;

f4)

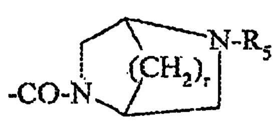

;

f5)

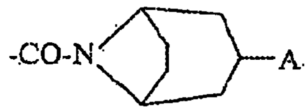 ;

f6)

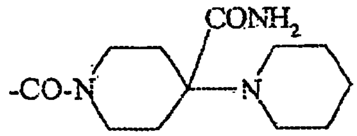 ;

f7)

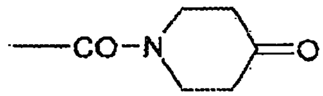 ;

- $R_4$ représente un atome d'hydrogène ou un groupe $(C_1-C_4)$alkyle ;
- A représente un groupe $NR_5R_6$ ;
- B représente un groupe

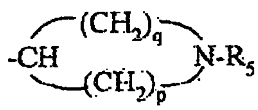 ;

- D représente un groupe

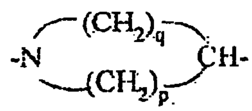 ;

- E représente un groupe

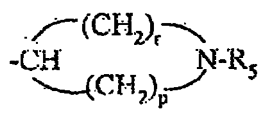 ;

- G représente un groupe

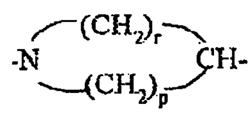 ;

- $R_5$ et $R_6$ représentent chacun indépendamment l'un de l'autre un atome d'hydrogène, un groupe $(C_1-C_6)$ alkyle, allyle, $(C_2-C_4)$ alk-O-$(C_1-C_4)$alkyle, $(C_2-C_4)$alk-OH, $(C_1-C_3)$alk-CON$(R_4)_2$, $(C_2-C_3)$alk-NHCO-$(C_1-C_4)$alkyle, $(C_3-C_7)$cycloakyle, $(C_3-C_7)$cycloalkylméthyle, -CO-$(C_1-C_4)$alkyle, benzyle, pyrrolidinyle éventuellement substitués par un groupe -CO$(C_1-C_4)$alkyle, tétrahydropyranyle, tétrahydropyranylméthyle, diméthyltétrahydropyranyle, tétrahydrofuryle, tétrahydrofurylméthyle;
- ou $R_5$ et $R_6$ ensemble avec l'atome d'azote auquel ils sont liés constituent un radical hétérocyclique choisi parmi : aziridinyle, azétidinyle, pyrrolidinyle, pipéridinyle, perhydroazépinyle, morpholinyle, pipérazinyle, tropanyle, quinuclidinyle, 2-azabicyclo[2,2,1]heptanyle, 2-azabicyclo[2,2,2]octanyle, lesdits radicaux hétérocycliques

étant non substitués ou substitués par un groupe phényle, halogénophényle, trifluorométhylphényle, trifluorométhyle, hydroxy, methoxy, hydroxyméthyle, méthoxyméthyle, formamido, trifluoroacétylamino, un groupement $-NR_4R_7$, tetrahydropyran-4-ylamino, $-CON(R_4)_2$, $-CONR_4R'_4$,$-CH_2CON(R_4)_2$ , $(C_1-C_4)$alkyle-$CONR_4$-, $(C_3-C_8)$cycloalkyle-$CONR_4$-, $(C_1-C_4)$alkyle-$OCONR_4$-, $((C_1-C_4)$alkyle-$OCO)_2N$-, $(C_1-C_4)$alkyle-COO- ; ou substitués par un ou plusieurs groupes méthyle ;
- $R'_4$ représente un groupe $(CH_2)_s$ lié à l'atome de carbone porteur de $-CONR_4R'_4$ ;
- $R_7$ représente un atome d'hydrogène, un $(C_1-C_4)$alkyle, un groupe $-SO_2CH_3$ ou $R_4$ et $R_7$ ensemble avec l'atome d'azote auquel ils sont liés constituent un radical pyrrolidinyle ou piperidinyle ;
- p représente 1, 2, 3, 4 ou 5 ;
- q représente 0, 1 ou 2 ;
- r représente 1 ou 2 ;
- s représente 2 ou 3 ;
- p + q étant inférieur ou égal à 5 ;
- p + r étant inférieur ou égal à 5 ;
- alk représente un alkylène ; à l'état de base ou de sel d'addition à un acide, ainsi qu'à létat d'hydrate ou de solvant.

**2.** Composés selon la revendication 1, de formule (Ia) dans laquelle :

- $R_1$ représente un groupe $(C_1-C_4)$alcoxy, cyclopropylméthoxy, $(C_1-C_4)$alkylthio ;
et/ou
- $R_2$ représente un atome d'halogène, un groupe $(C_1-C_8)$alkyle, trifluorométhyle, $(C_3-C_{10})$cycloalkyle ou $(C_1-C_8)$alcoxy ; et/ou
- $R_3$ représente un groupe f2 ou e2
à l'état de base ou de sel d'addition un acide, ainsi qu'à l'état d'hydrate ou de solvat.

**3.** Composés selon la revendication 1, de formule (Ia) :

- 4-((4-[3-(R)-(acétylamino)pyrrolidin-1-yl]pipéridin-1-yl)carbonyl)-N-[4-(5-butyl-2-méthoxyphényl)-1,3-thiazol-2-yl]benzamide;
- ester éthylique (1-(1(4-(4-(5-Butyl-2-methoxy-phenyl)-thiazol-2-ylcarbamoyl)-piperidin-4-yl)-pyrrolidin-3-yl) de l'acide carbarmique;
- N-(4-(5-butyl-2-methoxy-phenyl)-thiazol-2-yl)-4-(4-(3-(R) acetylamino-pyrrolidin-1-yl)-piperidine-1-carbonyl)-benzamide;
- N-(4-(5-butyl-2-methoxy-phenyl)-thiazol-2-yl)-4-(4-(tetrahydro-pyran-4-ylamino)-piperidine-1-carbonyl)-benzamide;
- N-(4-(5-Cyclohexyl-2-methoxy-phenyl)-thiazol-2-yl)-4-(2-(tetrahydro-pyran-4-ylamino)-ethoxy)-benzamide;
- N-(4-(5-Ethyl-2-methoxy-phenyl)-thiazol-2-yl)-4-(3-(tetrahydro-pyran-4-ylamino)-propyl)-benzamide;
- N-(4-(5-cyclohexyl-2-ethoxy-phenyl)-thiazol-2-yl)-4-(4-(pyrrolidin-1-yl)-piperidine-1-carbonyl)-benzamide;
- N-(4-(5-cyclohexyl-2-methoxy-phenyl)-thiazol-2-yl)-N'-pyrrolidin-2-ylmethyl-terephtalamide;
-N-(4-(5-butyl-2-methoxy-phenyl)-thiazol-2-yl)-4-(4-(3-(R)-(cyclopropanecarbonyl-amino)-pyrrolidin-1-yl)-piperidine-1-carbonyl)-benzamide;
- N-(4-(5-butyl-2-methoxy-phenyl)-thiazol-2-yl)-4-(4-(3-(R)-isobutyrylamino-pyrrolidin-1-yl)-piperidine-1-carbonyl)-benzamide;
- N-(4-(5-cyclohexyl-2-methoxy-phenyl)-thiazol-2-yl)-4-(4-(pyrrolidin-1-yl)-piperidine-1-carbonyl)-benzamide;
- N-(4-(5-butyl-2-methoxy-phenyl)-thiazol-2-yl)-4-(4-(3-(R)-hydroxy-pyrrolidin-1-yl)-piperidine-1-carbonyl)-benzamide:
- N-(4-(5-Butyl-2-methoxy-phenyl)-thiazol-2-yl)-N'-((S)-(1-ethyl-pyrrolidin-2-yl)-methyl)-terephtalamide;
- N-(4-(5-butyl-2-methoxy-phenyl)-thiazol-2-yl)-4-(4-(3-hydroxy-azetidin-1-yl)-piperidine-1-carbonyl)-benzamide;
- N-(4-(5-butyl-2-methoxy-phenyl)-thiazol-2-yl)-4-(4-(3-(S)-acetylamino-pyrrolidin-1-yl)-piperidine-1-carbonyl)-benzamide;
- N-(4-(5-Ethyl-2-ethoxy-phenyl)-thiazol-2-yl)-N'-piperidin-3-yl-terephtalamide;
-N-(4-(5-Ethyl-2-ethoxy-phenyl)-thiazol-2-yl)-4-(4-(3-(R)-hydroxy-pyrrolidin-1-yl)-piperidine-1-carbonyl)-benzamide;
- N-(4-(5-Ethyl-2-ethoxy-phenyl)-thiazol-2-yl)-N'-((S)-(1-ethyl-pyrrolidin-1yl)-methyl)-terephtalamide;
- N-(4-(5-Cyclohexyl-2-ethoxy-phenyl)-thiazol-2-yl)-N'-((S)-(1-ethyl-pyrrolidin-2-yl)-methyl)-terephtalamide;
- N-(4-(5-Cyclohexyl-2-methoxy-phenyl)-thiazol-2-yl)-N'-((S)-(1-ethyl-pyrrolidin-2-yl)-methyl)-terephtalamide;

- N-(4-(5-Cyclopentyl-2-methoxy-phenyl)-thiazol-2-yl)-4-(4-(tetrahydro-pyran-4-ylamino)-piperidine-1-carbonyl)-benzamide;
- N-(4-(5-Hexyl-2-methoy-phenyl)-thiazol-2-yl)-4-(4-(tetrahydro-pyran-4-ylamino)-piperidine-1-carbonyl)-benzamide;
- N-(4-(5-Cyclohexyl-2-methoxy-phenyl)-thiazol-2-yl)-4-(4-(3-(R)-acetylamino-pyrrolidin-1-yl)-piperidine-1-carbonyl)-benzamide;
- N-(4-(5-Butyl-2-methoxy-phenyl)-thiazol-2-yl)-4-(4-(3-(R)-formylamino-pyrrolidin-1-yl)-piperidine-1-carbonyl)-benzamide;
- N-(4-(5-Cyclohexyl-2-methoxy-phenyl)-thiazol-2-yl)-4-(4-(3-(S)-hydroxymethyl-pyrrolidin-1-yl)-piperidine-1-carbonyl)-benzamide;
- N-(4-(5-Propyl-2-methoxy-phenyl)-thiazol-2-yl)-4-(4-(3-(R)-acetylamino-pyrrolidin-1-yl)-piperidine-1-carbonyl)-benzamide;
- N-(4-(5-Cyclopentyl-2-methoxy-phenyl)-thiazol-2-yl)-4-(4-(3-(R)-acetylamino-pyrrolidin-1-yl)-piperidine-1-carbonyl)-benzamide;
- Ester ethylique de l'acide 1-(1-(4-(4-(5-Butyl-2-methoxy-phenyl)-thiazol-2-ylcarbamoyl)-benzoyl)-piperidin-4-yl)-pyrrolidin-3-(R)-yl propionique;
- N-(4-(5-Cyclohexyl-2-methoxy-phenyl)-thiazol-2-yl)-4-(4-(3-(R)-propionylamino-pyrrolidin-1-yl)-piperidine-1-carbonyl)-benzamide;
- N-(4-(5-Cyclohexyl-2-methoxy-phenyl)-thiazol-2-yl)-4-(4-(3-(R)-butyrylamino-pyrrolidin-1-yl)-piperidine-1-carbonyl)-benzamide;
- N-(4-(5-Cyclohexyl-2-ethoxy-phenyl)-thiazol-2-yl)-4-(4-(3-(R)-acetylamino-pyrrolidin-1-yl)-piperidine-1-carbonyl)-benzamide;
- N-(4-(5-Ethyl-2-ethoxy-phenyl)-thiazol-2-yl)-4-(4-(pyrrolidin-1-yl)-piperidine-1-carbonyl)-benzamide;
- N-(4-(5-Ethyl-2-ethoxy-phenyl)-thiazol-2-yl)-N'-((R)-(1-ethyl-pyrrolidin-2-yl)-methyl)-terephtalamide;
- Ester methylique de l'acide 1-(1-(4-(4-(5-Butyl-2-methoxy-phenyl)-thiazol-2-ylcarbamoyl)-piperidin-4-yl)-pyrrolidin-3-yl)-carbamique;
- N-(4-(5-Cyclopenlyl-2-methoxy-phenyl)-thiazol-2-yl)-4-(4-(3-(R)-propionylamino-pyrrolidin-1-yl)-piperidine-1-carbonyl)-benzamide;
- N-(4-(5-Butyl-2-ethoxy-phenyl)-thiazol-2-yl)-4-(4-(3-(R)-propionylamino-pyrrolidin-1-yl)-piperidine-1-carbonyl)-benzamide;
- N-(4-(5-Cyclopentyl-2-ethoxy-phenyl)-thiazol-2-yl)-4-(4-(3-(R)-propionylamino-pyrrolidin-1-yl)-piperidine-1-carbonyl)-benzamide;
- Ester ethylique de l'acide 1-(1-(4-(4-(5-Cyclopentyl-2-ethoxy-phenyl)-thiazol-2-ylcarbamoyl)-piperidin-4-yl)-pyrrolidin-3-yl)-carbamique.

**4.** Procédé de préparation d'un composé de formule (Ia) selon l'une quelconque des revendications 1 à 3 **caractérisé en ce que** l'on traite un dérivé fonctionnel d'un acide de formule :

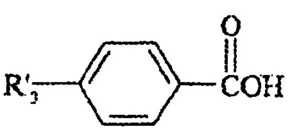 (II)

dans laquelle $R'_3$ représente $R_3$ tel que défini ci-dessus pour (Ia) ou un groupe choisi parmi :

- OPg, Pg étant un groupe protecteur tel que le *tert*-butyle, benzoyle ou arylsulfonyle choisi parmi phénylsulfonyle, tolylsulfonyle ou naphtylsulfonyle ;
- O-($C_1$-$C_3$)alk-Q, Q étant un groupe diméthoxyméthyle, diéthoxyméthyle ou formyle ;
- O-($C_2$-$C_4$)alk-OX, X représentant un atome d'hydrogène, un groupe tétrahydropyranyle ou un groupe $SO_2R'$, R' étant un groupe méthyle ou tolyle ;
- ($C_1$-$C_3$)alk-Q ;
- ($C_1$-$C_4$)alk-Hal, Hal représentant un atome d'halogéne ;
- I;
- COOH ; -COOR avec R représentant un atome d'hydrogène, un ($C_1$-$C_4$)alkyle, ou un benzyle non substitué ou substitué sur le phényle par un groupe méthoxy ;
- CONH-($C_1$-$C_3$)alk-Q;

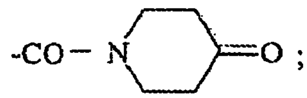

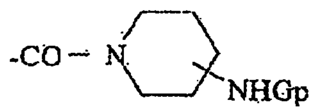

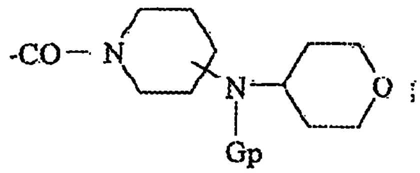

a)

a1) $-O-(C_2-C_4)alk-A'$ ;
a2) $-O-(C_1-C_4)alk-B'$ ;
a3) -O-E';

. b) $-(C_1-C_4)alk-A'$;
. c)-B';
. d)

d1) $-(C_1-C_4)alk-NR_4-(C_2-C_3)alk-A'$;
d2) $-(C_1-C_4)alk-NR_4-(C_1-C_3)alk-B'$;

. e)

e1) $-CONR_4-(C_2-C_4)alk-A'$;
e2) $-CONR_4-(C_1-C_4)alk-B'$;
e3) $-CONR_{4.}-E'$;

. f)

f1) $-CO-D-(C_1-C_2)alk-A'$;
f2) -CO-G-A';
f3)

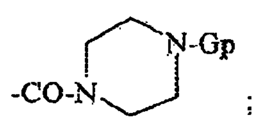

f4)

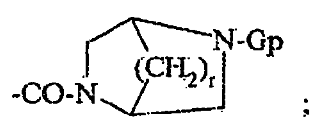

f5)

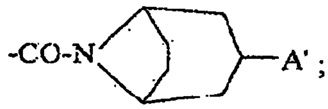

dans laquelle :

- A', B', E' représentent respectivement les groupes A, B, E tels que définis pour (Ia) dans lesquels $R_5$ est remplacé par Gp;
- Gp représente un groupe protecteur de l'azote choisi parmi : Boc, Fmoc, ($C_1$-$C_4$)alcanoyle, benzyloxycarbonyle ou benzyle ;

par un dérivé de 2-aminothiazole de formule :

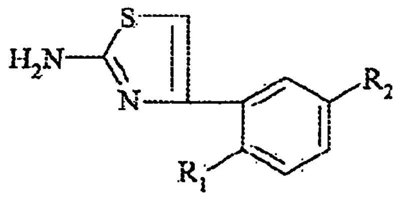

(III)

dans laquelle $R_1$ et $R_2$ sont tels que définis pour (Ia) à la revendication 1.

**5.** Composés de formule (IV):

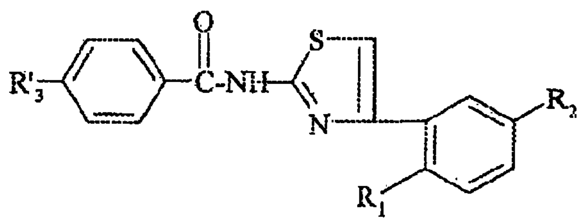

(IV)

dans laquelle :

- $R_1$ ou $R_2$ sont tels que définis à la revendication 1 ;
- $R'_3$ représente un groupe choisi parmi :

. -OPg, Pg étant un groupe protecteur tel que le *tert*-butyle, benzoyle ou arylsulfonyle choisi parmi phénylsulfonyle, tolylsulfonyle au naphtylsulfonyle ;
. -O-($C_1$-$C_3$)alk-Q, Q étant un groupe diméthoxyméthyle, diéthoxyméthyle ou formyle ;
. -O-($C_2$-$C_4$)alk-OX, X représentant un atome d'hydrogène, un groupe tetrahydropyranyle ou un groupe $SO_2$R', R' étant un groupe méthyle ou tolyle ;
. -($C_1$-$C_3$)alk-Q ;
. -($C_1$-$C_4$)alk-Hal, Hal représentant un atome d'halogène ;
. -I ;
. -COOH.; -COOR avec R représentant un atome d'hydrogène, un ($C_1$-$C_4$)alkyle, ou un benzyle non substitué ou substitué sur le phényle par un groupe methoxy ;
. -CONH-($C_1$-$C_3$)alk-Q ;

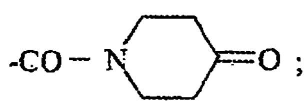

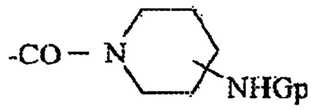

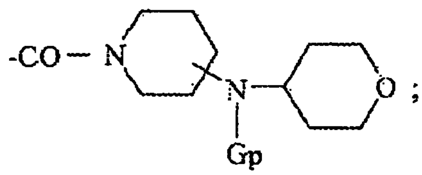

. a)

a1) -O-($C_2$-$C_4$)alk-A' ;
a2) -O-($C_1$-$C_4$)alk-B' ;
a3) -O-E' ;

. b)-($C_1$-$C_4$)alk-A' ;
. c)-B' ;
. d)

d1)-($C_1$-$C_4$)alk-$NR_4$-($C_2$-$C_3$)alk-A' ;
d2) -($C_1$-$C_4$)alk-$NR_4$-($C_1$-$C_3$)alk-B' ;

. e)

e1) -$CONR_4$-($C_2$-$C_4$)alk-A' ;
e2) -$CONR_4$-($C_1$-$C_4$)alk-B' ;
e3) -$CONR_4$-E' ;
.

f)

f1) -CO-D-($C_1$-$C_2$)alk-A' ;
f2) -CO-G-A';
f3)

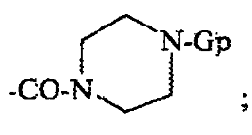

f4)

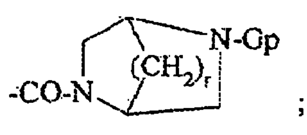

f5)

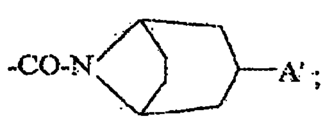

dans laquelle :

- A', B', E' représentent respectivement les groupes A, B, E tels que définis pour (Ia) dans lesquels $R_5$ est remplacé par Gp.;
- Gp représente un groupe protecteur de l'azote choisi parmi : Boc, Fmoc, $(C_1-C_4)$alcanoyle, benzyloxycarbonyle ou benzyle.

6. Médicament, **caractérisé en ce qu'**il comprend un composé de formule (Ia) selon l'une quelconque des revendications 1 à 3, ou un sel d'addition de ce composé à un acide pharmaceutiquement acceptable, ou encore un hydrate ou un solvat du composé de formule (Ia).

7. Composition pharmaceutique, caractérisé ce ce qu'elle comprend un composé de formule (Ia) selon l'une quelconque des revendications 1 à 3, ou un sel pharmaceutiquement acceptable, un hydrate ou un solvat de ce composé, ainsi qu'au moins un excipient pharmaceutiquement acceptable.

8. Composé de formule (Ia) selon l'une quelconque des revendications 1 à 3, pour utilisation dans le traitement et la prévention des maladies et des syndromes immuno-inflammatoires aigus ou chroniques, des maladies allergiques, des maladies dans lesquels les processus angiogéniques sont impliqués, des maladies virales ou bactériennes, des pathologies cardiaques, de l'obésité, des maladies dans lesquelles les processus angiogéniques sont impliqués comme les cancers ou les maladies rétiniennes.

## Claims

1. compounds corresponding to the formula (Ia):

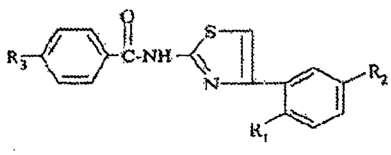

(Ia)

in which:

- $R_1$ represents a halogen atom or a $(C_1-C_4)$alkyl, hydroxyl, $(C_1-C_4)$alkoxy, $(C_3-C_8)$cycloalkyloxy, allyloxy, cyclopropylmethoxy or $(C_1-C_4)$alkylthio group; and/or
- $R_2$ represents a halogen atom or a $(C_1-C_8)$alkyl, trifluoromethyl, $(C_3-C_{10})$cycloalkyl, phenyl, $(C_1-C_8)$alkoxy, allyloxy, $(C_3-C_8)$cyclo-alkylmethoxy, $(C_3-C_8)$cycloalkyloxy or $(C_3-C_8)$cycloalkylmethyl group; and/or
- $R_3$ represents a group chosen from:

g)

a1) -O-$(C_2-C_4)$alk-A;
a2) -O-$(C_1-C_4)$alk-B;
a3) -O-E;

h) -$(C_1-C_4)$alk-A;
i) -B;
j)

d1) $(C_1-C_4)$alk-$NR_4$-$(C_2-C_3)$alk-A;
d2) $(C_1-C_4)$alk-$NR_4$-$(C_1-C_3)$alk-B;

k)

e1) -$CONR_4$-$(C_2-C_4)$alk-A;
e2) -$CONR_4$-$(C_1-C_4)$alk-B ;
$e_3$) -$CONR_4$-E;

l)

f1) -CO-D-$(C_1-C_2)$alk-A;

f2) -CO-G-A;

f3)

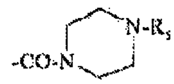

;

f4)

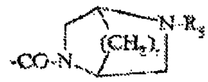

;

f5)

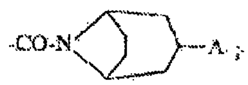

f6)

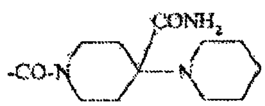

;

f7)

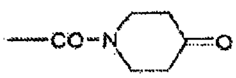

;

- $R_4$ represents a hydrogen atom or a $(C_1-C_4)$alkyl group;
- A represents an $NR_5R_6$ group;
- B represents a

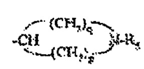

group;
- D represents a

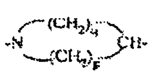

group;
- E represents a

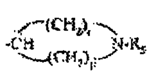

group;
- G represents a

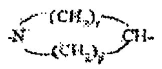

group;

- $R_5$ and $R_6$ each represent, independently of one another, a hydrogen atom or a $(C_1-C_6)$alkyl, allyl, $(C_2-C_4)$alk-O-$(C_1-C_4)$alkyl, $(C_2-C_4)$alk-OH, $(C_1-C_3)$alk-CON$(R_4)_2$, $(C_2-C_3)$alk-NHCO-$(C_1-C_4)$alkyl, $(C_3-C_7)$ cycloalkyl, $(C_3-C_7)$ cycloalkylmethyl, -CO-$(C_1-C_4)$alkyl, benzyl, pyrrolidinyl, optionally substituted by a -CO$(C_1-C_4)$alkyl group, tetrahydropyranyl, tetrahydropyranylmethyl, dimethyltetrahydropyranyl, tetrahydrofuryl or tetrahydrofurylmethyl group;
- or $R_5$ and $R_6$, together with the nitrogen atom to which they are bonded, constitute a heterocyclic radical chosen from: aziridinyl, azetidinyl, pyrrolidinyl, piperidinyl, perhydroazepinyl, morpholinyl, piperazinyl, tropanyl, quinuclidinyl, 2-azabicyclo[2.2.1]heptanyl or 2-azabicyclo[2.2.2]octanyl, the said heterocyclic radicals being unsubstituted or substituted by a phenyl, halophenyl, trifluoromethylphenyl, trifluoromethyl, hydroxyl, methoxy, hydroxymethyl, methoxymethyl, formamido, trifluoroacetylamino, -$NR_4R_7$, tetrahydropyran-4-ylamino, -CON$(R_4)_2$, -$CONR_4R'_4$, -$CH_2CON(R_4)_2$, $(C_1-C_4)$alkyl-$CONR_4$-, $(C_3-C_8)$cycloalkyl-$CONR_4$-, $(C_1-C_4)$alkyl-$OCONR_4$-, ($(C_1-C_4)$alkyl-OCO$)_2$N- or $(C_1-C_4)$alkyl-COO- group or substituted by one or more methyl groups;
- $R'_4$ represents a $(CH_2)_a$ group bonded to the carbon atom carrying -$CONR_4R'_4$;
- $R_7$ represents a hydrogen atom, a $(C_1-C_4)$alkyl group or an -$SO_2CH_3$ group or $R_4$ and $R_7$, together with the nitrogen atom to which they are bonded, constitute a pyrrolidinyl or piperidinyl radical;
- p represents 1, 2, 3, 4 or 5;
- q represents 0, 1 or 2;
- r represents 1 or 2;
- s represents 2 or 3;
- p + q being less than or equal to 5;
- p + r being less than or equal to 5;
- alk represents an alkylene;

in the form of the base or of an addition salt with an acid, and in the hydrate or solvate form.

**2.** Compounds according to Claim 1 of formula (Ia), in which:

- $R_1$ represents a $(C_1-C_4)$alkoxy, cyclopropylmethoxy or $(C_1-C_4)$alkylthio group; and/or
- $R_2$ represents a halogen atom or a $(C_1-C_8)$alKyl, trifluoromethyl, $(C_3-C_{10})$cycloalkyl or $(C_1-C_8)$alkoxy group; and/or
- $R_3$ represents an f2 or e2 group;

in the form of the base or of an addition salt with an acid, and in the hydrate or solvate form.

**3.** Compounds according to Claim 1 of formula (Ia):

- 4-((4-[3-(R)-(acetylamino)pyrrolidin-1-yl]piperidin-1-yl)carbonyl)-N-[4-(5-butyl-2-methoxyphenyl)-1,3-thiazol-2-yl)benzamide;
- ethyl ester of 1-(1-(4-(4-(5-butyl-2-methoxyphenyl)thiazol-2-ylcarbamoyl)benzoyl)piperidin-4-yl)pyrrolidin-3-ylcarbamic acid;
- N-(4-(5-butyl-2-methoxyphenyl)thiazol-2-yl)-4-(4-(3-(R)-(acetylamino)pyrrolidin-1-yl)piperidyl-1-carbonyl)benzamide;
- N-(4-(5-butyl-2-methoxyphenyl)thiazol-2-yl)-4-(4-(tetrahydropyran-4-ylamino)piperidyl-1-carbonyl)benzamide;
- N-(4-(5-cyclohexyl-2-methoxyphenyl)thiazol-2-yl)-4-(2-(tetrahydropyran-4-ylamino)ethoxy)benzamide;
- N-(4-(5-ethyl-2-methoxyphenyl)thiazol-2-yl)-4-(3-(tetrahydropyran-4-ylamino)propyl)benzamide;
- N-(4-(5-cyclohexyl-2-ethoxyphenyl)thiazol-2-yl)-4-(4-(pyrrolidin-1-yl)piperidyl-1-carbonyl)benzamide;
- N-(4-(5-cyclohexyl-2-methoxyphenyl)thiazol-2-yl)-N'-(pyrrolidin-2-ylmethyl)terephthalamide;
- N-(4-(5-butyl-2-methoxyphenyl)thiazol-2-yl)-4-(4-(3-(R)-(cyclopropylcarbonylamino)pyrrolidin-1-yl)piperidyl-1-carbonyl)benzamide;
- N-(4-(5-butyl-2-methoxyphenyl)thiazol-2-yl)-4-(4-(3-(R)-(isobutyrylamino)pyrrolidin-1-yl)piperidyl-1-carbonyl)benzamide;
- N-(4-(5-cyclohexyl-2-methoxyphenyl)thiazol-2-yl)-4-(4-(pyrrolidin-1-yl)piperidyl-1-carbonyl)benzamide;

- N-(4-(5-butyl-2-methoxyphenyl)thiazol-2-yl)-4-(4-(3-(R)-hydroxypyrrolidin-1-yl)piperidyl-1-carbonyl)benzamide;
- N-(4-(5-butyl-2-methoxyphenyl)thiazol-2-yl)-N'-((S)-(1-ethylpyrrolidin-3-yl)methyl)terephthalamide;
- N-(4-(5-butyl-2-methoxyphenyl)thiazol-2-yl)-4-(4-(3-hydroxyazetidin-1-yl)piperidyl-1-carbonyl)benzamide;
- N-(4-(5-butyl-2-methoxyphenyl)thiazol-2-yl)-4-(4-(3-(S)-(acetylamino)pyrrolidin-1-yl)piperidyl-1-carbonyl) benzamide;
- N-(4-(5-ethyl-2-ethoxyphenyl)thiazol-2-yl)-N'-(piperidin-3-yl)terephthalamide;
- N-(4-(5-ethyl-2-ethoxyphenyl)thiazol-2-yl)-4-(4-(3-(R)-hydroxypyrrolidin-1-yl)piperidyl-1-carbonyl)benzamide;
- N-(4-(5-ethyl-2-ethoxyphenyl)thiazol-2-yl)-N'-((S)-(1-ethylpyrrolidin-2-yl)methyl)terephthalamide;
- N-(4-(5-cyclohexyl-2-ethoxyphenyl)thiazol-2-yl)-N'-(S)-(1-ethylpyrrolidin-2-yl)methyl)terephthalamide;
- N-(4-(5-cyclohexyl-2-methoxyphenyl)thiazol-2-yl)-N'-((S)-(1-ethylpyrrolidin-2-yl)methyl)terephthalamide;
- N-(4-(5-cyclopentyl-2-methoxyphenyl)thiazol-2-yl)-4-(4-(tetrahydropyran-4-ylamino)piperidyl-1-carbonyl) benzamide;
- N-(4-(5-hexyl-2-methoxyphenyl)thiazol-2-yl)-4-(4-(tetrahydropyran-4-ylamino)piperidyl-1-carbonyl)benzamide;
- N-(4-(5-cyclohexyl-2-methoxyphenyl)thiazol-2-yl)-4-(4-(3-(R)-(acetylamino)pyrrolidin-1-yl)piperidyl-1-carbonyl)benzamide;
- N-(4-(5-butyl-2-methoxyphenyl)thiazol-2-yl)-4-(4-(3-(R)-(formylamino)pyrrolidin-1-yl)piperidyl-1-carbonyl) benzamide;
- N-(4-(5-cyclohexyl-2-methoxyphenyl)thiazol-2-yl)-4-(4-(3-(S)-(hydroxymethyl)pyrrolidin-1-yl)piperidyl-1-carbonyl)benzamide;
- N-(4-(5-propyl-2-methoxyphenyl)thiazol-2-yl)-4-(4-(3-ER)-(acetylamino)pyrrolidin-1-yl)piperidyl-1-carbonyl) benzamide;
- N-(4-(5-cyclopentyl-2-methoxyphenyl)thiazol-2-yl)-4-(4-(3-(R)-(acetylamino)pyrrolidin-1-yl)piperidyl-1-carbonyl)benzamide;
- ethyl ester of 1-(1-(4-(4-(5-butyl-2-methoxyphenyl)thiazol-2-ylcarbamoyl)benzoyl)piperidin-4-yl)pyrrolidin-3-(R)-ylpropionic acid;
- N-(4-(5-cyclohexyl-2-methoxyphenyl)thiazol-2-yl)-4-(4-(3-(R)-(proppionylamino)pyrrolidin-1-yl)piperidyl-1-carbonyl)benzamide ;
- N-(4-(5-cyclohexyl-2-methoxyphenyl)thiazol-2-yl)-4-(4-(3-(R)-(butyrylamino)pyrrolidin-1-yl)piperidyl-1-carbonyl)benzamide;
- N-(4-(5-cyclohexyl-2-ethoxyphenyl)thiazol-2-yl)-4-(4-(3-(R)-(acetylamino)pyrrolidin-1-yl)pippridyl-1-carbonyl) benzamide;
- N-(4-(5-ethyl-2-ethoxyphenyl)thiazol-2-yl)-4-(4-(pyrrolidin-1-yl)piperidyl-1-carbonyl)benzamide;
- N-(4-(5-ethyl-2-ethoxyphenyl)thiazol-2-yl)-N'-((R)-(1-ethylpyrrolidin-2-yl)methyl)terephthalamide;
- methyl ester of 1-(1-(4-(4-(5-butyl-2-methoxyphenyl)thiazol-2-ylcarbamoyl)benzoyl)piperidin-4-yl)pyrrolidin-3-ylcarbamic acid;
- N-(4-(5-cyclopentyl-2-methoxyphenyl)thiazol-2-yl)-4-(4-(3-(R)-(propionylamino)pyrrolidin-1-yl)piperidyl-1-carbonyl)benzamide;
- N-(4-(5-butyl-2-ethoxyphenyl)thiazol-2-yl)-4-(4-(3-(R)-(propionylamino)pyrrolidin-1-yl)piperidyl-1-carbonyl) benzamide;
- N-(4-(5-cyclopentyl-2-ethoxyphenyl)thiazol-2-yl)-4-(4-(3-(R)-(propionylamino)pyrrolidin-1-yl)piperidyl-1-carbonyl)benzamide;
- ethyl ester of 1-(2-(4-(4-(5-cyclopentyl-2-ethoxyphenyl)thiazol-2-ylcarbamoyl)benzoyl)piperidin-4-yl)pyrrolidin-3-ylcarbamic acid.

**4.** Process for the preparation of a compound of formula (Ia) according to any one of Claims 1 to 3, **characterized in that** a functional derivative of an acid of formula:

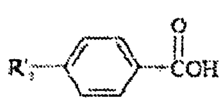 (II)

In which $R'_3$ represents $R_3$ as defined above for (Ia) or a group chosen from:

• -OPg, Pg being a protective group, such as tert-butyl, benzoyl or arylsulphonyl chosen from phenylsulphonyl, tolylsulphonyl or naphthylsulphonyl;

• -O-($C_1$-$C_3$)alk-Q, Q being a dimethoxymethyl, diethoxymethyl or formyl group;
• -O-($C_2$-$C_4$)alk-OX, X representing a hydrogen atom, a tetrahydropyranyl group or an $SO_2$R' group, R' being a methyl or tolyl group;
• -($C_1$-$C_3$)alk-Q;
• -($C_1$-$C_4$)alk-Hal, Hal representing a halogen atom;
• -I;
• -COOH or -COOR, with R representing a hydrogen atom, a ($C_1$-$C_4$)alkyl or a benzyl which is unsubstituted or substituted on the phenyl by a methoxy group;
• -CONH-($C_1$-$C_3$)alk-Q;

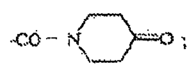

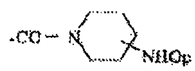

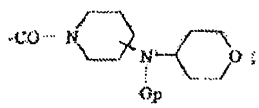

a)

a$_1$) O-($C_2$-$C_4$)alk-A':
a$_2$) -O-($C_1$-$C_4$)alk-R':
a3) -O-E',

b) -($C_1$-$C_4$)alk-A';
c) -B';
d)

d1) -($C_1$-$C_4$)alk-$NR_4$-($C_2$-$C_3$)alk-A';
d2) -($C_1$-$C_4$)alk-$NR_4$-($C_1$-$C_3$)alk-B';

e)

c1) -$CONR_4$-($C_3$-$C_4$)alk-A';
e2) -$CONR_4$-($C_1$-$C_4$)alk-B';
e3) -$CONR_4$-E';

. f)

f1) -CO-D-($C_1$-$C_2$)alk-A';
f2) -CO-G-A';
f3)

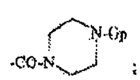

f4)

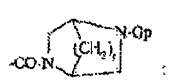

f5)

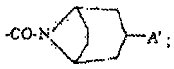

in which:

- A', B' and E' respectively represent the A, B and E groups as defined for (Ia) in which $R_5$ is replaced by Gp;
- Gp represents a projective group for the nitrogen chosen from: Boc, Fmoc, $(C_1\text{-}C_4)$alkanoyl, benzyloxycarbonyl or benzyl;

is treated with a 2-aminothiazole derivative of formula:

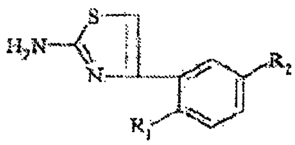

(III)

in which $R_1$ and $R_2$ are as defined for (Ia) in claim 1.

**5.** Compounds of formula (IV):

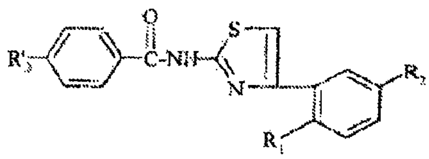

(IV)

in which:

- $R_1$ or $R_2$ are as defined in Claim 1;
- $R'_3$ represents a group chosen from:

  • -OPg. Pg being a protective group, such as tert-butyl, benzoyl or arylsulphonyl chosen from phenylsulphonyl, tolylsulphonyl or naphthylsulphonyl;
  • -O-$(C_1\text{-}C_3)$alk-Q, Q being a dimethoxymethyl, diethoxymethyl or formyl group;
  • -O-$(C_2\text{-}C_4)$alk-OX, X representing a hydrogen atom, a tetrahydropyranyl group or an $SO_2R'$ group, R' being a methyl or tolyl group;
  • - $(C_1\text{-}C_3)$alk-Q;
  • -$(C_1\text{-}C_4)$alk-Hal, Hal representing a halogen atom;
  • -I;
  • -COOH or -COOR, with R representing a hydrogen atom, a $(C_1\text{-}C_4)$alkyl or a benzyl which is unsubstituted or substituted on the phenyl by a methoxy group;
  • -CONH-$(C_1\text{-}C_3)$alk-Q;

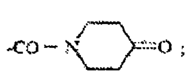

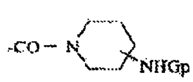

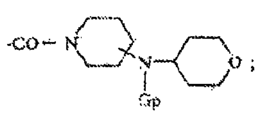

. a) a1) $-O-(C_2-C_4)alk-A'$ ;
a2) $-O-(C_1-C_4)alk-B'$ ;
a3) $-O-E'$ ;
. b) $-(C_1-C_4)alk-A'$ ;
. c) $-B'$ ;
. d) d1) $-(C_1-C_4)alk-NR_4-(C_2-C_3)alk-A'$ ;
d2) $-(C_1-C_4)alk-NR_4-(C_1-C_3)alk-B'$ ;
. e) e1) $-CONR_4-(C_2-C_4)alk-A'$ ;
e2) $-CONR_4-(C_1-C_4)alk-B'$ ;
e3) $-CONR_4-E'$ ;
. f) f1) $-CO-U-(C_1-C_3)alk-A'$ ;
f2) $-CO-G-A'$ ;

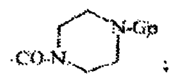

f4)

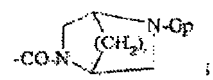

f5)

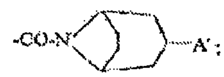

in which:

- A', B' and E' respectively represent the A, B and E groups as defined for (Ia) in which $R_5$ is replaced by Gp;
- Gp represents a protective group for the nitrogen chosen from: Boc, Fmoc, $(C_1-C_4)$alkanoyl, benzyloxycarbonyl or benzyl.

**6.** Medicine, **characterized in that** it comprises a compound of formula (Ia) according to any one of Claims 1 to 3, or an addition salt of this compound with a pharmaceutically acceptable acid, or a hydrate or a solvate of the compound of formula (Ia).

**7.** Pharmaceutical composition, **characterized in that** it comprises a compound of formula (Ia) according to any one of Claims 1 to 3, or a pharmaceutically acceptable salt, a hydrate or a solvate of this compound, and at least one pharmaceutically acceptable excipient.

**8.** Compound of formula (Ia) according to any one of Claims 1 to 3 for use in the treatment and prevention of acute or chronic immunoinflammatory diseases and syndromes, allergic diseases, viral or bacterial diseases, cardiac pathologies, obesity or diseases in which angiogenic processes are involved, such as cancers or retinal diseases.

## Patentansprüche

**1.** Verbindungen der Formel (Ia):

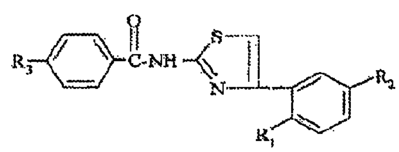

(Ia)

worin:

- $R_1$ für ein Halogenatom oder eine $(C_1-C_4)$-Alkyl-, Hydroxyl-, $(C_1 - C_4)$-Alkoxy-, $(C_3-C_8)$-Cycloalkyloxy, Allyloxy-, Cyclopropylmethoxy- oder $(C_1-C_4)$ -Alkylthiogruppe steht und/oder
- $R_2$ für ein Halogenatom oder eine $(C_1-C_8)$-Alkyl-, Trifluormethyl-, $(C_3-C_{10})$ -Cycloalkyl-, Phenyl-, $(C_1-C_8)$-Alkoxy-, Allyloxy-, $(C_3-C_8)$-Cycloalkylmethoxy-, $(C_3-C_8)$-cycloalkyloxy- oder $(C_3-C_8)$-Cycloalkylmethylgruppe steht und/oder
- $R_3$ für eine unter:

g)

a1) -O-$(C_2-C_4)$-alk-A;
a2) -O-$(C_1-C_4)$ -alk-B;
a3) -O-E;

h) -$(C_1-C_4)$-alk-A;
i) -B;
j)

d1) -$(C_1-C_4)$-alk-$NR_4$-$(C_2-C_3)$-alk-A;
d2) -$(C_1-C_4)$-alk-$NR_4$-$(C_1-C_3)$-alk-B;

k)

e1) -$CONR_4$-$(C_2-C_4)$-alk-A;
e2) -$CONR_4$-$(C_1-C_4)$-alk-B;
e3) -$CONR_4$-E;

1)

f1) -CO-D-$(C_1-C_2)$-alk-A;
f2) -CO-G-A;
f3)

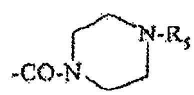
;

f4)

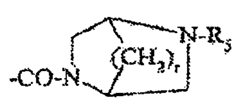
;

f5)

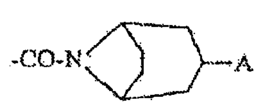
;

f6)

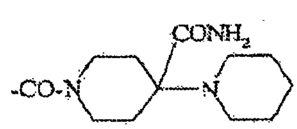

und
f7)

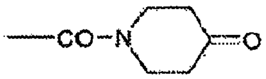

ausgewählte Gruppe steht;

- $R_4$ für ein Wasserstoffatom oder eine ($C_1$-$C_4$)-Alkylgruppe steht;
- A für eine Gruppe $NR_5R_6$ steht;
- B für eine Gruppe

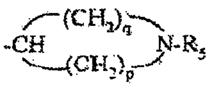

steht;
- D für eine Gruppe

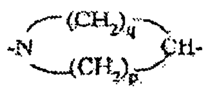

steht;
- E für eine Gruppe

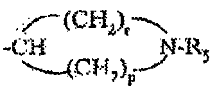

steht;
- G für eine Gruppe

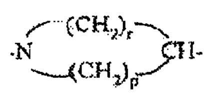

steht;
- $R_5$ und $R_6$ jeweils unabhängig voneinander für ein Wasserstoffatom oder eine ($C_1$-$C_6$)-Alkyl-, Allyl-, ($C_2$-$C_4$)-alk-O-($C_1$-$C_4$)-Alkyl-, ($C_2$-$C_4$)-alk-OH-, ($C_1$-$C_3$)-alk-CON($R_4$)$_2$-, ($C_2$-$C_3$)-alk-NHCO-($C_1$-$C_4$)-Alkyl-, ($C_3$-$C_7$)-Cycloalkyl-, ($C_3$-$C_7$)-Cycloalkylmethyl-,- CO-($C_1$-$C_4$)-Alkyl-, Benzyl-, gegebenenfalls durch eine -CO($C_1$-$C_4$)-Alkylgruppe substituierte Pyrrolidinyl-, Tetrahydropyranyl-, Tetrahydropyranylmethyl-, Dimethyltetrahydropyranyl-, Tetrahydrofuryl- oder Tetrahydrofurylmethylgruppe stehen;
- oder $R_5$ und $R_6$ zusammen mit dem Stäckstoffatom, an das sie gebunden sind, einen heterocyclicschen Rest bilden, der unter Aziridinyl, Azetidinyl, Pyrrolidinyl, Piperidinyl, Perhydroazepinyl, Morpholinyl, Piperazinyl, Tropanyl, Chinuclidinyl, 2-Azabicyclo[2.2.1]-heptanyl und 2-Azabicyclo[2.2.2]octanyl ausgewählt ist, wobei die heterocyclischen Reste gegebenenfalls durch eine Phenyl-, Halogenphenyl-, Trifluormethylphenyl-, Trifluormethyl-, Hydroxy-, Methoxy-, Hydroxymethyl-, Methoxymethyl-, Formamido- oder Trifluoracetylaminogruppe, eine -$NR_4R_7$-, Tetrahydropyran-4-ylamino-, -CON($R_4$)$_2$-, -$CONR_4R'_4$-, -$CH_2$CON($R_4$)$_2$-, ($C_1$-$C_4$)-Alkyl-$CONR_4$-, ($C_3$-$C_8$)-Cycloalkyl-$CONR_4$-, ($C_1$-$C_4$)-Alkyl-$OCONR_4$-, (($C_1$-$C_4$)-Alkyl-OCO)$_2$N- oder ($C_1$-$C_4$)-Alkyl-COO-Gruppe oder eine oder mehrere Methylgruppen substituiert sind;
- $R'_4$ für eine ($CH_2$)$_a$-Gruppe steht, die an das -$CONR_4R'_4$ tragende Kohlenstoffatom gebunden ist;
- $R_7$ für ein Wasserstoffatom, ein ($C_1$-$C_4$)-Alkyl oder eine -$SO_2CH_3$-Gruppe steht oder $R_4$ und $R_7$ zusammen mit dem Stickstoffatom, an das sie gebunden sind, einen Pyrrolidinyl- oder Piperidinylrest bilden;
- p für 1, 2, 3, 4 oder 5 steht;

- q für 0, 1 oder 2 steht;
- r für 1 oder 2 steht;
- s für 2 oder 3 steht;
- wobei p + q kleiner gleich 5 ist;
- wobei p + r kleiner gleich 5 ist;
- alk für einen Alkylenrest steht;

in Basen- oder Säureadditionssalz form sowie in Hydrat- oder Solvatform.

**2.** Verbindungen nach Anspruch 1 der Formel (Ia), worin:

- $R_1$ für eine ($C_1$-$C_4$)-Alkoxy-, Cyclopropylmethoxy-oder ($C_1$-$C_4$)-Alkylthiogruppe steht und/oder
- $R_2$ für ein Halogenatom oder eine ($C_1$-$C_8$)-Alkyl-, Trifluormethyl-, ($C_3$-$C_{10}$)-Cycloalkyl- oder ($C_1$-$C_8$)-Alkoxygruppe steht und/oder
- $R_3$ für eine Gruppe f2 oder e2 steht;

in Basen- oder Säureadditionssalzform sowie in Hydrat- oder Solvatform.

**3.** Verbindungen nach Anspruch 1 der Formel (Ia) :

- 4-((4-[3-(R)-(Acetylamino)pyrrolidin-1-yl]-piperidin-1-yl)carbonyl)-N-[4-(5-butyl-2-methoxyphenyl)-1,3-thiazol-2-yl]benzamid;
- (1-(1-(4-(4-(5-Butyl-2-methoxyphenyl)thiazol-2-ylcarbamoyl)pipiperidin-4-yl)pyrrolidin-3-yl)carbamidsäureethylester;
- N-(4-(5-Butyl-2-methoxyphenyl)thiazol-2-yl)-4-(4-(3-(R)-acetylaminopyrrolidin-1-yl)piperidin-1-carbonyl)benzamid;
- N-(4-(5-Butyl-2-methoxyphenyl)thiazol-2-yl)-4-(4-(tetrahydropyran-4-ylamino)piperidin-1-carbonyl)-benzamid;
- N-(4-(5-Cyclohexyl-2-methoxyphenyl)thiazol-2-yl)-4-(2-(tetrahydropyran-4-ylamino)ethoxy)benzamid;
- N-(4-(5-Ethyl-2-methoxyphenyl)thiazol-2-yl)-4-(3-(tetrahydropyran-4-ylamino)propyl)benzamid;
- N-(4-(5-Cyclohexyl-2-ethoxyphenyl)thiazol-2-yl)-4-(4-(pyrrolidin-1-yl)piperidin-1-carbonyl)benzamid;
- N-(4-(5-Cyclohexyl-2-methoxyphenyl)thiazol-2-yl)-N'-pyrrolidin-2-ylmethylterephthalamid;
- N-(4-(5-Butyl-2-methoxyphenyl)thiazol-2-yl)-4-(4-(3-(R)-(cyclopropancarbonylamino)pyrrolidin-1-yl)-piperidin-1-carbonyl)benzamid;
- N-(4-(5-Butyl-2-methoxyphenyl)thiazol-2-yl)-4-(4-(3-(R)-isobutyrylaminopyrrolidin-1-yl)piperidin-1-carbonyl) benzamid;
- N-(4-(5-Cyclohexyl-2-methoxyphenyl)thiazol-2-yl)-4-(4-(pyrrolidin-1-yl)piperidin-1-carbonyl)benzamid;
- N-(4-(5-Butyl-2-methoxyphenyl)thiazol-2-yl)-4-(4-(3-(R)-hydroxypyrrolidin-1-yl)piperidin-1-carbonyl)benzamid;
- N-(4-(5-Butyl-2-methoxyphenyl)thiazol-2-yl)-N-((S)-(1-ethylpyrrolidin-2-yl)methyl)terephthal-amid;
- N-(4-(5-Butyl-2-methoxyphenyl)thiazol-2-yl)-4-(4-(3-hydroxyazetidin-1-yl)piperidin-1-carbonyl)benzamid;
- N-(4-(5-Butyl-2-methoxyphenyl)thiazol-2-yl)-4-(4-(3-(S)-acetylaminopyrrolidin-1-yl-)piperidin-1-carbonyl)benzamid;
- N-(4-(5-Ethyl-2-ethoxyphenyl)thiazol-2-yl)-N'-piperidin-3-ylterephthalamid; N-(4-(5-Ethyl-2-ethoxyphenyl) thiazol-2-yl)-4-(4-(3-(R)-hydroxypyrrolidin-1-yl)piperidin-1-carbonyl)benzamid; N-(4-(5-Ethyl-2-ethoxyphenyl) thiazol-2-yl)-N'-((S)-(1-ethylpyrrolidin-2-yl)methyl)terephthal-amid;
- N-(4-(5-Cyclohexyl-2-ethoxyphenyl)thiazol-2-yl)-N'-((S)-(1-ethylpyrrolidin-2-yl)methyl)terephthal-amid;
- N-(4-(5-cyclohexyl-2-methoxyphenyl)thiazol-2-yl)-N'-((S)-(1-ethylpyrrolidin-2-yl)methyl)terephthal-amid;
- N-(4-(5-Cyclopentyl-2-methoxyphenyl)thiazol-2-yl)-4-(4-(tetrahydropyran-4-ylamino)piperidin-1-carbonyl) benzamid;
- N-(4-(5-Hexyl-2-methoxyphenyl)thiazol-2-yl)-4-(4-(tetrahydropyran-4-ylamino)piperidin-1-carbonyl)-benzamid;
- N-(4-(5-Cyclohexyl-2-methoxyphenyl)thiazol-2-yl)-4-(4-(3-(R)-acetylaminopyrrolidin-1-yl)piperidin-1-carbonyl)benzamid;
- N-(4-(5-Butyl-2-methoxyphenyl)thiazol-2-yl) -4- (4-(3-(R)-formylaminopyrrolidin-1-yl)piperidin-1-carbonyl) benzamid;
- N-(4-(5-Cyclohexyl-2-methoxyphenyl)thiazol-2-yl)-4-(4-(3-(S)-hydroxymethylpyrrolidin-1-yl)-piperidin-1-carbonyl)benzamid;

- N-(4-(5-Propyl-2-methoxyphenyl)thiazol-2-yl)-4-(4-(3-(R)-acetylaminopyrrolidin-1-yl)piperidin-1-carbonyl) benzamid;
- N-(4-(5-Cyclopentyl-2-methoxyphenyl)thiazol-2-yl)-4-(4-(3-R)-acetylaminopyrrolidin-1-yl)piperidin-1-carbonyl)benzamid;
- 1-(1-(4-(4-(5-Butyl-2-methoxyphenyl)thiazol-2-ylcarbamoyl)benzoyl)piperidin-4-yl)pyrrolidin-3-(R)-ylpropionsäureethylester;
- N-(4-(5-Cyclohexyl-2-methoxyphenyl)thiazol-2-yl)-4-(4-(3-(R)-propionylaminopyrrolidin-1-yl)-piperidin-1-carbonyl)benzamid;
- N-(4-(5-Cyclohexyl-2-methoxyphenyl) thiazol-2-yl)-4-(4-(3-(R)-butyrylaminopyrrolidin-1-yl)piperidin-1-carbonyl) benzamid; N-(4-(5-Cyclohexyl-2-ethoxyphenyl)thiazol-2-yl)-4-(4-(3-(R)-acetylaminopyrrolidin-1-yl)piperidin-1-carbonyl)benzamid;
- N-(4-(5-Ethyl-2-ethoxyphenyl)thiazol-2-yl)-4-(4-(pyrrolidin-1-yl)piperidin-1-carbonyl)benzamid;
- N-(4-(5-Ethyl-2-ethoxyphenyl)thiazol-2-yl)-N'-((R)-(1-ethylpyrrolidin-2-yl)methyl)terephthal-amid;
- 1-(1-(4-(4-(5-Butyl-2-methoxyphenyl)thiazol-2-yl-carbarmoyl)piperidin-4-yl) pyrrolidin-3-yl)carbamidsäuremethylester ;
- N-(4-(5-Cyclopentyl-2-methoxyphenyl)thiazol-2-yl)-4-(4-(3-(R) -propionylaminopyrrolidin-1-yl)-piperidin-1-carbonyl)benzamid;
- N-(4-(5-Butyl-2-ethoxyphenyl)thiazol-2-yl)-4-(4-(3-(R)-propionylaminopyrrolidin-1-yl)piperidin-1-carbonyl) benzamid;
- N-(4-(5-Cyclopentyl-2-ethoxyphenyl)thiazol-2-yl)-4-(4-(3-(R)-propionylaminopyrrolidin-1-yl)-piperidin-1-carbonyl)benzamid;

-1-(1-(4-(4-(5-cyclopentyl-2-ethoxyphenyl)thiazol-2-ylcarbamoyl)piperidin-4-yl)pyrrolidlin-3-yl)-carbamidsäureethylester.

4. Verfahren zur Herstellung einer Verbindung der Formel (Ia) nach einem der Ansprüche 1 bis 3, **dadurch gekennzeichnet, dass** man ein funktionelles Derivat einer Säure der Formel:

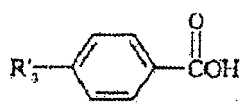 (II)

worin $R'_3$ für $R_3$ gemäß der oben für (Ia) angegebenen Definition oder eine unter

- OPg, wobei Pg für eine Schutzgruppe wie tert.-Butyl, Benzoyl oder Arylsulfonyl, das unter Phenylsulfonyl, Tolylsulfonyl oder Naphthylsulfonyl ausgewählt ist, steht;
- O-($C_1$-$C_3$)-alk-Q, wobei Q für eine Dimethoxymethyl-, Diethoxymethyl- oder Formylgruppe steht;
- O-($C_2$-$C_4$)-alk-OX, wobei X für ein Wasserstoffatom, eine Tetrahydropyranylgruppe oder eine Gruppe $SO_2R'$ steht, wobei $R^4$ für eine Methyl- oder Tolylgruppe steht;
- ($C_1$-$C_3$)-alk-Q;
- ($C_1$-$C_4$)-alk-Hal, wobei Hal für ein Halogenatom steht;
- COOH; -COOR, wobei R für ein Wasserstoffatom, ein ($C_1$-$C_4$)-Alkyl oder ein Benzyl, das gegebenenfalls an dem Phenyl durch eine Methoxygruppe substituiert ist, steht;
- CONH-($C_1$-$C_3$)-alk-Q;

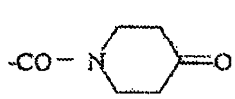 ;

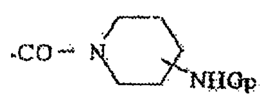

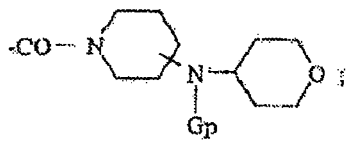

a)

a1) -O-($C_2$-$C_4$)-alk-A';
a2) -O-($C_1$-$C_4$)-alk-B';
a3) -O-E';

b) - ($C_1$-$C_4$) -alk-A';
c) -B';
d)

d1) - ($C_1$-$C_4$) -alk-$NR_4$- ($C_2$-$C_3$) -alk-A';
d2) - ($C_1$-$C_4$) -alk-$NR_4$- ($C_1$-$C_3$) -alk-B';

e)

e1) -$CONR_4$-($C_2$-$C_4$)-alk-A';
e2) -$CONR_4$-($C_1$-$C_4$)-alk-B';
e3) -$CONR_4$-E';

f)

f1) -CO-D-($C_1$-$C_2$)-alk-A';
f2) -CO-G-A';
f3)

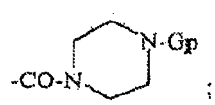

f4)

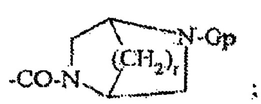

und
f5)

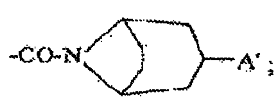

ausgewählte Gruppe steht; worin:
- A', B' und E' für die Gruppen A, B bzw. E gemäß der oben für (Ia) angegebenen Definition stehen, worin $R_5$ durch Gp ersetzt ist;
- Gp für eine Stickstoff-Schutzgruppe steht, die unter Boc, Fmoc, ($C_1$-$C_4$)-Alkanoyl, Benzyloxycarbonyl oder Benzyl ausgewählt ist;

mit einem 2-Aminothiazolderivat der formel:

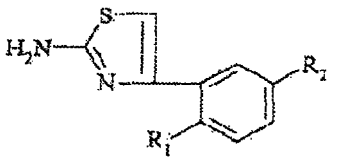 (III)

worin $R_1$ und $R_2$ wie oben für (Ia) in Anspruch 1 definiert sind; behandelt.

**5.** verbindungen der Formel (IV):

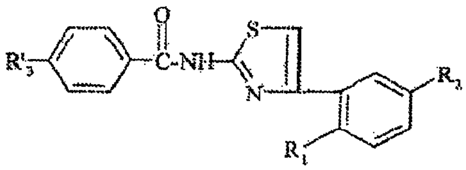 (IV)

worin:

- $R_1$ und $R_2$ wie in Anspruch 1 definiert sind;
-R'$_3$ für eine unter
-OPg, wobei Pg für eine Schutzgruppe wie tert.-Butyl, Benzoyl oder Arylsulfonyl, das unter Phenylsulfonyl, Tolylsulfonyl oder Naphthylsulfonyl ausgewählt ist, steht;
-O-($C_1$-$C_3$)-alk-Q, wobei Q für eine Dimethoxymethyl-, Diethoxymethyl- oder Formylgruppe steht;
-O-($C_2$-$C_4$)-alk-ox, wobei X für ein wasserstoffatom, eine Tetrahydropyranylgruppe oder eine Gruppe $SO_2R'$ steht, wobei R' für eine Methyl- oder Tolylgruppe steht;
-($C_1$-$C_3$)-alk-Q;
($C_1$-$C_4$)-alk-Hal, wobei Hal für ein Halogenatom steht;
-I;
-COOH; -COOR, wobei R für ein Wasserstoffatom, ein ($C_1$-$C_4$)-Alkyl oder ein Benzyl, das gegebenenfalls an dem Phenyl durch eine Methoxygruppe substituiert ist, steht;
-CONH-($C_1$-$C_3$) -alk-Q;

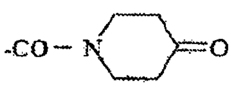 ;

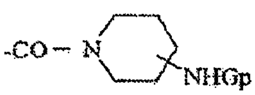

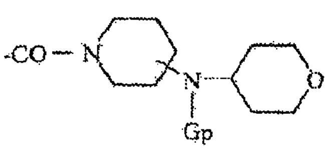 ;

a)

a1) -O- ($C_2$-$C_4$) -alk-A';
a2) -O-($C_1$-$C_4$)-alk-B';
a3) -O-E' ;

b) -($C_1$-$C_4$)-alk-A';
c) -B';

d)

d1) - -($C_1$-$C_4$)-alk-$NR_4$-($C_2$-$C_3$)-alk-A';
d2) - ($C_1$-$C_4$) -alk-$NR_4$- ($C_1$-$C_3$) -alk-B';

e)

e1) -$CONR_4$-($C_2$-$C_4$)-alk-A';
e2) -$CONR_4$-($C_1$-$C_4$)-alk-B';
e3) -$CONR_4$-E';

f)

f1) -CO-D-($C_1$-$C_2$)-alk-A';
f2) -CO-G-A';
f3)

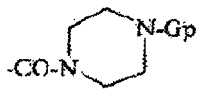

;

f4)

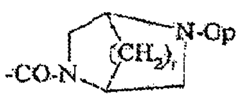

;

und
f5)

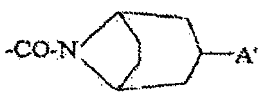

;

ausgewählte Gruppe steht;
worin:

- A', B' und E' für die Gruppen A, B bzw. E gemäß der oben für (Ia) angegebenen Definition stehen, worin $R_5$ durch Gp ersetzt ist;
- Gp für eine Stickstoff-Schutzgruppe steht, die unter Boc, Fmoc, ($C_1$-$C_4$) -Alkanoyl, Benzyloxycarbonyl oder Benzyl ausgewählt ist.

**6.** Medikament, **dadurch gekennzeichnet, dass** es eine Verbindung der Formel (Ia) nach einem der Ansprüche 1 bis 3 oder ein Additionssalz dieser Verbindung mit einer pharmazeutisch unbedenklichen Säure oder auch ein Hydrat oder ein Solvat der Verbindung der Formel (Ia) umfasst.

**7.** Pharmazeutische Zusammensetzung, **dadurch gekennzeichnet, dass** sie eine Verbindung der Formel (Ia) nach einem der Ansprüche 1 bis 3 oder ein pharmazeutisch unbedenkliches Salz, ein Hydrat oder ein Solvat dieser Verbindung sowie mindestens einen pharmazeutisch unbedenklichen Hilfsstoff umfasst.

**8.** Verbindung der Formel (Ia) nach einem der Ansprüche 1 bis 3 zur Verwendung bei der Behandlung und Prävention von akuten oder chronischen immunentzündlichen Erkrankungen und Syndromen, allergischen Erkrankungen, viralen oder bakteriellen Erkrankungen, Herzpathologien, Obesitas, Erkrankungen, an denen angiogene Prozesse beteiligt sind, wie Krebserkrankungen oder Retinaerkrankungen.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 0001727 A
- WO 02051397 A
- WO 02081449 A
- EP 518731 A
- EP 611766 A
- WO 9915525 A
- WO 0253534 A
- WO 0100206 A
- WO 9621656 A
- WO 0039087 A
- EP 62504 A
- EP 393607 A
- EP 997465 A
- WO 9856760 A
- US 5411984 A
- EP 819681 A
- EP 44442 A


### Littérature non-brevet citée dans la description


- **Lombardino, Joseph G.** *J. Med. Chem,* 1973, vol. 16 (5), 493-6
- **Green et al.** Protective Groups in Organic Synthesis. John Wiley & Sons, Inc,
- **J. March.** Advances in Organic Chemistry. Wiley Interscience, 310-316
- **Mitsunobu.** *Bull. Chem. Soc. Japan,* 1967, vol. 40, 2380
- *Synth. Commun.,* 1998, vol. 28 (10), 1897-1905
- *J. Org. Chem.,* 1992, vol. 57 (11), 3218-3225
- *J. Org. Chem,* 1996, vol. 61, 3849-3862
- *Tetrahedron Lett.,* 1990, vol. 31, 5595-5598
- *SY11. Lett.,* 1998, vol. 4, 379-380
- *Arch. Pharm.,* 1962, vol. 295, 292-304
- *Eur. J. Med. Chem.,* 1994, vol. 26 (9), 675-686
- *J. Med. Chem.,* 2002, vol. 45 (16), 3406-3417
- *Pesticide Sciences,* 1995, vol. 44 (1), 96-102
- *Indian J. Chem., section B,* 1987, vol. 26B (3), 287-289
- **C.D. Paavola et al.** *J. Biol. Chem.,* 1998, vol. 273 (50), 33157-33165
- **A. Albini et al.** *Cancer Res.,* 1987, vol. 47, 3239-3245
- **V. Dolle.** *J. Med. Chem.,* 2000, vol. 43, 3949, 3962